# EUROPEAN PATENT APPLICATION

(11) **EP 2 330 116 A1**
(43) Date of publication of application: **08.06.2011**
(21) Application number: 10012953.5
(22) Date of filing: 26.03.2002
(51) Int. Cl.: C07K 14/005

(54) **Replikin peptides and uses thereof**

(30) Priority: 27.03.2001 US 278761 P; 09.07.2001 US 303396 P; 26.10.2001 US 984056; 26.10.2001 US 984057
(62) Divisional of application: 02736514.7
(71) Applicant: Bogoch, Samuel, New York, NY 10028 (US); Bogoch, Elenore S., New York, NY 10028 (US)
(72) Inventor: Bogoch, Samuel, New York, NY 10028 (US); Bogoch, Elenore S., New York, NY 10028 (US)
(74) Representative: Friede, Thomas

(57) **Abstract**

Peptides of influenza virus hemagglutinin protein and Plasmodium falciparum malaria antigen, antibodies specific for the peptides, influenza vaccines, malaria vaccines and methods of stimulating the immune response of a subject to produce antibodies to influenza virus or malaria are disclosed. Also disclosed are methods for formulating vaccines for influenza virus. Isolated peptides of the Bacillus anthracis anthrax Toxin Lethal factor Protein pX01-107, antibodies specific for the peptides and methods of stimulating the immune response of a subject to produce antibodies to the Bacillus anthracis Anthrax Toxin Lethal factor Protein pX01-107 are disclosed. Also disclosed are isolated peptides of the Small Pox Virus Surface Antigen S Precursor Protein, antibodies specific for the peptides and methods of stimulating the immune response of a subject to produce antibodies to the Small Pox Virus Surface Antigen S Precursor Protein.

## Description

### CROSS REFERENCE TO OTHER APPLICATIONS

This application is a Continuation-In-Part of Application Nos. US 09/984,056 and US 09/984,057, both filed October 26, 2001, which claim priority from Provisional Applications 60/303,396, filed July 9, 2001 and 60/278,761 filed March 27, 2001, the subject matter of which is incorporated herein by reference.

### FIELD OF THE INVENTION

This invention relates to the identification and use of Replikins, a class of peptides that share structural characteristics. In particular, this invention relates to Replikins which have been identified in influenza viruses and their use in designing influenza virus vaccines. The invention also relates to Replikins which have been identified in *Bacillus anthracis* and Small Pox Virus (Variola).

### BACKGROUND OF THE INVENTION

Influenza is an acute respiratory illness of global importance. Despite international attempts to control influenza virus outbreaks through vaccination influenza infections remain an important cause of morbidity and mortality. Worldwide influenza pandemics have occurred at irregular and previously unpredictable intervals throughout history and it is expected that they will continue to occur in the future. The impact of pandemic influenza is substantial in terms of morbidity, mortality and economic cost.

Influenza vaccines remain the most effective defense against influenza virus, but because of the ability of the virus to mutate and the availability of non-human host reservoirs it is expected that influenza will remain an emergent or re-emergent infection. Global influenza surveillance indicates that influenza viruses may vary within a country and between countries and continents during an influenza season. Virologic surveillance is of importance in monitoring antigenic shift and drift. Disease surveillance is also important in assessing the impact of epidemics. Both types of information have provided the basis of vaccine composition and the correct use of antivirals. However, to date there has been only annual *post hoc* hematological classification of the increasing number of emerging influenza virus strains, and no specific chemical structure of the viruses has been identified as an indicator of approaching influenza epidemic or pandemic. Currently, the only basis for annual classification of influenza virus as active, inactive or prevalent in a given year is the activities of the virus hemagglutinin and neuraminidase proteins. No influenza viral chemical structure has been identified that can be used for quantitative warning of epidemics or pandemics or to design more effective and safer vaccines.

Because of the annual administration of influenza vaccines and the short period of time when a vaccine can be administered, strategies directed at improving vaccine coverage are of critical importance.

Another disease which has proved difficult to treat and for which there is no effective vaccine is malaria. Malaria causes much physical and economic hardship in tropical regions. Malaria is caused by *Plasmodium ƒalciparum,* which has proved to be extremely resistant to treatment and to date, a vaccine for malaria remains elusive. Thus, there is a need for effective malaria vaccines and methods of treating or preventing the disease.
Other diseases for which an effective vaccine is needed include anthrax and small pox. However, to date, no vaccine has been effective for prevention of disease caused by these pathological organisms. Thus, there is a need for vaccines for these pathogens, as well as a need for effective strategies for formulating vaccines to various pathogens.

### SUMMARY OF THE INVENTION

In one aspect of the invention there are provided isolated influenza virus peptides containing a Replikin sequence. The influenza virus peptides comprise from 7 to about 50 amino acids including (1) at least one lysine residue located six to ten amino acid residues from a second lysine residue; (2) at least one histidine residue; and (3) at least 6% lysine residues.

In another aspect of the invention there is provided a process for stimulating the immune system of a subject to produce antibodies that bind specifically to an influenza virus Replikin sequence, said process comprising administering to the subject an effective amount of a dosage of a composition comprising at least one influenza virus replikin peptide. In a preferred embodiment the composition comprises at least one peptide that is present in an emerging strain of influenza virus.

The present invention also provides antibodies that bind specifically to an influenza virus Replikin, as defined herein, as well as antibody cocktails containing a plurality of antibodies that specifically bind to influenza virus Replikins. In one embodiment of the invention, there are provided compositions comprising an antibody or antibodies that specifically bind to an influenza Replica and a pharmaceutically acceptable carrier.

The present invention also provides therapeutic compositions comprising one or more of isolated influenza virus peptides having from 7 to about 50 amino acids comprising
1) at least one lysine residue located six to ten residues from a second lysine residue; (2) at least one histidine residue; and (3) at least 6% lysine residues,
and a pharmaceutically acceptable carrier.

In another aspect of the invention there is provided an antisense nucleic acid molecule complementary to an influenza virus hemagglutinin Replikin mRNA sequence, said Replikin mRNA sequence having from 7 to about 50 amino acids comprising
(1) at least one lysine residue located six to ten residues from a second lysine residue; (2) at least one histidine residue; and (3) at least 6% lysine residues.

In yet another aspect of the invention, there is provided a method of stimulating the immune system of a subject to produce antibodies to influenza virus comprising administering an effective amount of at least one influenza virus Replikin peptide having from 7 to about 50 amino acids comprising (1) at least one lysine residue located six to ten amino acid residues from a second lysine residue; (2) at least one histidine residue; and (3) at least 6% lysine residues.

In another aspect, there is provided a method of selecting an influenza virus peptide for inclusion in an influenza virus vaccine comprising
(1) obtaining at least one isolate of each strain of a plurality of strains of influenza virus,
(2) analyzing the hemagglutinin amino acid sequence of the at least one isolate of each strain of the plurality of strains of influenza virus for the presence and concentration of Replikin sequences,
(3) comparing the concentration of Replikin sequences in the hemagglutinin amino acid sequence of the at least one isolate of each strain of the plurality of strains of influenza virus to the concentration of Replikin sequences observed in the hemagglutinin amino acid sequence of each of the strains at least one earlier time period to provide the concentration of Replikins for at least two time periods, said at least one earlier time period being within about six months to about three years prior to step (1),
(4) identifying the strain of influenza virus having the highest increase in concentration of Replikin sequences during the at least two time periods,
(5) selecting at least one Replikin sequence present in the strain of influenza virus peptide identified in step (4) as a peptide for inclusion in an influenza virus vaccine.

The present invention also provides a method of making an influenza virus vaccine comprising
(1) identifying a strain of influenza virus as an emerging strain,
(2) selecting at least one Replikin sequence present in the emerging strain as a peptide template for influenza virus vaccine manufacture,
(3) synthesizing peptides having the amino acid sequence of the at least one Replikin sequence selected in step (2), and
(4) combining a therapeutically effective amount of the peptides of step (4) with a pharmaceutically acceptable carrier and/or adjuvant.

In another aspect, the invention is directed to a method of identifying an emerging strain of influenza virus for diagnostic or therapeutic purposes comprising
(1) obtaining at least one isolate of each strain of a plurality of strains of influenza virus,
(2) analyzing the hemagglutinin amino acid sequence of the at least one isolate of each strain of the plurality of strains of influenza virus for the presence and concentration of Replikin sequences,
(3) comparing the concentration of Replikin sequences in the hemagglutinin amino acid sequence of the at least one isolate of each strain of the plurality of strains of influenza virus to the concentration of Replikin sequences observed in the hemagglutinin amino acid sequence of each of the strains at at least one earlier time period to provide the concentration of Replikins for at least two time periods, said at least one earlier time period being within about six months to about three years prior to step (1), and
(4) identifying the strain of influenza virus having the highest increase in concentration of Replikin sequences during the at least two time periods.

In yet another aspect of the invention, there is provided an influenza virus vaccine comprising at least one isolated Replikin present in the hemagglutinin protein of an emerging strain of influenza virus and a pharmaceutically acceptable carrier and/or adjuvant.

Also provided by the present invention is a method of preventing or treating influenza virus infection comprising administering to a patient in need thereof a vaccine comprising at least one isolated Replikin present in the hemagglutinin protein of an emerging strain of influenza virus and a pharmaceutically acceptable carrier and/or adjuvant.

In another aspect of the invention, there are provided vaccines and methods for preventing or treating malaria. The malaria vaccines comprise at least one isolated *Plasmodium ƒalciparum* Replikin. The present invention also provides methods for treating or preventing malaria comprising administering to a patient an effective amount of a vaccine comprising at least one isolated *Plasmodium ƒalciparum* Replikin.

Also provided by the present invention are antibodies, antibody cocktails and compositions that comprise antibodies that specifically bind to a Replikin or Replikins present in a malaria antigen *of Plasmodium ƒalciparum.*
In another aspect of the invention there are provided isolated *Bacillus anthracis* (Anthrax) peptides containing a replikin sequence. The Anthrax peptides comprise from 7 to about 50 amino acids including (1) at least one lysine residue located six to ten amino acid residues from a second lysine residue; (2) at least one histidine residue; and (3) at least 6% lysine residues. In another embodiment of this aspect of the invention there are provided Small Pox Virus peptides containing a replikin sequence which comprises from 7 to about 50 amino acids including (1) at least one lysine residue located six to ten amino acid residues from a second lysine residue; (2) at least one histidine residue; and (3) at least 6% lysine residues.
In another aspect of the invention there is provided a process for stimulating the immune system of a subject to produce antibodies that bind specifically to Anthrax polypeptides containing a replikin sequence, said process comprising administering to the subject an effective amount of a dosage of a composition comprising at least one Anthrax replikin peptide. In a preferred embodiment the composition comprises at least one peptide selected from SEQ ID NO. 91, SEQ ID NO. 92, SEQ ID NO. 93, SEQ 1D NO. 94, SEQ ID NO. 95, SEQ ID NO. 96, SEQ ID NO. 79, SEQ ID NO. 98 or a combination thereof.
In another embodiment of this aspect of the invention there is provided a process for stimulating the immune system of a subject to produce antibodies that bind specifically to Small Pox Virus polypeptides containing a replikin sequence, said process comprising administering to the subject an effective amount of a dosage of a composition comprising at least one Small Pox Virus replikin peptide. In a preferred embodiment the composition comprises a peptide selected from SEQ ID NO. 99, SEQ ID NO. 100, SEQ ID NO. 101, SEQ ID NO. 102, SEQ ID NO. 103, or a combination thereof.
In another aspect of the invention there are provided antisense nucleic acid molecules complementary to the coding strand of the gene or to the mRNA encoding the *Bacillus anthracis* Anthrax Lethal Factor Protein pX01-107 peptide, wherein said antisense nucleic acid molecule is complementary to a nucleotide sequence encoding the peptide of SEQ ID NO. 91, SEQ ID NO. 92, SEQ ID NO. 93, SEQ ID NO. 94, SEQ ID NO. 95, SEQ ID NO. 96, SEQ ID NO. 97, SEQ ID NO. 98.
There are also provided antisense nucleic acid molecule complementary to the coding strand of the gene or to the mRNA encoding the Small Pox Virus Surface Antigen S Precursor Protein, wherein said antisense nucleic acid molecule is complementary to a nucleotide sequence encoding the peptide of SEQ ID NO. 99, SEQ ID NO. 100, SEQ ID NO. 101, SEQ ID NO. 102, or SEQ ID NO. 103.
The present invention also provides methods for detecting the presence of a contaminating organism in a body sample or environmental sample comprising 1) isolating nucleic acids from the body sample or environmental sample; 2) screening the nucleic acids for the presence of a replikin structure; and 3) correlating the presence of a Replikin structure with the presence of the contaminating organism.
In yet another aspect of the invention there is provided a method for increasing the replication rate of an organism comprising transforming a gene encoding an enzyme having a replication function in the organism with at least one Replikin structure.

As used herein, the term "peptide" refers to a compound of two or more amino acids in which the carboxyl group of one is united with an amino group of another, forming a peptide bond. The term peptide is also used to denote the amino acid sequence encoding such a compound. Thus, a peptide sequence may be a subsequence of a larger polypeptide sequence. As used herein, a Replikin peptide is a peptide having 7 to about 50 amino acids comprising (1) at least one lysine residue located six to ten amino acid residues from a second lysine residue; (2) at least one histidine residue; and (3) at least 6% lysine residues. Similarly, a replikin sequence is the amino acid sequence encoding such a peptide.

The phrase "emerging strain" as used herein refers to a strain of influenza virus identified as having an increasing concentration of Replikin sequences in its hemagglutinin and/or neuraminidase protein sequence, relative to the concentration of replikins in other strains of influenza virus. The increase in concentration occurs over a period of at least about six months, and preferably over a period of at least about one year, most preferably over a period of at least about three years or more.

### BRIEF DESCRIPTION OF THE DRAWINGS

Figure 1 is a bar graph depicting the frequency of occurrence of replikins in various protein groups.

Figure 2 is a graph depicting the percentage of malignin per milligram total membrane protein during anaerobic replication of glioblastoma cells.

Figure 3 is a bar graph showing amount of antimalignin antibody produced in response to exposure to the recognin 16-mer.

Figure 4A is a photograph of a blood smear taken with ordinary and fluorescent light. Figure 4B is a photograph of a blood smear taken with ordinary and fluorescent light illustrating the presence of two leukemic cells. Figure 4C is a photograph of a dense layer of glioma cells in the presence of antimalignin antibody. Figure 4D and Figure 4E are photographs of the layer of cells in Figure 4C taken at 30 and 45 minutes following addition of antimalignin antibody.

Figure 4F is a bar graph showing the inhibition of growth of small cell lung carcinoma cells *in vitro* by antimalignin antibody.

Figure 5 is a plot of the amount of antimalignin antibody present in the serum of patients with benign or malignant breast disease pre-and post surgery.

Figure 6 is a box diagram depicting an embodiment of the invention wherein a computer is used to carry out the 3-point-recognition method of-identifying replikin sequences.

Figure 7 is a graph showing the concentration ofReplikins observed in hemagglutinin of influenza B and influenza A strain, H1N1, on a year by year basis from 1918 through 2001.

Figure 8 is a graph of the replikin concentration observed in hemagglutinin of influenza A strains, H2N2 and H3N2, as well as an emerging strain defined by its constituent Replilkins, designated H3N2(R), on a year by year basis from 1950 to 2001.

### DETAILED DESCRIPTION OF THE INVENTION

The present invention provides methods for predicting future epidemics or pandemics of influenza virus, and vaccines and methods of designing effective vaccines against influenza virus. Identification of a new family of small peptides related to the phenomenon of rapid replication, referred to herein as Replikins, provides new targets for detection of pathogens in a sample and vaccine development, such as for example, influenza virus detection and influenza vaccine development. Identification of this new family of peptides also provides for the detection of malaria and provides new targets for malaria vaccine development. The discovery of this family of peptides also provides for the detection and provides new targets for anthrax and small pox virus, for example. In general, knowledge of and identification of this family of peptides enables development of effective vaccines for any organism that harbors Replikins.

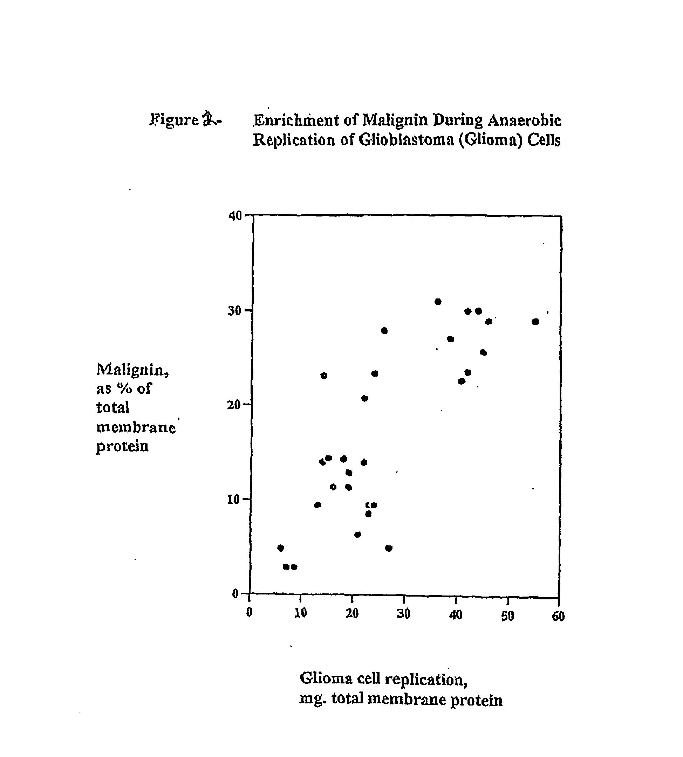

The first Replikin sequence to be identified was the cancer cell Replikin found in a brain cancer protein, malignin, which was demonstrated to be enriched ten-fold during rapid anaerobic replication of glioblastoma multiforme (glioma) cells. (**Figure 2**) Malignin is a 10KDa portion of the 250 KDa glycoprotein IOB, which was isolated *in vivo and in vitro* from membranes of glioblastoma multiforme (glioma) cells. Hydrolysis and mass spectroscopy of malignin revealed a16-mer peptide sequence, ykagvafthkkndide (**SEQ ID NO.:4**), which is referred to herein as the glioma Replikin and which includes the shorter peptide, kagvaflhkk (**SEQ ID NO.: 1**), both of which apparently are absent in the normal human genome.

Table 1 illustrates how the sequence of the glioma Replikin, the 16-mer peptide sequence, ykagvaflhkkndide **(SEQ ID NO.:4)** was determined.

**Table 1 16-mer peptide sequence ykagvaflhkkndide obtained from malignin by hydrolysis and mass spectrometry**

| **Seq** | **Fragment** | **MH+** | **Sequence** | **Method By Which Fragment Obtained** | | | |
|---|---|---|---|---|---|---|---|
| **ID NO.** | **Identified** | **(mass)** | | **Auto- hydrolysis of malignin free in solution** | **Auto- hydrolysis of malignin immobilized on bromoace-tyl cellulose** | **Microwaved 5 seconds** | **Microwaved 30 seconds** |
| 19 | 1-3 | 381.21 | 0yka(g) | | | | + |
| 20 | 1-5 | 537.30 | 0ykagv(a) | | + | | |
| 21 | 2-6 | 445.28 | (y)kagva(f) | | + | | |
| 22 | 2-7 | 592.35 | (Y)kagvaf(l) | | | + | |
| 23 | 4-11 | 899.55 | (a)gvaflhkk(n) | | | | + |
| 24 | 5-7 | 336.19 | (g)vaf(l) | | | | + |
| 25 | 6-7 | 237.12 | (v)af(1) | + | | | |
| 26 | 6-10 | 615.36 | (v)aflhk(k) | | | | + |
| 27 | 6-10 | 615.36 | (v)aflhk(k) | + | | | |
| 28 | 6-12 | 857.50 | (v)aflhkkn(d) | | + | | |
| 29 | 6-12 | 857.50 | (v)afbkkn(d) | + | | | |
| 30 | 7-8 | 279.17 | (a)fl(h) | | | + | |
| 31 | 10-16 | 861.43 | (h)kkndide0 | | + | | |
| 32 | 11-14 | 489.27 | (k)kndi(d) | | + | | |
| 33 | 12-15 | 476.2- | (k)ndid(e) | + | | | |

When the 16-mer glioma Replikin was synthesized and injected as a synthetic vaccine into rabbits, abundant antimalignin antibody was produced. (Bogoch et al., Cancer Detection and Prevention, 26(Supp. 1): 402 (2002). The concentration of antimalignin antibody in serum *in vivo* has been shown to relate quantitatively to the survival of cancer patients. (Bogoch et al., Protides of Biological Fluids, 31:739-747 (1984). *In vitro* antimalignin antibodies have been shown to be cytotoxic to cancer cells at a concentration of picograms (femtomolar) per cancer cell. (Bogoch et al., Cancer Detection and Prevention, 26(Supp. 1): 402 (2002).

Studies carried out by the inventors showed that the glioma Replikin is not represented in the normal healthy human genome. Consequently, a search for the origin and possible homologues of the Replikin sequence was undertaken by analysis of published sequences of various organisms.

By using the 16-mer glioma Replikin sequence as a template and constructing a recognition proteomic system to visually scan the amino acid sequences of proteins of several different organisms, a new class of peptides, the Replikins, was identified. The present invention provides a method for identifying nucleotide or amino acid sequences that include a Replikin sequence. The method is referred to herein as a 3-point-recognition method. By use of the "3-point recognition" method, described herein below, a new class of peptides was revealed in algae, yeast, fungi, amoebae, bacteria, plant and virus proteins having replication, transformation, or redox functions. Surprisingly, the Replikin peptides were found to be concentrated in larger 'replicating' and 'transforming' proteins (so designated by their investigators, See Table 2). No sequences were found to be identical to the malignin 16-mer peptide.

Table 2 illustrates several Replikin sequences that were identified by the 3-point recognition method of the invention.

**Table 2: Examples of Replikins in various organisms - prototype: Glioma Replikin* kagvaflhkk (SEQ ID No.:1)**

| Algae: | SEQ ID NO. | | |
|---|---|---|---|
| | 34 | Caldophera prolifera | kaskftkh |
| | 35 | Isolepisprosfera | kaqaetgeikgh |
| Yeast: | 36 | Schizosaccharomyces pombe | ksfltypkkhk |
| | 37 | Oryza sativa | kkaygnelhk |
| | 2 | Sacch. cerevisiae replication binding protein | hsikrelgiifdk |
| Fungi: | | Isocitrate lyase ICI l,Penicillium marneffei | kvdivthqk |
| | 38 | DNA-dependent RNA polymerase 11, Diseula destructiva | kleedaayhrkk |
| | 39 | Ophiostoma novo-u1m 1,RNA in Dutch elm disease | kvilplrgnikgiffkh |
| | 40 | fungus | |
| Amoeba: | 41 | Entamoeba invadens, histone H2B | klilkgdlnkh |
| Bacteria: | 42 | Pribosomal protein replication factor, Helicobacter pylori | ksvhaflk |
| | | Replication-associated protein Staph. aureus | |
| | 10 | Mycoplasma pulmonic, chromosome replication | kkektthnk |
| | 43 | Macrophage infectivity potentiator, L legionella | kvhffqlkk |
| | 90 | Bacillus anthracis | kihlisvkk |
| | 91 | Bacillus anthracis | hvkhekeknk |
| | 92 | Bacillus anthracis | khivkievk |
| | 93 | Bacillus anthracis | kkkkikdiygkdallh |
| | 94 | Bacillus anthracis | kwekikqh |
| | 95 | Bacillus anthracis | kklqipppiepkkddiih |
| | 96 | Bacillus anthracis | hnryasnivesayllinew- |
| | | | knniqsdlikk |
| | 97 | Bacillus anthracis | havddyagylldknqsdlv- |
| | | | tnskk haerlkvqknapk |
| | 98 | Bacillus anthracis | |
| Plants: | 44 | Arabidopsis thaliana, prolifera | kdhdfdgdk |
| | 45 | Arabidopsis thaliana, cytoplasmic ribosomal | kmkglkqkkah |
| | 46 | Arabidopsis thaliana, DNA binding protein | kelssttqeksh |
| Viruses: | 9 | Replication associated protein A [Maize streak virus] | kekkpskdeimrdiish |
| | 11 | Bovine herpes virus 4, DNA replication protein | hkinitngqk |
| | 12 | Meleagrid herpesvirus 1, replication binding protein | hkdlyrllmk |
| | 47 | Feline immunodeficiency | hlkdyklvk |
| | 3 | Foot and Mouth Disease (O) | hkqkivapvk |
| | 5 | HIV Type 1 | kcfncgkegh |
| | 7 | HIV Type 2 | kcwncgkegh |
| | 99 | Small Pox Virus (Variola) | khynnitwyk |
| | 100 | Small Pox Virus (Variola) | kysqtgkeliih |
| | 101 | Small Pox Virus (Variola) | hyddvrikndivvsrck |
| | 102 | Small Pox Virus (Variola) | hrfklildski |
| | 103 | Small Pox Virus (Variola) | kerghnyyfek |
| | | | |
| Tumor | 48 | Rous sarcoma virus tyrosine-protein kinase | kklrhek |
| Viruses: | 49 | v-yes, avian sarcoma | kklrhdk |
| | 50 | c-yes, colon cancer, malignant melanoma | kklrhdk |
| | 51 | v-srcC, avian sarcoma | kklrhek |
| | 52 | c-src, colon, mammary, panrcreatic cancer | kklrhek |
| | 53 | Neuroblastoma RAS viral (v-ras) oncogene | kqahelak |
| | 54 | VPI (major capsid protein) (polyamavirus sp.] | kthrfskh |
| | 55 | Sindbis | knlhekik |
| | 56 | EI [Human papilloamavirus type 71] | khrpllqlk |
| | 57 | v-erbB from AEV and c-erb | kspnhvk |
| | 58 | v-fms (feline sarcoma) | knihlekk |
| | 59 | c-fms (acute and chronic myclomonocytic tumors) | knihlekk |
| | 60 | large antigen I [Polyomavirus sp.I | kphlaqslek |
| | 61 | middle t-antigen [Polyomavirus sp,I- | kqhrelkdk |
| | 62 | small t-antigen [Polyomavirus spJ, | kqhrelkdk |
| | 63 | v-abl, murine acute leukemia | kvpvlisptlkh |
| | 64 | Human T-cell lymphotropic virus typo 2 | kslllevdkdish |
| | 65 | c-kit, GI tumors, small cell lung carcinoma | kagitimvkreyh |
| | 18 | Hepatitis C | hyppkpgcivpak |
| | | | |
| Trans- | 66 | Transforming protein myb | ksgkhlgk |
| forming | 67 | Transforming protein myc, Burkitt lymphoma | krreqlkhk |
| Proteins: | 68 | Ras-related GTP-binding protein | ksfevikvih |
| | 69 | Transforming protein ras (teratocarcinoma) | kkkhtvkk |
| | 70 | TRAF-associated NF•kB activator TANK | kaqkdhlsk |
| | 71 | RFP transforming protein | hlkrvkdlkk |
| | 72 | Transforming protein D (S.C.) | kygspkhrlik |
| | 73 | Papilloma virus type 11, transforming protein | klkhilgkarfik |
| | 74 | Protein tryosine kinasc (EC 2.7.1.ll2slk | kgdhvkhykirk |
| | 75 | Transforming protein (axl(-)) | keklrdvmvdrhk |
| | 76 | Transforming protein (N-myc) | klqarqqqllkkieh |
| | 77 | Fibroblast growth factor 4 (Kaposi sarcoma) | kkgnrvsptmkvth |
| Cancer | 78 | Matrix metaloproteinase 7 (uterine) | keiplhfrk |
| Cell | 79 | Transcription factor 7-like | kkkphikk |
| Proteins: | 80 | Breast cancer antigen NY-BR-87 | ktrhdplak |
| | 81 | BRCA-1-Associated Ring Domain Protein (breast) | khhpkdnlik |
| | 82 | 'Autoantigen from a breast tumor' | khkrkkfrqk |
| | 83 | Glioma Replikin (this study) | kagvaflhkk |
| | 84 | Ovarian cancer antigen | khkrkkfrqk |
| | 85 | EE L leukemia | kkkskkhdk |
| | 86 | Proto-oncogene tyrosine-protein kinase C-ABLE | hksekpalprk |
| | 87 | Adenomatosis polyposis coli | kkkpsrlkgdnek |
| | 88 | Gastric cancer transforming protein | ktkkgnrvsptmkvth |
| | 89 | Transforming protein (K-RAS 2B),lung | khkekmskdgkkkkkksk |
| | | | |

Identification of an amino acid sequence as a Replikin or as containing a Replikin, *i.e.,* a homologue of the glioma peptide, kagvaflhkk, requires that the three following requirements be met. The peptide sequence must have (1) at least one lysine residue located six to ten residues from another lysine residue; (2) at least one histidine residue; and (3) a composition of at least 6% lysine within an amino acid sequence of 7 to about 50 residues.

Databases were searched using the National Library of Medicine keyword "PubMed" descriptor for protein sequences containing Replikin sequences. Over 4,000 protein sequences were visually examined for homologues. Sequences of all individual proteins within each group of PubMed-classified proteins were visually scanned for peptides meeting the three above-listed requirements. An infrequent occurrence of homologues was observed in "virus peptides" as a whole (1.5%) (N=953), and in other peptides not designated as associated with malignant transformation or replication such as "brain peptides" and "neuropeptides" (together 8.5%) (N=845). However, surprisingly, homologues were significantly more frequently identified in large "replicating proteins," which were identified as having an established function in replication in bacteria, algae, and viruses. Even more surprising was the finding that Replikin homologues occurred in 100% of "tumor viruses" (N=250), in 97% of "cancer proteins" (N=401), and in 85% of "transforming viruses" (N=248). These results suggest that there are shared properties of cancer pathogenesis regardless of cell type and suggest a role of viruses in carcinogenesis, i.e., conversion of cells from a transformed albeit dormant state to a more virulent actively replicating state.

To permit classification of subtypes of Replilkins, additional or "auxiliary specifications" to the basic "3-point-recognition" requirements maybe added: (a) on a structural basis, such as the common occurrence of adjacent di- and polylysines in cancer cell proteins (e.g., transforming protein P21B(K-RAS 2B), lung, Table 2, **SEQ ID NO.:89**), and other adjacent di-amino acids in TOLL-like receptors, or b) on a functional basis, such as exhibiting ATPase, tyrosine kinase or redox activity as seen in Table 2.

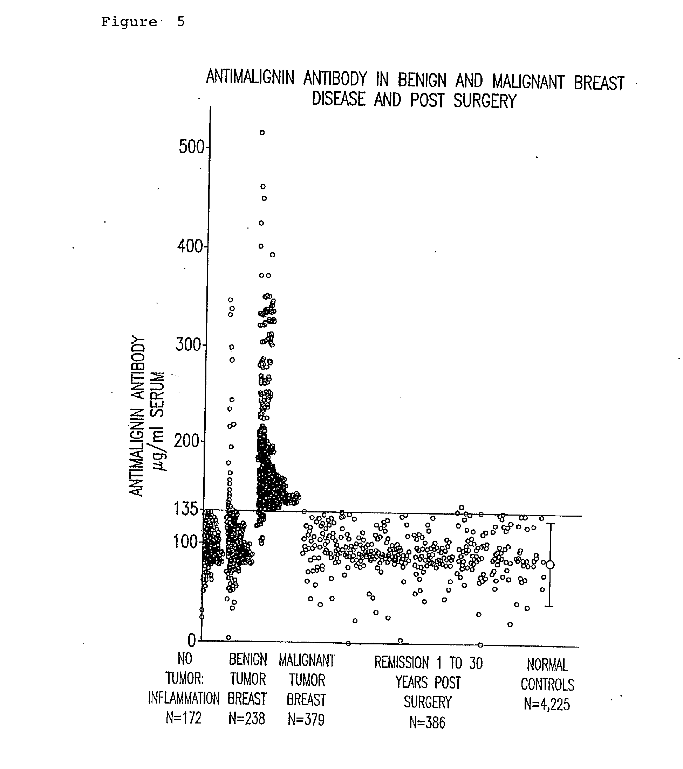

Whether Replikin structures are conserved or are subject to extensive natural mutation was examined by scanning the protein sequences of various isolates of foot and mouth disease virus (FMDV), where mutations in proteins of these viruses have been well documented worldwide for decades. Protein sequences of FMDV isolates were visually examined for the presence of both the entire Replilkin and each of the component Replikin amino acid residues observed in a particular Replikin. For example, in the protein VP1 of FMDV type O, the Replikin **(SEQ ID NO.: 3) "hkqkivapvk"** was found to be conserved in 78% of the 236 isolates reported in PubMed, and each amino acid was found to be conserved in individual isolates as follows: his, 95.6%; lys, 91.8%; gln 92.3%; lys, 84.1%; ile, 90.7%; val, 91.8%; ala, 97.3%; pro, 96.2%; ala, 75.4%; and lys, 88.4%. The high rate of conservation suggests structural and functional stability of the Replikin structure. Similarly, sequence conservation was observed in different isolates of HIV for its Replikins, such as **(SEQ ID NO.:5) "kcfncgkegh"** or **(SEQ ID NO.:6) "kvylawvpahk"** in HIV Type 1 and **(SEQ ID NO.:7) "kcwncgkegh"** in HIV Type 2 (Table 2). Other examples of conservation are seen in the constant presence of malignin in successive generations, over ten years of tissue culture of glioma cells, and by the constancy of affinity of the glioma Replikin for antimalignin antibody isolated by immunoadsorption from 8,090 human sera from the U.S., U.K., Europe and Asia (e.g., **Figure 5** and U.S. Patent 6,242,578 B1).

As seen in **Figure 2**, during anaerobic respiration when the rate of cell replication is increased, malignin is enriched. That is, malignin is found to increase not simply in proportion to the increase in cell number and total membrane proteins, but is enriched as much as tenfold in concentration, starting with 3% at rest and reaching 30% of total membrane protein. This clear demonstration of a marked increases in Replikin concentration with glioma cell replication points to and is consistent with the presence of Replikins here sought by the 3-point recognition method and found in the proteins of various organisms which were found by mutation studies and other previous studies to be critical to replication. For example, Replikins were identified in such proteins as *"Saccharomyces cerevisiae* replication binding protein" **(SEQ ID NO.: 2) (hsikrelgiifdk);** the "replication associated protein A of maize streak virus" **(SEQ ID NO.:8) (kyivcareahk)** and **(SEQ ID NO.:9) (kekkpskdeimrdiish);** the "replication-associated protein of *Staphylococcus aureus"* **(SEQ ID NO.:10) (kkektthnk);** the "DNA replication protein of bovine herpes virus 4" **(SEQ ID NO.:11) (hkinitngqk);** and the "Mealigrid herpes virus 1 replication binding protein" **(SEQ ID NO.:12) (hkdlyrllmk).** Previous studies of tomato leaf curl gemini virus show that the regulation of virus accumulation appears to involve binding of amino acids 1-160 of the "replicating protein" of that virus to leaf DNA and to other replication protein molecules during virus replication. Analysis of this sequence showed that amino acids 1-163 of this "replicating protein" contain five Replikins, namely: **(SEQ ID NO.:13) kfrinaknyfltyph, (SEQ ID NO.:14) knletpvnklfirierefh, (SEQ ID NO.:15) hpniqaaksstdvk, (SEQ ID NO.:16) ksstdvkaymdkdgdvldh,** and **(SEQ ID NO.:17) kasalnilrekapkdfvlqfh.**

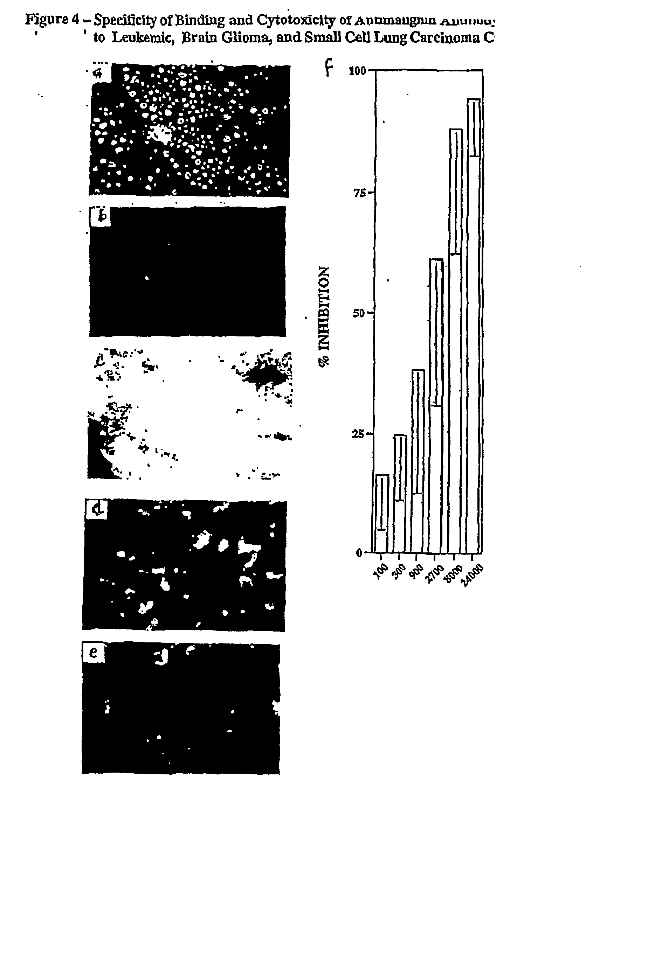

Table 2 shows that Replikin-containing proteins also are associated frequently with redox functions, and protein synthesis or elongation, as well as with cell replication. The association with metal-based redox functions, the enrichment of the Replikin-containing glioma malignin concentration during anaerobic replication, and the cytotoxicity of antimalignin at low concentrations (picograms/cell) (Figure 4c-f), all suggest that the Replikins are related to central respiratory functions, which are perhaps less often subjected to the mutations characteristic of proteins of more superficial location or less central survival function.

Of particular interest, it was observed that at least one Replikin per 100 amino acids was found to be present in the hemagglutinin proteins of almost all of the individual strains of influenza viruses examined. The replikin sequences that were observed to occur in the hemagglutinin proteins of isolates of each of the four prevalent strains of influenza virus, influenza B, H1N1, H2N2, and H3N2, for each year that amino acid sequence data are available (1902-2001) are shown in Tables 3, 4, 5 and 6, below.

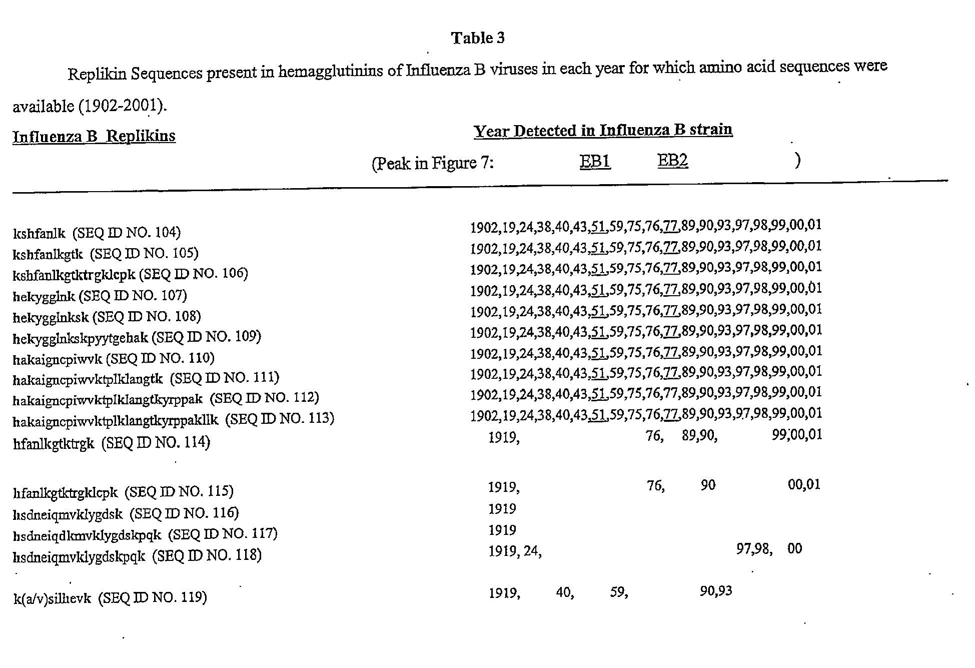

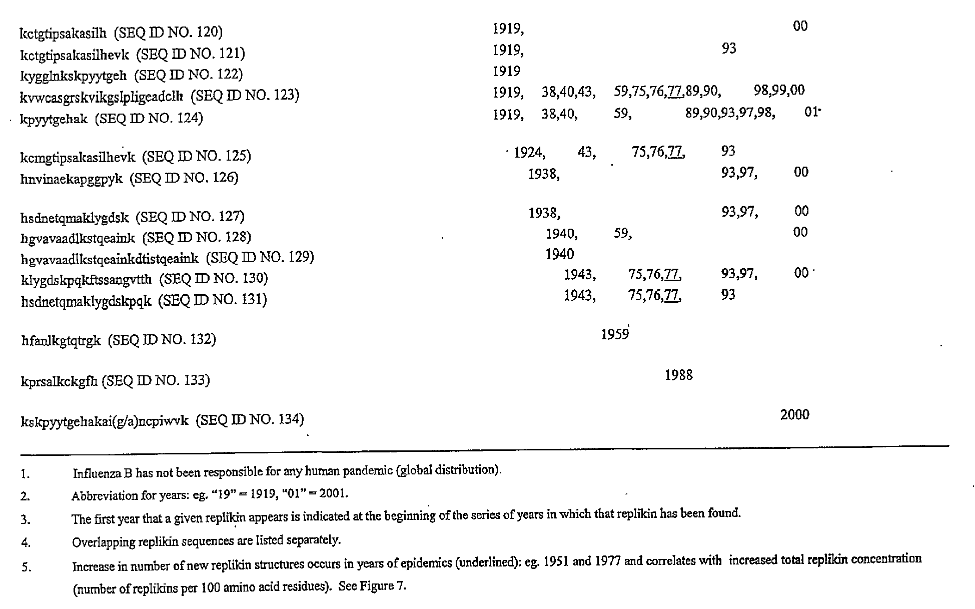

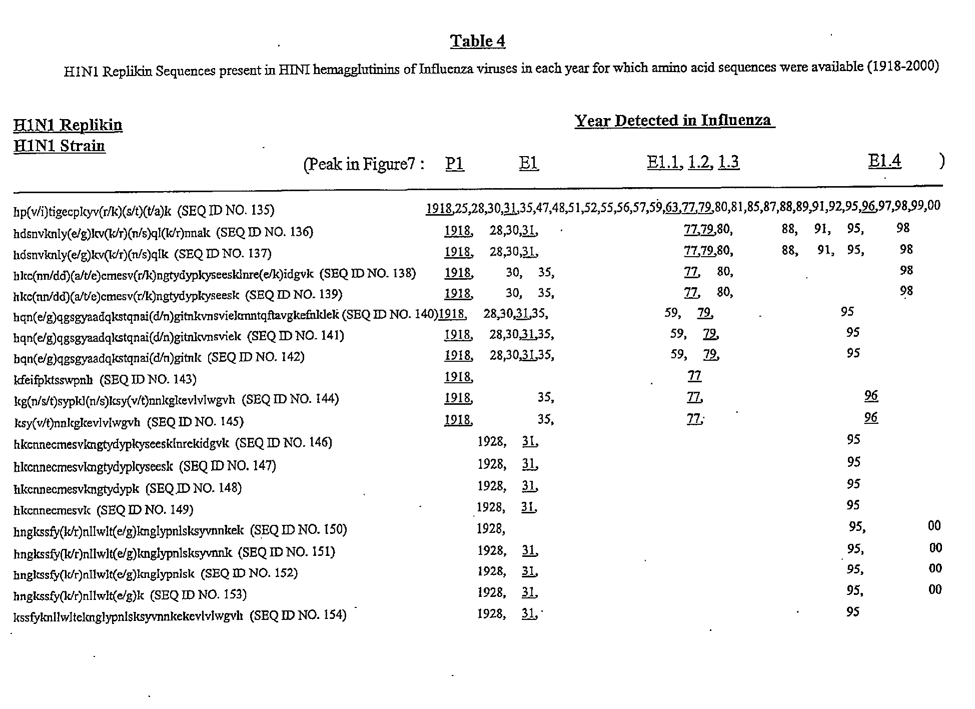

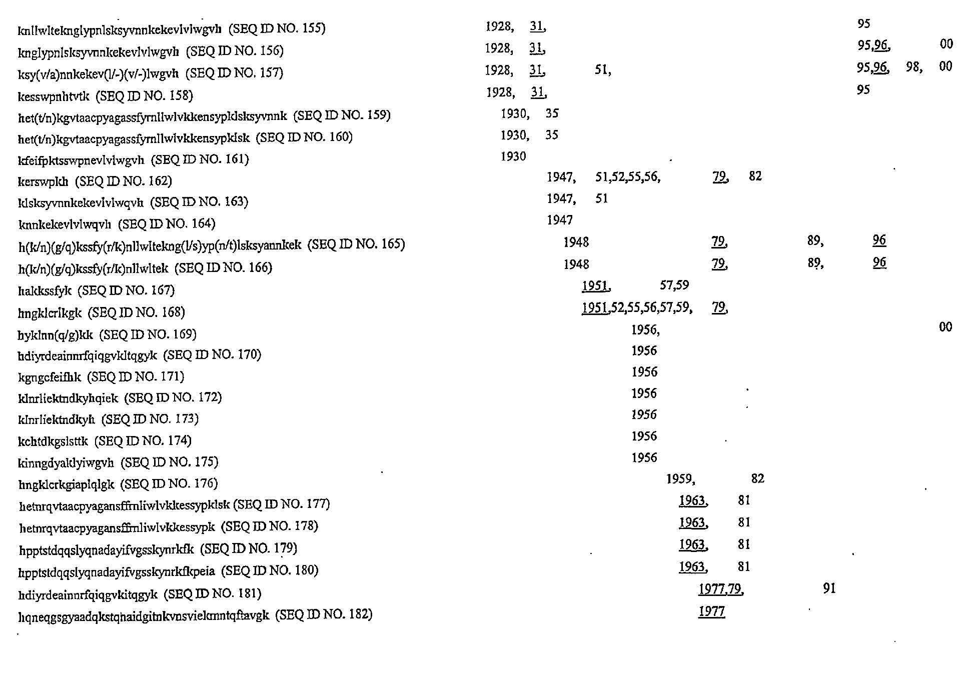

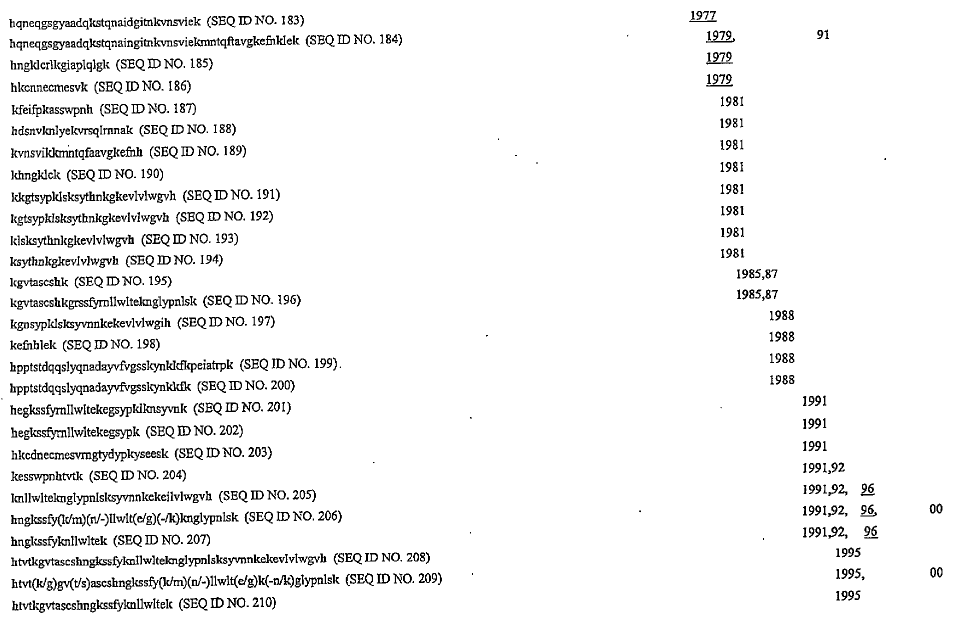

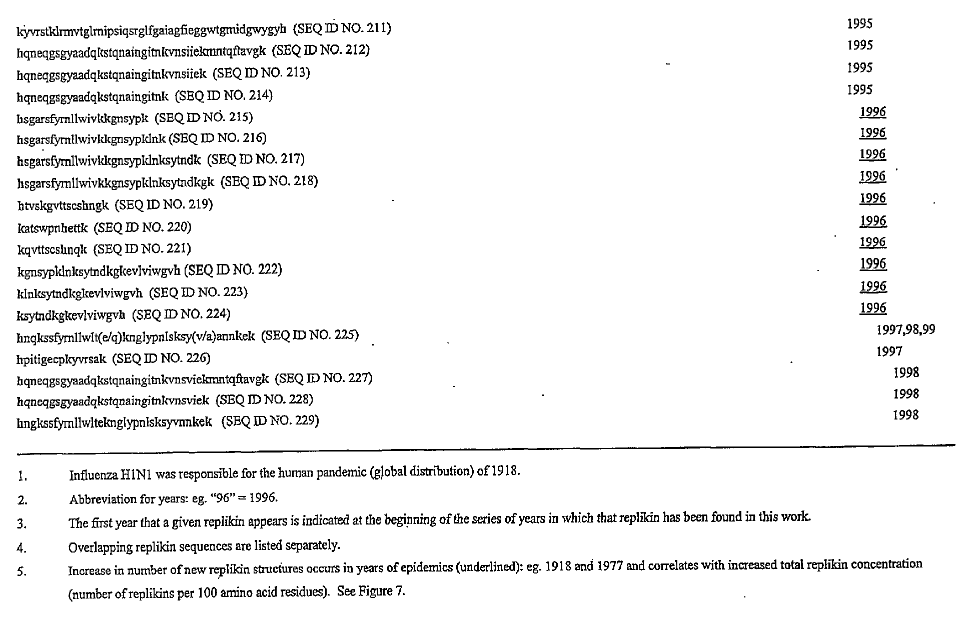

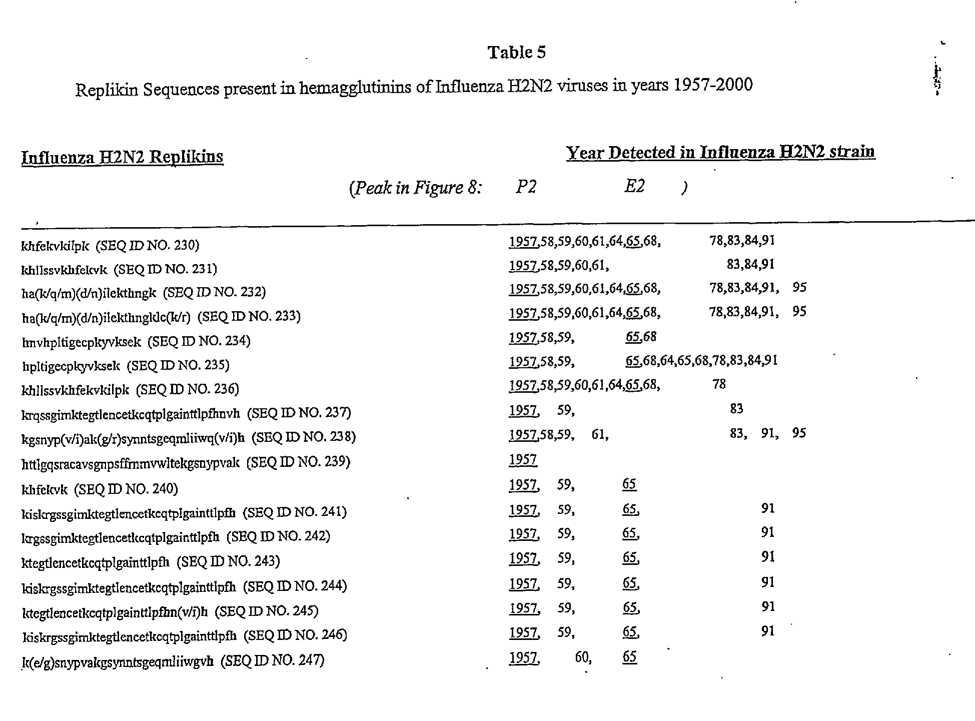

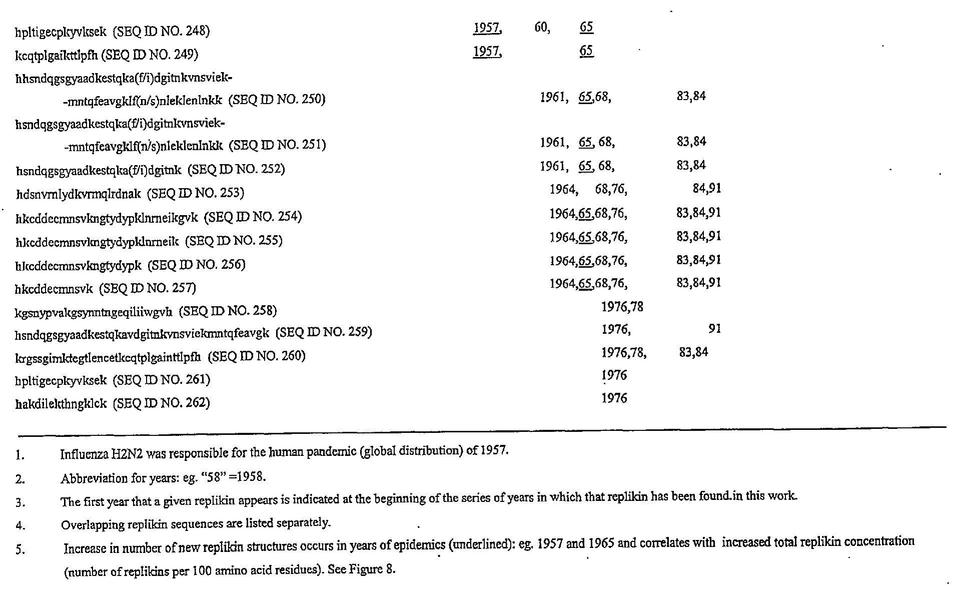

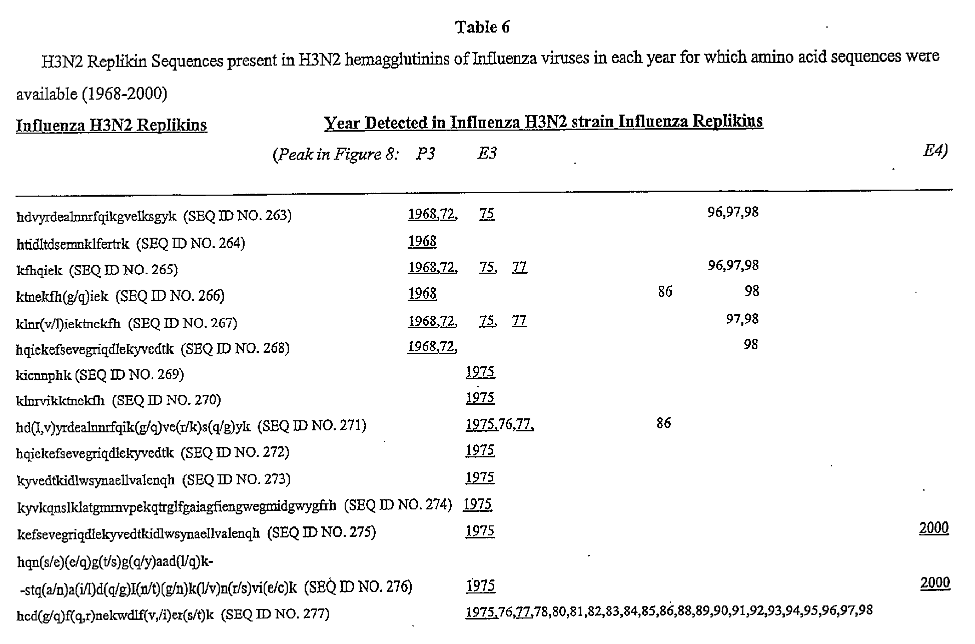

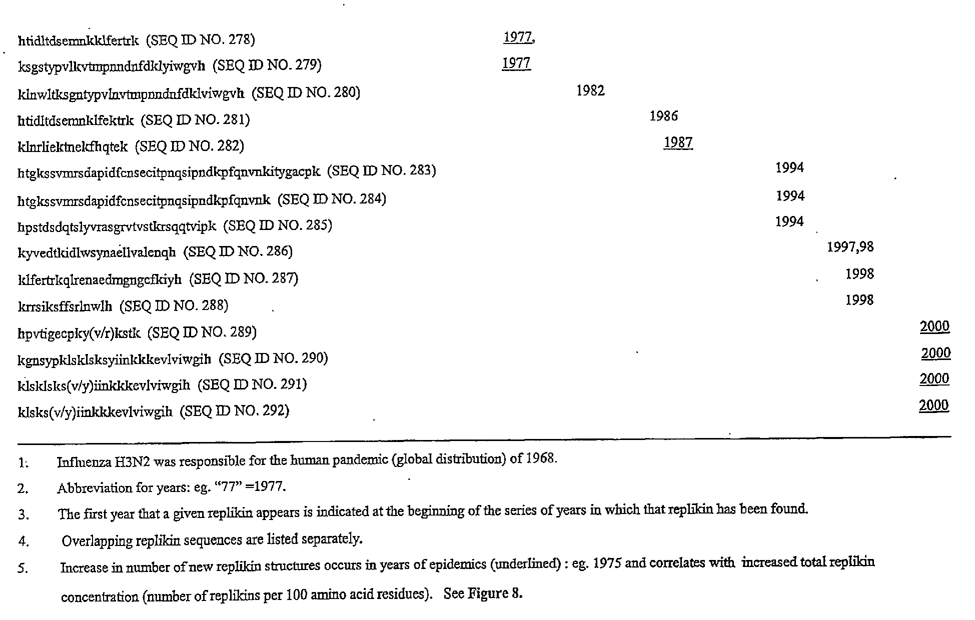

Both the concentration and type, i.e., composition of Replikins observed were found to relate to the occurrence of influenza pandemics and epidemics. The concentration of Replikins in influenza viruses was examined by visually scanning the hemagglutinin amino acid sequences published in the National Library of Medicine "PubMed" data base for influenza strains isolated world wide from human and animal reservoirs year by year over the past century, i.e., 1900 to 2001. These Replikin concentrations (number of Replikins per 100 amino acids, mean +/- SD) were then plotted for each strain.

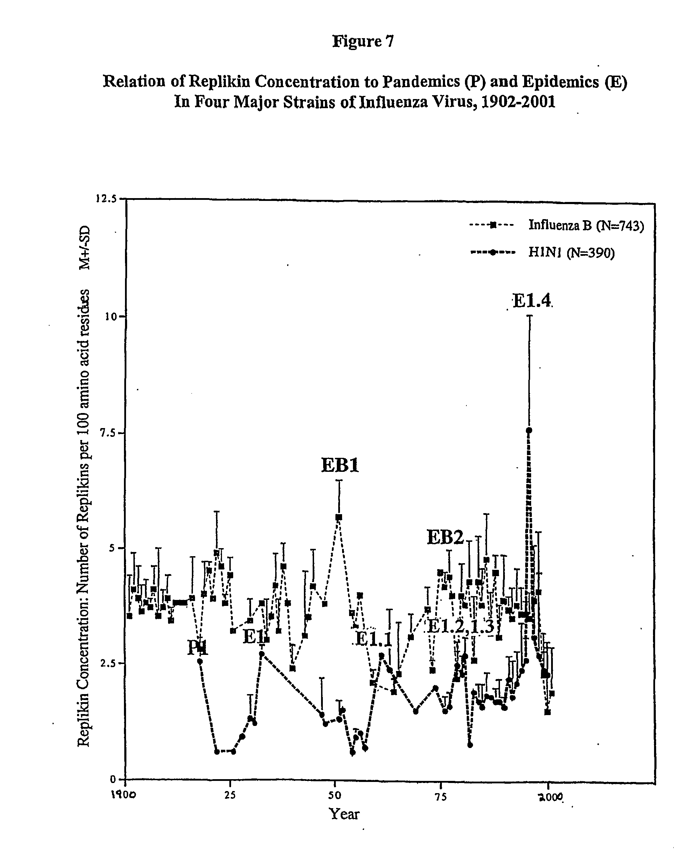

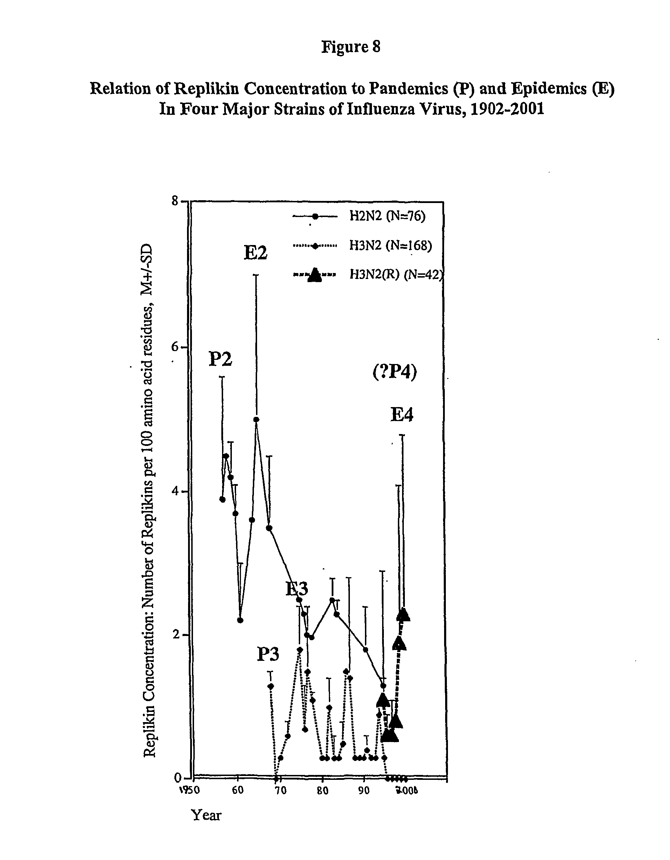

The concentration of Replikins was found to directly relate to the occurrence of influenza pandemics and epidemics. The concentration of Replikins found in influenza B hemagglutinin and influenza A strain, H1N1, is shown in Figure 7, and the concentration of Replikins found in the two other common influenza virus A strains, H2N2 and H3N2 is shown in Figure 8 (H2N2, H3N2). The data in Figure 8 also demonstrate an emerging new strain of influenza virus as defined by its constituent Replikins (H3N2(R)).

Each influenza A strain has been responsible for one pandemic: in 1918, 1957, and 1968, respectively. The data in Figures 7 and 8 show that at least one replikin per 100 amino acids is present in each of the influenza hemagglutinin proteins of all isolates of the four common influenza viruses examined, suggesting a function for Replikins in the maintenance of survival levels of replication. In the 1990s, during the decline of the H3N2 strain there were no Replikins present in many isolates of H3N2, but a high concentration of new replikins appeared in H3N2 isolates, which define the emergence of the H3N2(R) strain.

Several properties of Replikin concentration are seen in Figure 7 and Figure 8 to be common to all four influenza virus strains: (1) Concentration is cyclic over the years, with a single cycle of rise and fall occurring over a period of two to thirty years. This rise and fall is consistent with the known waxing and waning of individual influenza virus strain predominance by hemagglutinin and neuraminidase classification. (2) Peak Replikin concentrations of each influenza virus strain previously shown to be responsible for a pandemic were observed to relate specifically and individually to each of the three years of the pandemics. For example, for the pandemic of 1918, where the influenza virus strain, H1N1, was shown to be responsible, a peak concentration of the Replikins in H1N1 independently occurred (P1); for the pandemic of 1957, where H2N2 emerged and was shown to be responsible, a peak concentration of the Replikins in H2N2 occurred (P2); and for the pandemic of 1968, where H3N2 emerged and was shown to be the cause of the pandemic, a peak concentration of the Replikins in H3N2 occurred (P3). (3) In the years immediately following each of the above three pandemics, the specific Replikin concentration decreased markedly, perhaps reflecting the broadly distributed immunity generated in each case. Thus, this post-pandemic decline is specific for H1N1 immediately following the pandemic (P1) for which it was responsible, and is not a general property of all strains at the time. An increase of Replikin concentration in influenza B repeatedly occurred simultaneously with the decrease in Replikin concentration in H1N1, e.g., EB1 in 1951 and EB2 in 1976, both associated with influenza B epidemics having the highest mortality. (Stuart-Harris, et al., Edward Arnold Ltd. (1985). (4) A secondary peak concentration, which exceeded the primary peak increase in concentration, occurred 15 years after each of the three pandemics, and this secondary peak was accompanied by an epidemic: 15 years after the 1918 pandemic in an H1N1 'epidemic' year (E1); eight years after the 1957 pandemic in an H2N2 'epidemic' year (E2); and occurred seven years after the 1968 pandemic in an H3N2 'epidemic' year (E3). These secondary peak concentrations of specific Replikins may reflect recovery of the strain. (5) Peaks of each strain's specific Replikin concentration frequently appear to be associated with declines in Replikin concentration of one or both other strains, suggesting competition between strains for host sites. (6) There is an apparent overall tendency for the Replikin concentration of each strain to decline over a period of 35 years (H2N2) to 60 years (influenza B). This decline cannot be ascribed to the influence of vaccines because it was evident in the case of influenza B from 1901 to 1964, prior to common use of influenza vaccines. In the case of influenza B, Replikin recovery from the decline is seen to occur after 1965, but Replikin concentration declined again between 1997 and 2000 (Figure 7), and this correlates with the low occurrence of influenza B in recent case isolates. H1N1 Replikin concentration peaked in 1978-1979 (**Figure 7**) together with the reappearance and prevalence of the H1N1 strain, and then peaked in 1996 coincident with an H1N1 epidemic. (**Figure 7**). H1N1 Replikin concentration also declined between 1997 and 2000, and the presence of H1N1 strains decreased in isolates obtained during these years. For H2N2 Replikins, recovery from a 35 year decline has not occurred (**Figure 8**), and this correlates with the absence of H2N2 from recent isolates. For H3N2, the Replikin concentration of many isolates fell to zero during the period from 1996 to 2000, but other H3N2 isolates showed a significant, sharp increase in Replikin concentration. This indicates the emergence of a sub-strain of H3N2, which is designated herein as H3N2(R).

**Figures 7** and **8** demonstrate that frequently a one to three year stepwise increase is observed before Replikin concentration reaches a peak. This stepwise increase proceeds the occurrence of an epidemic, which occurs concurrently with the Replikin peak. Thus, the stepwise increase in concentration of a particular strain is a signal that that particular strain is the most likely candidate to cause an epidemic or pandemic.

Currently, Replikin concentration in the H3N2(R) strain of influenza virus is increasing (**Figure 8**, 1997 to 2000). Three similar previous peak increases in H3N2 Replikin concentration are seen to have occurred in the H3N2-based pandemic of 1968 (Figure 8), when the strain first emerged, and in the H3N2-based epidemics of 1972 and 1975 (**Figure 8**). Each of these pandemic and epidemics was associated with excess mortality. (Ailing, et al., Am J. Epidemiol.,113(1):30-43 (1981). The rapid ascent in concentration of the H3N2(R) subspecies of the H3N2 Replikins in 1997-2000, therefore, statistically represents an early warning of an approaching severe epidemic or pandemic. An H3N2 epidemic occurred in Russia in 2000 (**Figure 8**, E4); and the CDC report of December 2001 states that currently, H3N2 is the most frequently isolated strain of influenza virus world wide. (Morbidity and Mortality Weekly Reports (mMVVR), Center for Disease Control; 50(48):1084-68 (Dec.7, 2001).

In each case of influenza virus pandemic or epidemic new Replikins emerge. There has been no observation of two of the same Replikins in a given hemagglutinin in a given isolate. To what degree the emergence of a new Replikin represents mutations versus transfer from another animal or avian pool is unknown. In some cases, each year one or more of the original Replikin structures is conserved, while at the same time, new Replikins emerge. For example, in influenza virus B hemagglutinin, five Replikins were constantly conserved between 1919 and 2001, whereas 26 Replikins came and went during the same period (some recurred after several years absence). The disappearance and re-emergence years later of a particular Replikin structure suggests that the Replikins return from another virus host pool rather than through *de novo* mutation.

In the case of H1N1 Replikins, the two Replikins present in the P 1 peak associated with the 1918 pandemic were not present in the recovery E1 peak of 1933, which contains 12 new Replikins. Constantly conserved Replikins, therefore, are the best choice for vaccines, either alone or in combination. However, even recently appearing Replikins accompanying one year's increase in concentration frequently persist and increase further for an additional one or more years, culminating in a concentration peak and an epidemic, thus providing both an early warning and time to vaccinate with synthetic Replikins (see for example, H1N1 in the early 1990's, **Figure 7**).

The data in **FIGURES 7** **and** **8** demonstrate a direct relationship between the presence and concentration of a particular Replikin in influenza protein sequences and the occurrence of pandemics and epidemics of influenza. Thus, analysis of the influenza virus hemagglutinin protein sequence for the presence and concentration of Replikins provides a predictor of influenza pandemics and/or epidemics, as well as a target for influenza vaccine formulation.

**Composition of Replikins in Strains of Influenza Virus B: Of a total** of 26 Replikins identified in this strain (**Table 3**), the following ten Replikins are present in every influenza B isolate examined from 1902-2001. Overlapping Replikin sequences are listed separately. Lysines and histidines are in bold type to demonstrate homology consistent with the "3-point recognition."
kshfanlk (SEQ ID NO. 104)
kshfanlkgtk (SEQ ID NO. 105)
kshfanlkgtktrgklcpk (SEQ ID NO. 106)
hekygglnk (SEQ ID NO. 107)
hekygglnksk (SEQ ID NO. 108)
hekygglnkskpyytgehak (SEQ ID NO. 10)
hakaigncpiwvk (SEQ ID NO. 110)
hakaigncpiwwkktplklangtk (SEQ ID NO. 111)
hakaigncpiwvktplklangtkyrppak (SEQ ID NO. 112)
hakaigncpiwvktplk1angtkyrppakllk (SEQ ID NO. 113)

**Tables 3 and 4** indicate that there appears to be much greater stability of the Replikin structures in influenza B hemagglutinins compared with H1N1 Replikins. Influenza B has not been responsible for any pandemic, and it appears not to have an animal or avian reservoirs. (Stuart-Hairis et al., Edward Arnold Ltd., London (1985)).

Influenza H1N1 Replikins: Only one replikin "hp(v/i)tigecpkyv(r/k)(s/t)(t/a)k" is present in every H1N1 isolate for which sequences are available from 1918, when the strain first appeared and caused the pandemic of that year, through 2000. (**Table 4**). ("(v/i)" indicates that the amino acid v or i is present in the same position in different years.) Although H1N1 contains only one persistent replikin, H1N1 appears to be more prolific than influenza B. There are 95 different replikin structures in 82 years on H1N1 versus only 31 different Replikins in 100 years of influenza B isolates (**Table 4**). An increase in the number of new Replikin structures occurs in years of epidemics (**Tables 3, 4, 5 and 6**) and correlates with increased total Replilcin concentration (**Figures 7** **and** **8**).

**Influenza H2N2 Replikins:** Influenza H2N2 was responsible for the human pandemic of 1957. Three of the 20 Replikins identified in that strain for 1957 were conserved in each of the H2N2 isolates available for examination on PubMed until 1995 **(Table 5**).
ha(k/q/m)(d/n)ilekt**h**ngk (SEQ ID NO. 232)
ha(k/q/m)(d/n)ilekt**h**ngklc(k/r) (SEQ ID NO. 233)
kgsnyp(v/i)ak(g/r)synntsgeqmliiwq(v/i)h (SEQ ID No. 238)

However, in contrast to H1N1, only 13 additional Replikins have been found in H2N2 beginning in 1961. This paucity of appearance of new Replikins correlates with the decline in the concentration of the H2N2 Replikins and the appearance of H2N2 in isolates over the years. (**Figure 8**).

**Influenza H3N2 Replikins:** Influenza H3N2 was responsible for the human pandemic of 1968. Five Replikins which appeared in 1968 disappeared after 1977, but reappeared in the 1990s (Table 6). The only Replikin structure which persisted for 22 years was hcd(g/q)f(q/r)nelcwdlf(v/i)er(slt)k, which appeared first in 1977 and persisted through 1998. The emergence of twelve new H3N2 replikins in the mid 1990s (**Table 6**) correlates with the increase in Replikin concentration at the same time **(****Figure 8**), and with the prevalence of the H3N2 strain in recent isolates. together with the concurrent disappearance of all Replikins from some of these isolates (**Figure. 8**), this suggests the emergence of the new substrain H3N2(R).

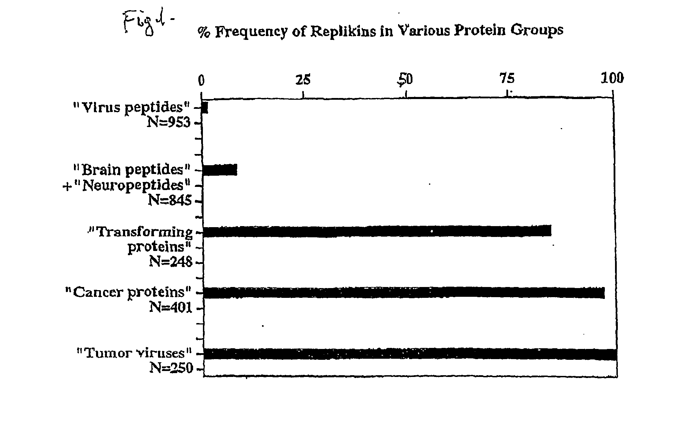

Figures 1 and 2 show that influenza epidemics and pandemics correlate with the increased concentration of replikins in influenza virus, which is due to the reappearance of at least one replikin from one to 59 years after its disappearance. Also, in the A strain only, there is an emergence of new strain-specific Replikin compositions (Tables 4-6). Increase in Replikin concentration by repetition of individual replikins within a single protein appears not to occur in influenza virus, but is seen in other organisms.

It has been believed that changes in the activity of different influenza strains are related to sequence changes in influenza hemagglutinins, which in turn are the products of substitutions effected by one of two poorly understood processes:
i) antigenic drift, thought to be due to the accumulation of a series of point mutations in the hemagglutinin molecule, or ii) antigenic shift, in which the changes are so great that genetic reassortment is postulated to occur between the viruses of human and non-human hosts. First, the present data suggests that the change in activity of different influenza strains, rather than being related to non-specific sequence changes, are based upon, or relate to the increased concentration of strain-specific replikins and strain-specific increases in the replication associated with epidemics. In addition, the data were examined for a possible insight into which sequence changes are due to "drift" or "shift", and which due to conservation, storage in reservoirs, then reappearance. The data show that the epidemic-related increase in replikin concentration is not due to the duplication of existing replikins per hemagglutinin, but is due to the reappearance of at least one replikin composition from 1 to up to 59 years after its disappearance, plus in the A strains only, the emergence of new strain-specific replikin compositions (Tables 3-6). Thus the increase in replikin concentration in the influenza B epidemics of 1951 and 1977 are not associated with the emergence of new replikin compositions in the year of the epidemic but only with the reappearance of replikin compositions which had appeared in previous years then disappeared (Table 3). In contrast, for the A strains, in addition to the reappearance of previously disappeared virus replikins, new compositions appear (e.g. in H1N1 in the year of the epidemic of 1996, in addition to the reappearance of 6 earlier replikins, 10 new compositions emerged). Since the A strains only, not influenza B, have access to non-human animal and avian reservoirs, totally new compositions probably derive from non-human host reservoirs rather than from mutations of existing human replikins which appear to bear no resemblance to the new compositions other than the basic requirements of "3-point recognition" (Tables 2-5). The more prolific nature of H1N1compared with B, and the fact that pandemics have been produced by the three A strains only, but not by the B strain, both may also be a function of the ability of the human A strains to receive new replikin compositions from non-human viral reservoirs.

Some replikins have appeared in only one year, disappeared, and not reappeared to date (Tables 3-6). Other replikins disappear for .from one to up to 81 years, when the identical replikin sequence reappears. Key replikin'k' and'h' amino acids, and the spaces between them, are conserved during the constant presence of particular replikins over many years, as shown in Tables 23-6for the following strain-specific replikins: ten of influenza B, the single replikin of H1N1, and the single replikin of H2N3, as well as for the reappearance of identical replikins after an absence. Despite the marked replacement or substitution activity of other amino acids both inside the replikin structure and outside it in the rest of the hemagglutinin sequences, influenza replikin histidine (h) appears never to be, and lysine (k) is rarely replaced. Examples of this conservation are seen in the H1N1 replikin"hp(v/i)tigecpkyv(r/k)(s/t)(t/a)k," (SEQ ID NO. 135) constant between 1918 and 2000, in the H3N2 replikin "hcd(g/q)f(q,r)nekwdlf(v/i)er(s/t)lc" (SEQ ID NO. 277) constant between 1975 and 1998 and in the H3N2 replikin "**h**qn(s/e)(e/q)g(t/s)g(q/y)aad(1/q)kstq(a/n)a(i/l)d(q/g)I(n/t)(g/n)k,(1/v)n(r/s )vi(e/c)k" (SEQ ID NO. 276) which first appeared in 1975, disappeared for 25 years, and then reappeared in 2000. While many amino acids were substituted, the basic replikin structure of 2 Lysines, 6 to 10 residues apart, one histidine, a minimum of 6% lysine in not more than approximately 50 amino acids, was conserved.

Totally random substitution would not permit the persistence of these H1N1 and H3N2 replikins, nor from 1902 to 2001 in influenza B the persistence of 10 replikin structures, nor the reappearance in 1993 of a 1919 18mer replikin after an absence of 74 years. Rather than a random type of substitution, the constancy suggests an orderly controlled process, or in the least, protection of the key replikin residues so that they are fixed or bound in some way: lysines, perhaps bound to nucleic acids, and histidines, perhaps bound to respiratory redox enzymes. The mechanisms which control this conservation are at present unknown.

Whether the conservation of replikin structures is unique to influenza or occurs in other virus replikins was examined in foot and mouth disease virus (FMDV) isolates, where extensive mutations in proteins of this virus have been well-documented worldwide over decades. In the protein VP1 of FMDV type 0, the replikin "hkqkivapvk" (SEQ ID NO. 3) was found to be conserved in 78% of the 236 isolates reported in PubMed, and each amino acid was found to be conserved in individual isolates as follows: h,95.6%; k,91.8%; q,92.3%; k,84.1%; i,90.7%; v,91.8%; a..97.3%; p,96.2%; a,75.4%; k,88.4%. Similarly, conservation was observed in different isolates of HIV for its replikins such as "kcfncgkegh" (SEQ ID NO. 5) or "kvylawvpahk" (SEQ ID NO. 6) in HIV Type 1 and "kcwncgkegh" (SEQ ID NO. 7) in HIV Type 2¹⁶. The high rate of conservation observed in FMVD and HIV replikins suggests that conservation observed in influenza replikins is a general property of viral replikins.

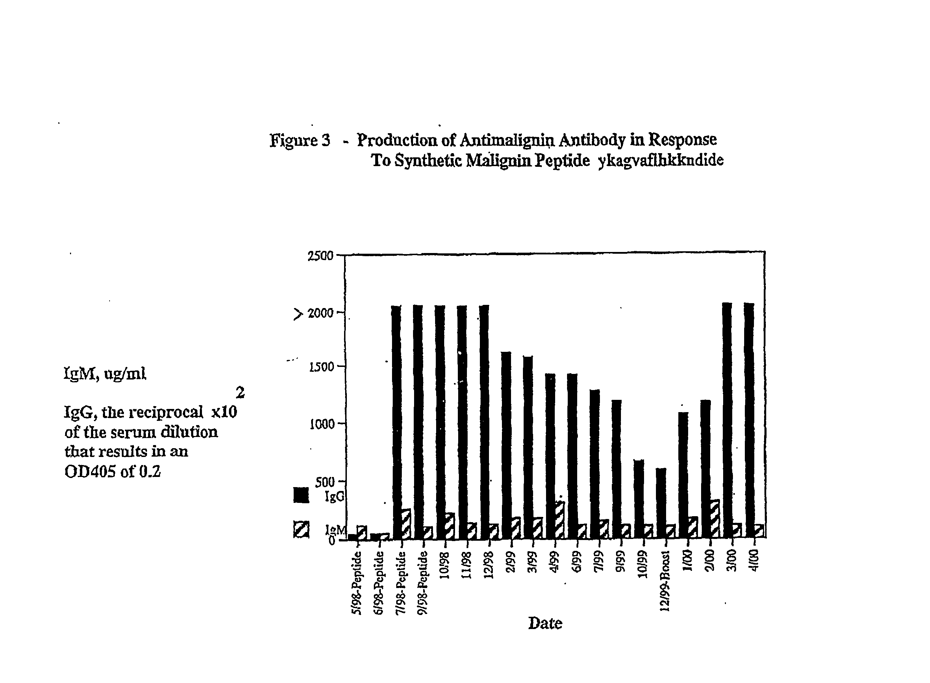

Data on anti-Replikin antibodies also support Replikin class unity. An anti-Replikin antibody response has been quantified by immunoadsorption of serum antimalignin antibody to immobilized malignin (see Methods in U.S. Patent #5,866,690). The abundant production of antimalignin antibody by administration to rabbits of the synthetic version of the 16-mer peptide whose sequence was derived from malignin, absent carbohydrate or other groups, has established rigorously that this peptide alone is an epitope, that is, it is a sufficient basis for this immune response (Figure 3). The 16-mer peptide produced both IgM and IgG forms of the antibody. Antimalignin antibody was found to be increased in concentration in serum in 37% of 79 cases in the U.S. and Asia of hepatitis B and C, early, in the first five years of infection, long before the usual observance of liver cancer, which develops about fifteen to twenty-five years after infection. Relevant to both infectious hepatitis and HIV infections, transformed cells may be one form of safe haven for the virus: prolonging cell life and avoiding virus eviction, so that the virus remains inaccessible to anti-viral treatment.

Because administration of Replikins stimulates the immune system to produce antibodies having a cytotoxic effect, peptide vaccines based on the particular influenza virus Replikin or group of Replikins observed to be most concentrated over a given time period provide protection against the particular strain of influenza most likely to cause an outbreak in a given influenza season., e.g., an emerging strain or re-emerging strain For example, analysis of the influenza virus hemagglutinin amino acid sequence on a yearly or bi-yearly basis, provides data which are useful in formulating a specifically targeted influenza vaccine for that year. It is understood that such analysis may be conducted on a region-by-region basis or at any desired time period, so that strains emerging in different areas throughout the world can be detected and specifically targeted vaccines for each region can be formulated.

Currently, vaccine formulations are changed twice yearly at international WHO and CDC meetings. Vaccine formulations are based on serological evidence of the most current preponderance of influenza virus strain in a given region of the world. However, prior to the present invention there has been no correlation of influenza virus strain specific amino acid sequence changes with occurrence of influenza epidemics or pandemics.

The observations of specific Replikins and their concentration in influenza virus proteins provides the first specific quantitative early chemical correlates of influenza pandemics and epidemics and provides for production and timely administration of influenza vaccines tailored specifically to treat the prevalent emerging or re-emerging strain of influenza virus in a particular region of the world. By analyzing the protein sequences of isolates of strains of influenza virus, such as the hemagglutinin protein sequence, for the presence, concentration and/or conservation of Replikins, influenza virus pandemics and epidemics can be predicted. Furthermore, the severity of such outbreaks of influenza can be significantly lessened by administering an influenza peptide vaccine based on the Replikin sequences found to be most abundant or shown to be on the rise in virus isolates over a given time period, such as about one to about three years.

An influenza peptide vaccine of the invention may include a single Replikin peptide sequence or may include a plurality of Replikin sequences observed in influenza virus strains. Preferably, the peptide vaccine is based on Replikin sequence(s) shown to be increasing in concentration over a given time period and conserved for at least that period of time. However, a vaccine may include a conserved Replikin peptide(s) in combination with a new Replikin(s) peptide or may be based on new Replikin peptide sequences. The Replikin peptides can be synthesized by any method, including chemical synthesis or recombinant gene technology, and may include non-Replikin sequences, although vaccines based on peptides containing only Replikin sequences are preferred. Preferably, vaccine compositions of the invention also contain a pharmaceutically acceptable carrier and/or adjuvant.

The influenza vaccines of the present invention can be administered alone or in combination with antiviral drugs, such as gancyclovir; interferon; interleukin; M2 inhibitors, such as, amantadine, rimantadine; neuraminidase inhibitors, such as zanamivir and oseltamivir; and the like, as well as with combinations of antiviral drugs.

Analysis of the primary structure of a *Plasmodium farciparum* malaria antigen located at the merozoite surface and/or within the parasitophorous vacuole revealed that this organism, like influenza virus, also contains numerous Replikins. However, there are several differences between the observation of Replikins in *Plasmodium falciparum* and influenza virus isolates. For example, *Plasmodium falciparum* contains several partial Replikins, referred to herein as "Replikin decoys." These decoy structures contain an abundance of lysine residues, but lack the histidine required of Replikin structures. It is believed that the decoy structure maximizes the chances that an anti-malarial antibody or other agent will bind to the relatively less important structure containing the lysines, i.e., the Replikin decoys, rather than binding to histidine, which is present in Replikin structure, such as replikins in respiratory enzymes, which could result in destruction of the trypanosome.

Another difference seen in *Plasmodium falciparum* is a frequent repetition of individual Replikin structures within a single protein, which was not observed with influenza virus. Repetition may occur by (a) sharing of lysine residues between Replikins, and (b) by repetition of a portion of a Replikin sequence within another Replikin sequence.

A third significant difference between Replikin structures observed in influenza virus isolates and *Plasmodium falciparum* is a marked overlapping of Replikin structures throughout malarial proteins, e.g., there are nine overlapping replikins in the 39 amino acid sequence of SEQ ID NO. 393 (Replikin concentration = 23.1/100 amino acids); and 15 overlapping replikins in the 41 amino acids of SEQ ID NO. 467 (Replikin concentration = 36.6/100 amino acids). Both of these overlapping Replikin structures occur in blood stage trophozoites and schizonts. In contrast, influenza virus Replikins are more scattered throughout the protein and the maximum Replikin concentration is about 7.5/100 amino acids **(****Figure 7****)**; and tomato leaf curl gemini virus, which was also observed to have overlapping replikins has only about 3.1/100 amino acids.

This mechanism of lysine multiples is also seen in the Replikins of cancer proteins such as in gastric cancer transforming protein, ktkkgnrvsptmkvth (SEQ ID NO. 88), and in transforming protein P21B (K-RAS 2B) of lung, kbkekmskdgkkkkkks (SEQ ID NO. 89).

The relationship of higher Replikin concentration to rapid replication is also confirmed by analysis of HIV isolates. It was found that the slow-growing low titer strain of HIV (NSI, "Bru", which is prevalent in early stage HIV infection has a Replikin concentration of 1.1 I (+/- 1 .6) Replikins per 100 amino acids, whereas the rapidly-growing high titer strain of HIV (S1, "Lai"), which is prevalent in late stage HIV infection has a Replikin concentration of 6.8 (+/- 2.7) Replikins per 100 amino acid residues.

The high concentration of overlapping Replikins in malaria, influenza virus and cancer cells is consistent with the legendary high and rapid replicating ability of malaria organisms. The multitude of overlapping Replikins in malaria also provides an opportunity for the organism to flood and confuse the immune system of its host and thereby maximize the chance that the wrong antibody will be made and perpetuated, leaving key malaria antigens unharmed.

As in the case of influenza virus, for example, peptide vaccines based on the Replikin structure(s) found in the malaria organism can provide an effective means of preventing and/or treating malaria. Vaccination against malaria can be - achieved by administering a composition containing one or a mixture of Replikin structures observed in *Plasmodiulm falciparum.* Furthermore, antibodies to malaria Replikins can be generated and administered for passive immunity or malaria detection purposes.

Table 7 provides a list of several *Plasmodium falciparum* Replikin sequences. It should be noted that this list is not meant to be complete. Different isolates of the organism may contain other Replikin structures.

**Table 7**

| **Malaria replikins** |
|---|
| **a) Primary structure of a Plasmodium falciparum malaria antigen located at the merozoite surface and within the parasitophorous vacuole** |
| |
| **a) i) DECOYS:** |
| (C-Terminal) |
| keeeekekekekekeekelceekekeekekekeekekekeekeeekk (SEQ ID NO. 293), or |
| keeeekekekekekeekekeekekeekekekeekekekeekeeekkek (SEQ ID NO. 294), or |
| keeeekekekekekeekekeekekekeekekeekekeekeekeeekk (SEQ ID NO. 295), or |
| keeeekekek (SEQ ID NO. 296) |
| |

| **ii) REPLIKINS:** |
|---|
| Hkklikalkkniesiqnkk (SEQ ID NO. 297) |
| hkklikalkkmesiqnkm (SEQ lD NO. 298) |
| bkklikalkk (SEQ ID NO. 299) |
| hkklikalk (SEQ ID NO. 300) |
| katysfvntkkldislksqghkk (SEQ ID NO. 301) |
| katysfvntkkkiislksqghk (SEQ ID NO. 302) |

| |
|---|
| katysfvntkkkiislksqgh (SEQ ID NO. 303) |
| htyvkgkkapsdpqca dilceeckellkelc (SEQ ID NO. 304) |
| kiislksqghk (SEQ ID NO. 305) |
| kkkkfeplkngnvsetiklih (SEQ ID NO. 306) |
| kkkfeplkngnvsetiklih (SEQ ID NO. 307) |
| kkfeplkngnvsetiklih (SEQ ID NO. 308) |
| kngnvsetiklih (SEQ ID NO. 309) |
| klihignkdkk (SEQ ID NO. 310) |
| kvkkigvtlkkfeplkngnvsetilhihignkdkkh (SEQ ID NO. 311) |
| hliyknksynplllscvkkmnmlkenvdyiqnqnlfkelmnqkatysfvntkkkiislk (SEQ ID NO. 312) |
| hliylcnksynplllscvklanntnllcenvdyiqnqnlffcelmnqkatysfvntk (SEQ ID NO. 313) |
| hliyknksynplllscvkkmnnlkenvdyiqnqnlfkehnnqk (SEQ lD NO. 314) |
| hliyknksynplllsykkmnmlkenvdyiqknqnlfk (SEQ ID NO. 315) |
| hliyknksynplllscvkkmnmlk (SEQ ID NO. 316) |
| ksannsanngklamaeemknlvnflqshkklikalkkniesiqnkkh (SEQ ID NO. 317) |
| klamaeemlmlvnflqshkklikalklkkniesiqnkkh (SEQ ID NO. 318) |
| knivnilqshkklikalkkniesiqnkkh (SEQ ID NO. 319) |
| kklikalkkniesiqnkkh (SEQ ID NO. 320) |
| klikalkkniesiqnkkh (SEQ ID NO. 321) |
| kkniesiqnkkh (SEQ ID NO. 322) |
| kniesiqnkkh (SEQ ID NO. 323) |
| knnaeemknlvnflqsh (SEQ ID NO. 324) |
| kklikakkniesiqnkkqghkk (SEQ ID NO. 325) |
| kknnaeemknlcnlvnflqshk (SEQ ID NO. 326) |
| knnaeemknlvnflqsh (SEQ ID NO. 327) |
| klikalkkniesiqnkkqghkk (SEQ ID NO. 328) |
| kvkkigvtlkkfeplkngnvsetiklih (SEQ ID NO. 329) |
| kngnvsetiklih (SEQ ID NO. 330) |
| klihlgnkdkk (SEQ ID NO. 331) |
| ksannsanngkknnaeemkmlvnflqsh (SEQ ID NO. 332) |
| kknnaeemknlvnflqsh (SEQ ID NO. 333) |
| klclikalkkniesiqnkkch (SEQ ID NO. 334) |
| kalkkniesiqnkkh (SEQ ID NO. 335) |
| kkniesiqnkkh (SEQ ID NO. 336) |
| kelmnqkatysfvntkkkiislksqgh (SEQ ID NO. 337) |
| ksqghkk (SEQ ID NO. 338) |
| kkkiislksqgh (SEQ ID NO. 339) |
| kkiislksqgh (SEQ ID NO. 340) |
| kkniesiqnkkh (SEQ ID NO. 341) |
| kniesiqnkkh (SEQ ID NO. 342) |
| htyvkgkkapsdpqcadikeeckellkek (SEQ ID NO. 343) |
| htyvlcgkkapsdpqcadikeeckellk (SEQ ID NO. 344 ) |

| **b)"liver stage antigen-3" " gene="LSA-3" Replikins** |
|---|
| henvlsaalentqseeekkevidvieevk (SEQ ID NO. 345) |
| kenvvttilckveeftaesvttfsnfleeiqentitndtieeldeelh (SEQ lD NO. 346) |
| hylqqmkekfskek (SEQ ID NO. 347) |
| hylqqmkekfskeknnnvievtnkaekkgnvqvtnktekttk (SEQ ID NO. 348) |
| hylqqmkekfskeknnnvievtnkaekkgnvqvtnktekttkvdknnk (SEQ ID NO. 349) |
| hylqqmkekfskeknnnvievtnkaekkgnvqvtnvqttkvdknnkvpkkrrqk (SEQ ID NO. 350) |
| hylqqmkekfskeknnnvievtnkaekkgnvqvtnktekttkvdknnkvpkkrrtqksk (SEQ ID NO. 351) |
| hvdevmkyvqkidkevdkevskaleskndvtnvlkqnqdffskvknfvkkyk (SEQ ID NO. 352) |
| hvdevmkyvqkidkevdkevskaleskndvtnvlkqnqfffskvlmfvkk (SEQ ID NO. 353) |
| hvdevmkyvqkidkevdkevskaleskndvtnvlkqnqdffsk (SEQ ID NO. 354) |
| hvdevmkyvqkidkevdkevskaleskndvtnvlk (SEQ ID NO. 355) |
| hvdevinkyvqkidkevdkevskalesk (SEQ ID NO. 356) |
| hvdcvmkyvqkidkevdkevsk (SEQ ID NO. 357) |
| hvdevmkyvqkidkevdk (SEQ ID NO. 358) |
| hvdevmkyvqkidk (SEQ ID NO. 359) |
| kdevidlivqkekriekvkakkklekkveegvskkh (SEQ ID NO. 360) |
| kvkakkkklekkveegvsglkkh (SEQ ID NO. 361) |
| kakkkklekkveegvsglkkh (SEQ ID NO. 362) |
| kkkklekkveegvsglkkh (SEQ ID NO. 363) |
| kkklekkveegvsglkkh (SEQ ID NO. 364) |
| kklekkveegvsglkkh (SEQ ID NO. 365) |
| klekkveegvsglkkh (SEQ ID NO. 366) |
| kkveegvsglkkh (SEQ ID NO. 367) |
| kveegvsglkkh (SEQ ID NO.368) |
| hveqnvyvdvdvpamkdqflgilneagglkemffnledvfksesdvitveeikdepvqk (SEQ ID NO. 369) |
| hikgleeddleevddlkgsildmlkgdmelgdmdkesledvttklgerveslk (SEQ ID NO. 370) |
| hikgleeddleevddlkgsildmlkgdmelgdmdkesledvttk (SEQ ID NO. 371) |
| hikgleeddleevddlkgsildmlkgdmelgdmdk (SEQ ID NO. 372) |
| hilcgleeddleevddlkgsildmlk (SEQ ID NO. 373) |
| hiisgdadvlssalgmdeeqmktrldcaqrpk (SEQ ID NO. 374) |
| hditttldevvelkdveedkiek (SEQ ID NO. 375) |

| |
|---|
| kkleevhelk (SEQ ID NO. 376) |
| kleevhelk (SEQ ID NO. 377) |
| ktietdileekkkeiekdh (SEQ ID NO. 378) |
| kkeiekdhfek (SEQ ID NO. 379) |
| kdhfek (SEQ ID NO. 380) |
| kfeeeaeeikh (SEQ ID NO. 381) |
| |

| **c) 28 KDA ookinete surface antigen precursor Replikins:** |
|---|
| kdgdtkctlecaqgkkcikhksdhnhksdhnhksdpnhkkknnnnk (SEQ ID NO. 382) |
| kdgdtkctlecaqgkkcikhksdhnhksdhnhksdpnhkk (SEQ.ID NO. 383) |
| kdgdtkctlecaqgkkcikhksdhnhksdhnhksdpnhk (SEQ ID NO. 384) |
| kdgdtkctlecaqgkkcikhksdhnhksdhnhk (SEQ ID NO. 385) |
| kdgdtkctlecaqgkkcikhksdhnhk (SEQ ID NO. 386) |
| kdgdtkctlecaqgkkcikhk (SEQ ID NO. 387) |
| kdgdtkctlecaqgkk (SEQ ID NO. 388) |
| kdgdtkctlecaqgk (SEQ ID NO. 389) |
| kciqaecnykecgeqkcvwdgih (SEQ lD NO. 390) |
| kecgeqkcvwdgih (SEQ ID NO. 391) |
| hieckcnndyvltnryecepknkctsledtnk (SEQ ID NO. 392) |
| |

| **d) Blood stage trophozoites and schizonts Replikins:** |
|---|
| ksdhnhksdhnhksdhnhksdhnksdpnhkkknnnnnk (SEQ ID NO. 393) |
| ksdhnhksdhnhksdhnhksdpnhkkknnnk (SEQ ID NO. 394) |
| ksdhnhksdhnhksdpnhkkknnnmk (SEQ ID NO. 395) |
| ksdhnhksdpnhkkknnnnnnk (SEQ ID NO. 396) |
| kkknnnnnkdnksdpnhk (SEQ ID NO. 397) |
| kknnnnnkdnksdpnbk (SEQ ID NO. 398) |
| knnnnnnkdnksdpnhk (SEQ ID NO. 399) |
| kdnksdpnhk (SEQ ID NO. 400) |
| ksdpnhk (SEQ ID NO. 401) |
| hslyalqqneeyqkvkuekdqneikkkqlieknk (SEQ ID NO. 402) |

| |
|---|
| hslyalqqneeyqkvknekdqneikkik (SEQ ID NO. 403) |
| hslyalqqneeyqkvknekdqneikk (SEQ ID NO. 404) |
| hslyalqqneeyqkvknekdqneik (SEQ ID NO. 405) |
| hklenleemdk (SEQ ID NO. 406) |
| khfddntneqk (SEQ ID NO. 407) |
| kkeddekh (SEQ ID NO. 408) |
| keennkkeddekh (SEQ ID NO. 409) |
| ktssgilnkeennkkeddekh (SEQ ID NO. 410) |
| knihikk (SEQ ID NO. 411) |
| hikkkegidigyk (SEQ ID NO. 412) |
| kkmwtcklwdkgneitknith (SEQ 1D NO. 413 ) |
| kkgiqwnlllckmwtcldwdnkgneitknih (SEQ ID NO. 414 ) |
| kekkdsnenrkkkqkedkknpnklkieytnkithffkakrmkqqnnvth (SEQ ID NO. 415) |
| kkdsnenrkkkqkedkknpnkllcldeytnkithfflcaknnkqqnnvth (SEQ ID NO. 416) |
| kdsnenrkkkqkedkknpnklkkieytnkithffkaknnkqqnnvth (SEQ ID NO. 417) |
| kkqlcedkknpnklkkieytnkithffkaknnkqqnnvth (SEQ ID NO. 418) |
| kqkedklrnpnlcllckieytnkithfflcaknnkqqnnvth (SEQ ID NO. 419) |
| kedkknpnklkkieytnkithffkaknnkqqnnvth (SEQ ID NO. 420) |
| knpnklkkieytnkithffkaknnkqqnnvth (SEQ ID NO. 421) |
| kkieytnkithfflcaknnkqqnnvth (SEQ ID NO. 422) |
| kieytnkithffkaknnkqqnnvth (SEQ ID NO. 423) |
| kithffkaknnkqqnnvth (SEQ ID NO. 424) |
| hknnedikndnskdilrnchiskdikndnskdikndnnedikndnskdik (SEQ ID NO. 425 ) |
| hknnedikndnskdiknchiskdilrndnskdikndnnedikndnslc (SEQ ID NO. 426) |
| hbmediknduskdiknduskdilmduskdikndnnedik (SEQ ID NO. 427) |
| hknnedikndnskdikndnskdilmdnskdik (SEQ ID NO. 428) |
| hknnedikndnskdikndnskdikndnsk (SEQ ID NO. 429) |
| hkunedikndnskdikndnskdik (SEQ ID NO. 430) |
| hknnedikndnskdikndnsk (SEQ ID NO. 431) |
| hknnedikndnskdik (SEQ ID NO. 432) |
| hknnedik (SEQ ID NO. 433) |
| kkyddlqnkynilnklknsleekneelkkyh (SEQ ID NO. 434) |
| kyddlqnkynilnklknsleekneelkkyh (SEQ ID NO. 435) |
| kynilnkklnsleelmeelkkyh (SEQ ID NO. 436) |
| kflnsleekneelkkyh (SEQ ID NO. 437) |
| knsleekneelkkyh (SEQ ID NO. 438) |
| kneelkkyh (SEQ ID NO. 439) |
| hmgnnqdinenvynikpqefkeeeeedismvntkk (SEQ ID NO. 440) |
| knsnelkrindnffklh (SEQ ID NO. 441) |
| kpclykkckisqclykkckisqvwwcmpvkdtfntyernnvlnskienniekiph (SEQ ID NO. 442) |
| hinneytnknpkncllykneernyndnnikdyinsmnfkk (SEQ m NO. 443) |
| hinneytnknpknellykneemyndnnikdyinsmnfk (SEQ m NO. 444) |
| hinneytnknpkncllyk (SEQ ID NO. 445) |
| knktnqskgvkgeyekkketngh (SEQ ill NO. 446) |
| ktnqskgvkgeyekkketngh (SEQ m NO. 447) |
| kgvkgeyekkketngh (SEQ ID NO. 448) |
| kgeyekkketngh (SEQ m NO. 449) |
| ksgmytnegnkscecsykkkssssnkvh (SEQ ID NO. 450) |
| kscecsykkkssssnkvh (SEQ ID NO. 451) |
| kkkssssnkvh (SEQ ID NO. 452) |
| kkssssnkvh (SEQ ID NO. 453) |
| kssssnkvh (SEQ m NO. 454) |
| himlksgmytnegnkscecsykkkssssnk (SEQ LID NO. 45S) |
| himlksgmytnegnkscecsykkk (SEQ m NO. 456) |
| himlksgmytnegnkscecsykk (SEQ ID NO. 457) |
| himlksgmytnegnkscecsyk (SEQ ID NO. 458) |
| kplaklrkrektqinktkyergdviidnteiqkiiirdyhetlnvhlddh (SEQ ID NO. 459) |
| krektqinktkyergdviidnteiqkiiirdyhetlnvhkldh (SEQ m NO. 460) |
| ktqinktkyergdviidnteiqkiiirdyhethlvhkldh (SEQ ID NO. 461) |
| kplaklrkrektqinktkyergdviidnteiqkiiirdyhetlnvh (SEQ ID NO. 462) |
| kplaklrkrektqinktkyergdviidnteiqkiiirdyh (SEQ ID NO. 463) |
| klrkrektqinktkyergdviidnteiqkiiirdyh (SEQ ID NO. 464) |
| krektqinktkyergdviidnteiqkiiirdyh (SEQ ID NO. 465) |
| ktqinktkyergdviidnteiqkiiirdyh (SEQ ID NO. 466) |
| kkdkekklcdsnenrkkkqkedkloepndnklkkieytnkith (SEQ ID NO. 467) |
| 1cdlcelckkdsnenrkkkqkedkloepndnlc1kkieytnldth (SEQ ID NO. 468) |
| kekkkdsnenrkkkqkedklmpndnklkkieytnkith (SEQ ID NO. 469) |
| kkkdsnenrkkkqkedkknpndnklkkieytnkith (SEQ ID NO. 470) |
| kkdsnenrkkkqkedkknpndnklkkieytnkith (SEQ ID NO. 471) |
| kdsnenrkkkqkedkknpndnklkkieytnkith (SEQ ID NO. 472) |
| kkkqkedkknpndnklkkieytnkith (SEQ ID NO. 473) |
| kkqkedklmpndnk1kkieytnkith (SEQ ID NO. 474) |
| kqkedkknpndnklkieytnkith (SEQ ID NO. 475) |
| kedkknpndnklkkieytnkith (SEQ ID NO. 476) |
| kknpndnklkkieytnkith (SEQ ID NO. 477) |
| knpndnklkkieytnkith (SEQ m NO. 478) |
| klkkieytnkith (SEQ ID NO. 479) |
| kkieytukith (SBQ ID NO. 480) |
| kieytnkith (SEQ ID NO. 481) |
| hgqikiedvnnenfnneqmknkyndeekmdiskskslksdflek (SEQ ID NO. 482) |
| hgqildedvnneofmeqmknkyndcekmdiskskslk (SEQ ID NO. 483) |
| hgqikiedvnnenfuneqmknkyndeekmdisksk (SEQ ID NO. 484) |
| hgqikiedvnnenfuneqmknkyndeelandisk (SEQ ID NO. 485) |
| kkyddlqnkynilniklknsleekneelkkyh (SEQ ID NO. 486) |
| kyddlqnkynilnklknsleekneelkkyh (SEQ ID NO. 487) |
| kynikklknsleekneekkyh (SEQ ID NO. 488) |
| klknsleekneelkkyh (SEQ ID NO. 489) |
| Knsleekneelkkyh (SEQ ID NO. 490) |
| kneelkkyh (SEQ ID NO. 491) |
| hmgniiqdmenvynikpqefkeeeeedismvntklccddiqenil< (SEQ ID NO. 492) |
| ktnlyniynnknddkdnildnenreglylcdvmlmsnelkrindnffklh (SEQ ID NO. 493) |
| knsnelkrindnffklh (SEQ ID NO. 494) |
| krindnffklh (SEQ ID NO. 495) |
| hinneytnknpkncllykneernyndnnikdyinsmnfkk (SEQ ID NO. 496) |
| hinneytukrpknalykneemyndrdkdyinsmnfk (SEQ ID NO. 497) |
| hinneytnknpkncllyk (SEQ ID NO. 498) |
| kpclykkckisqvwwcmpvkdtfntyernnvlnskieniekiph (SEQ ID NO. 499) |
| kckisqvwwcmpvkdtfntyernnvlnskienniekiph (SEQ ID NO. 500) |
| kienniekiph (SEQ ID NO. 501) |
| knktngskgvkgeyekkketngh (SEQ ID NO. 502) |
| ktngskgvkgeyekkketngh (SEQ ID NO. 503) |
| kgvkgeyekkketngh (SEQ ID NO. 504) |
| kgeyekkketngh (SEQ ID NO. 505) |
| ktiekinkskswffeeldeidkplaklrlaekiqinktkyergdviidnteiqkiirdyh (SEQ ID NO. 506) |
| kinkskswffeeldeidkplaklrkrektqinktkyergdviidnteiqkiirdyh (SEQ ID NO. 507) |
| kplaklrkrektqinktkyergdviidnteiqkiirdyh (SEQ ID NO. 508) |
| himlksqmytnegnkscecsyklckssssnkvh (SEQ ID NO. 509) |
| kh-laektqinktkyergdviidnteiqkiirdyh (SEQ ID NO. 510) |
| krektqinktkyergdviidnteiqkiirdyh (SEQ ID NO. 511) |
| ktqinktkyergdviidnteiqkiirdyh (SEQ ID NO. 512) |
| kplaklrkrektqinktkyergdviidnteiqkiirdyhtlnvhkldh (SEQ ID NO. 513) |
| klrlaektqinktkyergdviidnteiqkiirdyhtlnvhkldh (SEQ ID NO. 514) |
| krektqinktkyergdviidnteiqkiirdyhtlnvhkldh (SEQ ID NO. 515) |
| ktqinktlyergdviidnteiqkiirdyhtlnvhlddh (SEQ ID NO. 516) |
| kplaklrkrektqinlctkyergdviidnteiqlciirdyhtlnvh (SEQ ID NO. 517) |
| klrkrektqinktkyergdviidnteiqlciirdyhtlnvh (SEQ ID NO. 518) |
| krektqinktkyergdviidnteiqkiirdyhtlnvh (SEQ ID NO. 519) |
| ktqinktkyergdviidnteiqkiirdyhtlnvh (SEQ ID NO. 520) |
| himlksqmytnegnkscecsykkkssssnkvh (SEQ ID NO. 521) |
| ksqmytnegnkscecsykkkssssnkvh (SEQ ID NO. 522) |
| kscecsykkkssssnkvh (SEQ ID NO. 523) |
| kkkssssnkvh (SEQ ID NO. 524) |
| kkssssnkvh (SEQ ID NO. 525) |
| kssssnkvh (SEQ ID NO. 526) |
| himlksqmytnegnkscecsykkkssssnk (SEQ ID NO. 527) |
| himlksqmytnegnkscecsykkk (SEQ ID NO. 528) |
| himllcsqmytnegnkscecsykk (SEQ ID NO. 529) |
| himlksqmytnegnkscecsyk (SEQ ID NO. 530) |
| hnnhmqiykdimnmmpbkvmyhdmnsknertek (SEQ ID NO. 531) |
| hnnhmqiykdkrinfmnpbkvmyhdnmsk (SEQ ID NO. 532) |
| hnnhniqiykdkrinfmnphk (SEQ ID NO. 533) |
| hkvrnylidnmsknertek (SEQ ID NO. 534) |
| hkvmyhdnrnsk (SEQ ID NO. 535) |
| |

Other microorganisms found to contain numerous Replikin structures are *Bacillus anthracis,* the organism responsible for anthrax infections, in which eight different replikins were identified; and small pox virus, in which five different Replikins were identified. The eight *Bacillus anthracis* peptides are present in the Anthrax Toxin Lethal Factor Protein pX01-107 and have the amino acid sequence of SEQ ID NO. 91, SEQ ID NO.92, SEQ ID NO. 93, SEQ ID NO. 94, SEQ ID NO. 95, SEQ ID NO. 96, SEQ ID NO. 97 and SEQ ID NO. 98, respectively. The five small pox virus peptides are present in the Small Pox Virus Surface Antigen S Precursor Protein, which purportedly enhances Small Pox Virus replication. The five peptides have the amino acid sequence of SEQ ID NO. 99, SEQ ID NO. 100, SEQ ID NO.101, SEQ ID NO. 102 and SEQ ID NO. 103, respectively.

Synthetic Replikin vaccines, based on Replikins such as the glioma Replikin **(SEQ ID NO.: 1) "kagvaflhkk"** or the hepatitis C Replikin **(SEQ ID NO.: 18) "hyppkpgcivpak",** or HIV Replikins such as **(SEQ ID NO.: 5)** "kcfncgkegh" or **(SEQ ID NO.: 6) "kvylawvpahk"** or preferably, an influenza vaccine based on conserved and/or emerging or re-emerging Replikin(s) over a given time period may be used to augment antibody concentration in order to lyse the respective virus infected cells and release virus extracellularly where chemical treatment can then be effective. Similarly, a malaria vaccine, based on Replikins observed in *Plasmodium falciparum* malaria antigens on the merozoite surface or within the parasitophorous vacuole, for example, can be used to generate cytotoxic antibodies to malaria. Vaccines based on the Replikin structures identified in small pox virus, or *Bacillus anthracis,* or any pathological organism in which Replikins are identified, can be generated and administered to prevent the respective disease.
Recognin and/or Replikin peptides may be administered to a subject to induce the immune system of the subject to produce anti-Replikin antibodies. Generally, a 0.5 to about 2 mg dosage, preferably a 1 mg dosage of each peptide is administered to the subject to induce an immune response. Subsequent dosages may be administered if desired.

In another embodiment of the invention, isolated Replikin peptides may be used to generate antibodies, which may be used, for example to provide passive immunity in an individual. Passive immunity to the strain of influenza identified by the method of the invention to be the most likely cause of future influenza infections may be obtained by administering antibodies to Replikin sequences of the identified strain of influenza virus to patients in need. Similarly, passive immunity to malaria may be obtained by administering antibodies to *Plasmodium falciparum* Replikin(s); immunity to Small pox is achieved by administering antibodies to small pox virus Replin(s); immunity to anthrax is achieved by administering antibodies to *Bacillus anthracis* Replikin(s); and the like.

Various procedures known in the art may be used for the production of antibodies to Replikin sequences. Such antibodies include but are not limited to polyclonal, monoclonal, chimeric, humanized, single chain, Fab fragments and fragments produced by an Fab expression library. Antibodies that are linked to a cytotoxic agent may also be generated. Antibodies may also be administered in combination with an antiviral agent. Furthermore, combinations of antibodies to different Replikins may be administered as an antibody cocktail.

For the production of antibodies various host animals may be immunized by injection with a Replikin peptide or a combination of Replikin peptides, including but not limited to rabbits, mice, rats, and larger mammals. Various adjuvants may be used to enhance the immunological response, depending on the host species, including but not limited to Freund's (complete and incomplete), mineral gels, such as aluminum hydroxide, surface active substances such as lysolecithin, pluronic polyols, polyanions, peptides, oil emulsions, key limpet hemocyanin, dintrophenol, and potentially useful human adjuvants such as BCG and *Corynebacterium parvum.*

Monoclonal antibodies to Replikins may be prepared by using any technique that provides for the production of antibody molecules. These include but are not limited to the hybridoma technique originally described by Kohler and Milstein, (Nature, 1975, 256:495-497), the human B-cell hybridoma technique (Kosbor et al., 1983, Immunology Today, 4:72), and the EBV hybridoma technique (Cole et al., Monoclonal Antibodies and Cancer Therapy, Alan R. Liss, Inc., pp. 77-96). In addition, techniques developed for the production of chimeric antibodies (Morrison et al., 1984, Proc. Nat. Acad. Sci USA, 81:6851-6855) or other techniques may be used. Alternatively, techniques described for the production of single chain antibodies (US 4,946,778) can be adapted to produce Replikin-specific single chain antibodies.

Particularly useful antibodies of the invention are those that specifically bind to Replikin sequences contained in peptides and/or polypeptides of influenza virus. For example, antibodies to any of peptides observed to be present in an emerging or re-emerging strain of influenza virus and combinations of such antibodies are useful in the treatment and/or prevention of influenza. Similarly, antibodies to any replikins present on malaria antigens and combinations of such antibodies are useful in the prevention and treatment of malaria.

Antibody fragments which contain binding sites for a Replikin may be generated by known techniques. For example, such fragments include but are not limited to F(ab')₂ fragments which can be produced by pepsin digestion of the antibody molecules and the Fab fragments that can be generated by reducing the disulfide bridges of the F(ab')₂ fragments. Alternatively, Fab expression libraries can be generated (Huse et al., 1989, Science, 246:1275-1281) to allow rapid and easy identification of monoclonal Fab fragments with the desired specificity.

The fact that antimalignin antibody is increased in concentration in human malignancy regardless of cancer cell type (Figure 5), and that this antibody binds to malignant cells regardless of cell type now may be explained by the presence of the Replikin structures herein found to be present in most malignancies (Figure 1 and Table 2). Population studies have shown that antimalignin antibody increases in concentration in healthy adults with age, and more so in high-risk families, as the frequency of cancer increases. An additional two-fold or greater antibody increase which occurs in early malignancy has been independently confirmed with a sensitivity of 97% in breast cancers 1-10 mm in size. Shown to localize preferentially in malignant cells *in vivo,* histochemically the antibody does not bind to normal cells but selectively binds to **(****Figure 4a,b)** and is highly cytotoxic to transformed cells *in vitro* **(****Figure 4c****-f)**. Since in these examples the same antibody is bound by several cell types, that is, brain glioma, hematopoietic cells (leukemia), and small cell carcinoma of lung, malignant Replikin class unity is again demonstrated.

Antimalignin does not increase with benign proliferation, but specifically increases only with malignant transformation and replication in breast *in vivo* and returns from elevated to normal values upon elimination of malignant cells (Figure 5). Antimalignin antibody concentration has been shown to relate quantitatively to the survival of cancer patients, that is, the more antibody, the longer the survival. Taken together, these results suggest that anti-Replikin antibodies may be a part of a mechanism of control of cell transformation and replication. Augmentation of this immune response may be useful in the control of replication, either actively with synthetic Replikins as vaccines, or passively by the administration of anti-Replikin antibodies, or by the introduction of non-immune based organic agents, such as for example, carbohydrates, lipids and the like, which are similarly designed to target the Replikin specifically. For organisms such as diatom plankton, foot and mouth disease virus, tomato leaf curl gemini virus, hepatitis B and C, HIV, influenza virus and malignant cells, identified constituent Replikins are useful as vaccines, and also may be usefully targeted for diagnostic purposes. Blood collected for transfusions, for example, may be screened for contamination of organisms, such as HIV, by screening for the presence ofReplikins shown to be specific for the contamination organism. Also, screening for Replikin structures specific for a particular pathological organism, e.g., anthrax., leads to diagnostic detection of the organism in body tissue or in the environment.

The Replikin sequence structure is associated with the function of replication. Thus, whether the Replikins of this invention are used for targeting sequences that contain Replikins for the purpose of diagnostic identification, promoting replication, or inhibiting or attacking replication, for example, the structure-function relationship of the Replikin is fundamental. Thus, while the structure of the Replikin may be a part of a larger protein sequence, which may have been previously identified, it is preferable to utilize only the specific Replikin structure when seeking to induce antibodies that will recognize and attach to the Replikin fragment and there by cause destruction of the cell. Even though the larger protein sequence may be known in the art as having a "replication associated function," vaccines using the larger protein often have failed or proven ineffective, even though they contain one or more Replikin sequences.

Although the present inventors do not wish to be held to a single theory, the studies herein suggest that the prior art vaccines are ineffective because they are based on the use of the larger protein sequence. The larger protein sequence invariably has one or more epitopes (independent antigenic sequences that can induce specific antibody formation); Replikin structures usually comprise one of these potential epitopes. The presence of other epitopes within the larger protein may interfere with adequate formation of antibodies to the Replikin, by "flooding" the immune system with irrelevant antigenic stimuli which may preempt the Replikin antigens, *See,* e.g., Webster, R.G., J. Immunol., 97(2):177-183 (1966); and Webster et al., J. Infect. Dis., 134:48-58, 1976; Klenerman et al, Nature 394:421-422 (1998) for a discussion of the well-known phenomenon "original antigenic sin"). The formation of an antibody to a non-Replikin epitope may allow binding to the cell, but not necessarily lead to cell destruction. The presence of structural "decoys" on the C-termini of malaria proteins is another aspect of this ability of other epitopes to interfere with binding of effective anti-Replikin antibodies, since the decoy epitopes have many lysine residues, but no histidine residues. Thus, decoy epitopes may bind anti-Replikin antibodies, but keep the antibodies away from histidine - bound respiratory enzymes.

It is well known in the art that in the course of antibody production against a "foreign" protein, the protein is first hydrolyzed into smaller fragments. Usually fragments containing from about six to ten amino acids are selected for antibody formation. Thus, if hydrolysis of a protein does not result in Replikin-containing fragments, anti-Replikin antibodies will not be produced. In this regard, it is interesting that Replikins contain lysine residues located six to ten amino acids apart, since lysine residues are known to bind to membranes.

Furthermore, Replikin sequences contain at least one histidine residue. Histidine is frequently involved in binding to redox centers. Thus, an antibody that specifically recognizes a Replikin sequence has a better chance of inactivating or destroying the cell in which the Replikin is located, as seen with anti-malignin antibody, which is perhaps the most cytotoxic antibody yet described, being active at picograms per cell.

One of the reasons that vaccines directed towards a particular protein antigen of a disease causing agent have not been fully effective in providing protection against the disease (such as foot and mouth vaccine which has been developed against the VP1 protein or large segments of the VP1 protein) is that antibody to the Replikins have not been produced. That is, either epitopes other than Replikins present in the larger protein fragments may interfere according to the phenomenon of "original antigenic sin", and/or because the hydrolysis of larger protein sequences into smaller sequences for processing to produce antibodies results in loss of integrity of any Replikin structure that is present, e.g., the Replikin is cut in two and/or the histidine residue is lost in the hydrolytic processing. The present studies suggest that for an effective vaccine to be produced, the Replikin sequences, and no other epitope, should be used as the vaccine. For example, a vaccine of the invention can be generated using any one of the Replikin peptides identified by the three point recognition system. Particularly preferred peptides for an influenza vaccine include peptides that have been demonstrated to be conserved over a period of one or more years, preferably about three years or more, and/or which are present in a strain of influenza virus shown to have the highest increase in concentration of Replikins relative to Replikin concentration in other influenza virus strains, e.g., an emerging strain. The increase in Replikin concentration preferably occurs over a period of at least about six months to one year, preferably at least about two years or more, and most preferably about three years or more. Among the preferred Replikin peptides for use in an influenza virus vaccine are those replikins observed to "re-emerge" after an absence from the hemagglutinin amino acid sequence for one or more years.

The Replikin peptides of the invention, alone or in various combinations are administered to a subject, preferably by i.v. or intramuscular injection, in order to stimulate the immune system of the subject to produce antibodies to the peptide. Generally the dosage of peptides is in the range of from about 0.1 µg to about 10 mg, preferably about 10 µg to about 1 mg, and most preferably about 50 µg to about 500 ug. The skilled practitioner can readily determine the dosage and number of dosages needed to produce an effective immune response.

Replikin DNA or RNA may have a number of uses for the diagnosis of diseases resulting from infection with a virus, bacterium or other Replikin encoding agent. For example, Replikin nucleotide sequences may be used in hybridization assays of biopsied tissue or blood, e.g., Southern or Northern analysis, including *in situ* hybridization assays, to diagnose the presence of a particular organism in a tissue sample or an environmental sample, for example. The present invention also contemplates kits containing antibodies specific for particular Replikins that are present in a particular pathogen of interest, or containing nucleic acid molecules (sense or antisense) that hybridize specifically to a particular Replikin, and optionally, various buffers and/or reagents needed for diagnosis.

Also within the scope of the invention are oligoribonucleotide sequences, that include antisense RNA and DNA molecules and ribozymes that function to inhibit the translation of Replikin- or recognin-containing mRNA. Both antisense RNA and DNA molecules and ribozymes may be prepared by any method known in the art. The antisense molecules can be incorporated into a wide variety of vectors for delivery to a subject. The skilled practitioner can readily determine the best route of delivery, although generally i.v. or i.m. delivery is routine. The dosage amount is also readily ascertainable.

Particularly preferred antisense nucleic acid molecules are those that are complementary to a Replikin sequence contained in a mRNA encoding an influenza virus polypeptide, wherein the Replikin sequence comprises from 7 to about 50 amino acids including (1) at least one lysine residue located six to ten residues from a second lysine residue; (2) at least one histidine residue; and (3) at least 6% lysine residues. More preferred are antisense nucleic acid molecules that are complementary to a Replikin present in the coding strand of the gene or to the mRNA encoding the influenza virus hemagglutinin protein, wherein the antisense nucleic acid molecule is complementary to a nucleotide sequence encoding a Replikin that has been demonstrated to be conserved over a period of six months to one or more years and/or which are present in a strain of influenza virus shown to have an increase in concentration of Replikins relative to Replikin concentration in other influenza virus strains. The increase in Replikin concentration preferably occurs over a period of at least six months, preferably about one year, most preferably about two or three years or more.
Similarly, antisense nucleic acid molecules that are complementary to mRNA those that are complementary to a mRNA encoding a *Bacillus anthracis* polypeptide comprising a replikin sequence comprising from 7 to about 50 amino acids including (1) at least one lysine residue located six to ten residues from a second lysine residue; (2) at least one histidine residue; and (3) at least 6% lysine residues. More preferred are antisense nucleic acid molecules that are complementary to the coding strand of the gene or to the mRNA encoding the *Bacillus anthracis* Anthrax Lethal Factor Protein pX01-107 peptide, wherein the antisense nucleic acid molecule is complementary to a nucleotide sequence encoding the peptide of SEQ ID NO. 91, SEQ ID NO. 92, SEQ ID NO. 93, SEQ ID NO. 94, SEQ ID NO. 95, SEQ ID NO. 96, SEQ ID NO. 97, or SEQ ID NO. 98.

[049] Another preferred set of antisense nucleic acid molecules includes those that are complementary to a mRNA encoding a *Small Pox Virus* polypeptide comprising a replikin sequence comprising from 7 to about 50 amino acids including (1) at least one lysine residue located six to ten residues from a second lysine residue; (2) at least one histidine residue; and (3) at least 6% lysine residues. More preferred are antisense nucleic acid molecules that are complementary to the coding strand of the gene or to the mRNA encoding the Small Pox Virus Surface Antigen S Precursor Protein, wherein the antisense nucleic acid molecule is complementary to a nucleotide sequence encoding the peptide of SEQ ID NO. 99, SEQ ID NO. 100, SEQ ID NO. 101, SEQ ID NO. 102, or SEQ ID NO. 103.

In another embodiment of the invention, immune serum containing antibodies to one or more Replikins obtained from an individual exposed to one or more Replikins may be used to induce passive immunity in another individual or animal. Immune serum may be administered *via* i.v. to a subject in need of treatment. Passive immunity also can be achieved by injecting a recipient with preformed antibodies to one or more Replikins. Passive immunization may be used to provide immediate protection to individuals who have been exposed to an infectious organism. Administration of immune serum or preformed antibodies is routine and the skilled practitioner can readily ascertain the amount of serum or antibodies needed to achieve the desired effect.

In another aspect of the invention, Replikin structures are used to increase the replication of organisms. The present invention demonstrates that in influenza virus, for example, increased replication associated with epidemics is associated with increased concentration of Replikins. The increase is due to 1) the reappearance of particular replikin structures, which were present in previous years, but which then disappeared for one or more years; and/or 2) by the appearance of new replikin compositions. In addition, in malaria Replikins, repetition of the same Replikin in a single protein occurs.
Thus, the present invention provides methods and compositions for increasing the replication of organisms. For example, the production of crops which are critical to feeding large populations throughout the world, such as rice, for example, can be improved by increasing the concentration (number of Replikins/100 amino acid residues) of any particular strain of the food crop.

As an example, in the *Oryza sativa* strain of rice, catalase isolated from immature seeds was observed to contain three different Replikins within the 491 amino acid sequence of the protein. Thus, by using recombinant gene cloning techniques well known in the art, the concentration of Replikin structures in an organism, such as a food crop plant, can be increased, which will promote increased replication of the organism.

The present invention also provides methods for identifying Replikin sequences in an amino acid or nucleic acid sequence. Visual scanning of over four thousand sequences was performed in developing the present 3-point-recognition methods. However, data banks comprising nucleotide and/or amino acid sequences can also be scanned by computer for the presence of sequences meeting the 3 point recognition requirements.

The three point recognition method may also be modified to identify other useful compounds of covalently linked organic molecules, including other covalently linked amino acids, nucleotides, carbohydrates, lipids or combinations thereof. In this embodiment of the invention a sequence is screened for subsequences containing three or more desired structural characteristics. In the case of screening compounds composed of covalently linked amino acids, lipids or carbohydrates the subsequence of 7 to about 50 covalently linked units should contain (1) at least one first amino acid, carbohydrate or lipid residue located seven to ten residues from a second of the first amino acid, carbohydrate or lipid residue; (2) encoding at least one second amino acid, lipid or carbohydrate residue; and (3) at least 6% of the first amino acid, carbohydrate or lipid residue. In the case of screening nucleotide sequences, the subsequence of about 21 to about 150 nucleotides should contain (1) at least one codon encoding a first amino acid located within eighteen to thirty nucleotides from a second codon encoding the first amino acid residue; (2) at least one second amino acid residue; and (3) encodes at least 6% of said first amino acid residue.

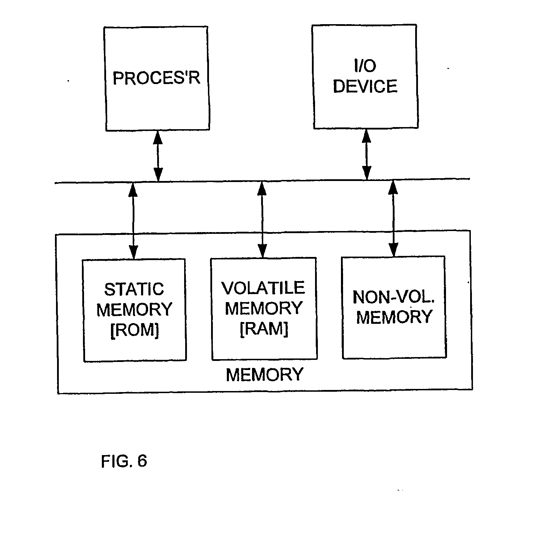

According to another embodiment of the invention, the methods described herein may be performed by a computer. Figure 6 is a block diagram of a computer available for use with the foregoing embodiments of the present invention. The computer may include a processor, an input/output device and a memory storing executable program instructions representing the 3-point-recognition methods of the foregoing embodiments. The memory may include a static memory, volatile memory and/or a nonvolatile memory. The static memory conventionally may be a read only memory ("ROM") provided on a magnetic, or an electrical or optical storage medium. The volatile memory conventionally may be a random access memory ("RAM") and may be integrated as a cache within the processor or provided externally from the processor as a separate integrated circuit. The non-volatile memory may be an electrical, magnetic or optical storage medium.

From a proteomic point of view the construction of a "3-point - recognition" template based on the new glioma peptide sequence led directly to identification of a biology-wide class of proteins having related structures and functions. The operation of the 3-point-recognition method resembles identification by the use of a "keyword"search; but instead of using the exact spelling of the keyword "kagvaflhkk" **(SEQ ID NO:1)** as in a typical sequence homology search, or in the nucleotide specification of an amino acid, an abstraction of the keyword delimited by the "3-point-recognition" parameters is used. This delimited abstraction, although derived from a single relatively short amino acid sequence leads to identification of a class of proteins with structures that are defined by the same specifications. That particular functions, in this case transformation and replication, in addition to structures, turn out also to be shared by members of the exposed class suggests that these structures and functions are related. Thus, from this newly identified short peptide sequence, a molecular recognition 'language' has been formulated, which previously has not been described. Further, the sharing of immunological specificity by diverse members of the class, as here demonstrated for the cancer Replikins, suggests that B cells and their product antibodies recognize Replikins by means of a similar recognition language. Since "3-point-recognition" is a proteoinic method that specifies a particular class of proteins, using three or more different recognition points for other peptides similarly should provide useful information concerning other proteins classes. Further, the "3-point- recognition" method is applicable to other recognins, for example to the TOLL 'innate' recognition of lipopolyssacharides of organisms.

Several embodiments of the present invention are specifically illustrated and described herein. However, it will be appreciated that modifications and variations of the present invention are encompassed by the above teachings and within the purview of the appended claims without departing from the spirit and intended scope of the invention.

### EXAMPLE 1

### PROCESS FOR EXTRACTION, ISOLATION AND IDENTIFICATION OF REPLIKINS AND THE USE OF REPLIKINS TO TARGET, LABEL OR DESTROY REPLIKIN-CONTAINING ORGANISMS

### a) Algae

The following algae were collected from Bermuda water sites and either extracted on the same day or frozen at -20 degrees C and extracted the next day. The algae were homogenized in a cold room (at 0 to 5 degrees C) in 1 gram aliquots in neutral buffer, for example 100 cc. of 0.005M phosphate buffer solution, pH 7 ("phosphate buffer") for 15 minutes in a Waring blender, centrifuged at 3000 rpm, and the supernatant concentrated by perevaporation and dialyzed against phosphate buffer in the cold to produce a volume of approximately 15 ml. The volume of this extract solution was noted and an aliquot taken for protein analysis, and the remainder was fractionated to obtain the protein fraction having a pK range between 1 and 4. The preferred method of fractionation is chromatography as follows:

The extract solution is fractionated in the cold room (4 degrees C) on a DEAE cellulose (Cellex-D) column 2.5x11.0 cm, which has been equilibrated with 0.005M phosphate buffer. Stepwise eluting solvent changes are made with the following solutions:
Solution 1- 4.04 g. NaH2P04 and 0.5g NaH2P04 are dissolved in 15 litres of distilled water (0.005 molar, pH 7);
Solution 2 - 8.57 g. NaH2P04 is dissolved in 2,480 ml. of distilled water;
Solution 3 - 17.1 g. of NaH2P04 is dissolved in 2480 ml of distilled water (0.05 molar, pH 4.7);
Solution 4 - 59.65 g. of NaH2P04 is dissolved in 2470 ml distilled water (0.175 molar);
Solution 5 - 101.6 g. of NaH2P04 is dissolved in 2455 ml distilled water (pH 4.3);
Solution 6 - 340.2 g. of NaH2P04 is dissolved in 2465 of distilled water (1.0 molar, pX-i 4.1);
Solution 7 - 283.63 g. of 80% phosphoric acid (H3P04) is made up in 2460 ml of distilled water (1.0 molar, pH 1.0).

The extract solution, in 6 to 10 ml volume, is passed onto the column and overlayed with Solution 1, and a reservoir of 300 ml of Solution 1 is attached and allowed to drip by gravity onto the column. Three ml aliquots of eluant are collected and analyzed for protein content at OD 280 until all of the protein to be removed with Solution 1 has been removed from the column. Solution 2 is then applied to the column, followed in succession by Solutions 3, 4, 5, 6 aid 7 until all of the protein which can, be removed with each Solution is removed from the column. The eluates from Solution 7 are combined, dialyzed against phosphate buffer, the protein content determined of both dialysand and dialyzate, and both analyzed by gel electrophoresis. One or two bands of peptide or protein of molecular weight between 3,000 and 25,000 Daltons are obtained in Solution 7. For example the algae *Caulerpa mexicana, Laurencia obtura, Cladophexa proliƒera, Sargassum natans, Caulerpa verticillata, Halimeda tuna,* and *Penicillos capitatus,* after extraction and treatment as above, all demonstrated in Solution 7 eluates sharp peptide bands in this molecular weight region with no contaminants. These Solution 7 proteins or their eluted bands are hydrolyzed, and the amino acid composition determined. The peptides so obtained, which have a lysine composition of 6% or greater are Replikin precursors. These Replikin peptide precursors are then determined for amino acid sequence and the replikins are determined by hydrolysis and mass spectrometry as detailed in U.S. patent 6,242,578 B1. Those which fulfill the criteria defined by the " 3-point-recognition" method are identified as Replikins. This procedure can also be applied to obtain yeast, bacterial and any plant Replikins.

### b) Virus

Using the same extraction and column chromatography separation methods as above in a) for algae, Replikens in virus-infected cells are isolated and identified.

### c) Tumor cells in vivo and in vitro tissue culture

Using the same extraction and column chromatography separation methods as above in a) for algae, Replikins in tumor cells are isolated and identified. For example, Replikin precursors of Astrocytin isolated from malignant brain tumors, Malignin (Aglyco 10B) isolated from glioblastoma tumor cells in tissue cu0lture, MCF7 mammary carcinoma cells in tissue culture, and P₃J Lymphoma cells in tissue culture each treated as above in a) yielded Replikin precursors with lysine content of 9.1%, 6.7%, 6.7%, and 6.5% respectively. Hydrolysis and mass spectrometry of Aglyco IOB as described in Example 10 U.S. 6,242,578 B1 produced the amino acid sequence, ykagvaflhkkndiide the 16-mer Replikin.

### EXAMPLE 2:

As an example of diagnostic use of Replikins: Aglyco IOB or the 16-mer Repliken may be used as antigen to capture and quantify the amount of its corresponding antibody present in serum for diagnostic purposes are as shown in Figures 2,3,4 and 7 of U.S. 6,242,578 B1.

As an example of the production of agents to attach to Replikins for labeling, nutritional or destructive purposes: Injection of the 16-mer Replikin into rabbits to produce the specific antibody to the 16-mer Replikin is shown in Example 6 and Figures 9A and 9B of U.S. 6,242,578 B1.

As an example of the use of agents to label Replikins: The use of antibodies to the 16-mer Replikin to label specific cells which contain this Replikin is shown in Figure 5 and Example 6 of U.S. 6,242,578 B1.

As an example of the use of agents to destroy Replikins: The use of antibodies to the 16-mer Replikin to inhibit or destroy specific cells which contain this Replikin is shown in Figure 6 of U.S. 6,242,578 B1.

### EXAMPLE 3

Analysis of sequence data of isolates of influenza virus hemagglutinin protein or neuraminidase protein for the presence and concentration of Replikins is carried out by visual scanning of sequences or through use of a computer program based on the 3-point recognition system described herein. Isolates of influenza virus are obtained and the amino acid sequence of the influenza hemagglutinin and/or neuraminidase protein is obtained by any art known method, such as by sequencing the hemagglutinin or neuraminidase gene and deriving the protein sequence therefrom. Sequences are scanned for the presence of new Replikins, conservation of Replikins over time and concentration of Replikins in each isolate. Comparison of the Replikin sequences and concentrations to the amino acid sequences obtained from isolates at an earlier time, such as about six months to about three years earlier, provides data that are used to predict the emergence of strains that are most likely to be the cause of influenza in upcoming flu seasons, and that form the basis for seasonal influenza peptide vaccines or nucleic acid based vaccines. Observation of an increase in concentration, particularly a stepwise increase in concentration of Replikins in a given strain of influenza virus for a period of about six months to about three years or more is a predictor of emergence of the strain as a likely cause of influenza epidemic or pandemic in the future.

Peptide vaccines or nucleic acid-based vaccines based on the Replikins observed in the emerging strain are generated. An emerging strain is identified as the strain of influenza virus having the highest increase in concentration of replikin sequences within the hemagglutinin and/or neuraminidase sequence during the time period. Preferably, the peptide or nucleic acid vaccine is based on or includes any Replikin sequences that are observed to be conserved in the emerging strain. Conserved replikins are preferably those Replikin sequences which are present in the hemagglutinin or neuraminidase protein sequence for about two years and preferably longer. The vaccines may include any combination of Replikin sequences identified in the emerging strain.

For vaccine production, the Replikin peptide or peptides identified as useful for an effective vaccine are synthesized by any method, including chemical synthesis and molecular biology techniques, including cloning, expression in a host cell and purification therefrom. The peptides are preferably admixed with a pharmaceutically acceptable carrier in an amount determined to induce a therapeutic antibody reaction thereto. Generally, the dosage is about 0.1 µg to about 10 mg.

The influenza vaccine is preferably administered to a patient in need thereof prior to the onset of "flu season." Influenza flu season generally occurs in late October and lasts through late April. However, the vaccine may be administered at any time during the year. Preferably, the influenza vaccine is administered once yearly, and is based on Replikin sequences observed to be present, and preferably conserved in the emerging strain of influenza virus. Another preferred Replikin for inclusion in an influenza vaccine is a Replikin demonstrated to have re-emerged in a strain of influenza after an absence of one or more years.

### EXAMPLE 4

Analysis of sequence data of isolates of *Plasmodium ƒalciparum* antigens for the presence and concentration of Replikins is carried out by visual scanning of sequences or through use of a computer program based on the 3-point recognition system described herein. Isolates of *Plasmodium ƒalciparum* are obtained and the amino acid sequence of the protein is obtained by any art known method, such as by sequencing the gene and deriving the protein sequence therefrom. Sequences are scanned for the presence of Replikins, conservation of Replikins over time and concentration of Replikins in each isolate. This information provides data that are used to form the basis for anti-malarial peptide vaccines or nucleic acid based vaccines.

Peptide vaccines or nucleic acid-based vaccines based on the Replikins observed in the malaria causing organism are generated. Preferably, the peptide or nucleic acid vaccine is based on or includes any Replikin sequences that are observed to be present on a surface antigen of the organism. The vaccines may include any combination of Replikin sequences identified in the malaria causing strain.

For vaccine production, the Replikin peptide or peptides identified as useful for an effective vaccine are synthesized by any method, including chemical synthesis and molecular biology techniques, including cloning, expression in a host cell and purification therefrom. The peptides are preferably admixed with a pharmaceutically acceptable carrier in an amount determined to induce a therapeutic antibody reaction thereto. Generally, the dosage is about 0.1 µg to about 10 mg.

Then malaria vaccine is preferably administered to a patient in need thereof at any time during the year, and particularly prior to travel to a tropical environment.

Further preferred embodiments are described hereinafter.
Claim 1. An isolated influenza virus peptide having from 7 to about 50 amino acids comprising
   (1) at least one lysine residue located six to ten residues from a second lysine residue; (2) at least one histidine residue; and (3) at least 6% lysine residues.
Claim 2. The peptide of claim 1 wherein the peptide is present in an emerging strain of influenza virus.
Claim 3. An antibody that specifically binds to an influenza virus peptide sequence having from 7 to about 50 amino acids comprising (1) at least one lysine residue located six to ten amino acid residues from a second lysine residue; (2) at least one histidine residue; and (3) at least 6% lysine residues.
Claim 4. The antibody of claim 3, wherein said antibody specifically binds to an influenza virus peptide present in an emerging strain of influenza virus.
Claim 5. An antibody cocktail comprising a plurality of antibodies, wherein each of said antibodies specifically binds to an influenza virus peptide sequence having from 7 to about 50 amino acids comprising (1) at least one lysine residue located six to ten amino acid residues from a second lysine residue; (2) at least one histidine residue; and (3) at least 6% lysine residues.
Claim 6. The antibody cocktail of claim 5 wherein the plurality of antibodies each independently and specifically binds to a Replikin peptide sequence present in an emerging strain of influenza virus.
Claim 7. A composition comprising the antibody of claim 3 or claim 4 and a pharmaceutically acceptable carrier and/or adjuvant.
Claim 8. A composition comprising the antibody cocktail of claim 5 or 6 and a pharmaceutically acceptable carrier and/or adjuvant.
Claim 9. A therapeutic composition comprising the isolated influenza virus peptide of claim 1 and a pharmaceutically acceptable carrier and/or adjuvant.
Claim 10. The therapeutic composition of claim 9 wherein the peptide is conserved in a strain of influenza virus for at least two consecutive years including the current year.
Claim 11. A therapeutic composition comprising a plurality of isolated influenza virus peptides each having from 7 to about 50 amino acids comprising
   (1) at least one lysine residue located six to ten residues from a second lysine residue; (2) at least one histidine residue; and (3) at least 6% lysine residues,
   and a pharmaceutically acceptable carrier.
Claim 12. The therapeutic composition of claim 11 wherein at least one of the plurality of influenza virus peptides is conserved in the influenza virus hemagglutinin amino acid sequence for at least two consecutive years including the current year.
Claim 13. An antisense nucleic acid molecule complementary to an influenza virus hemagglutinin Replikin mRNA sequence, said Replikin mRNA sequence having from 7 to about 50 amino acids comprising
   (1) at least one lysine residue located six to ten residues from a second lysine residue; (2) at least one histidine residue; and (3) at least 6% lysine residues.
Claim 14. An antisense nucleic acid molecule complementary to the coding strand of the gene or the mRNA encoding influenza virus hemagglutinin, wherein said antisense nucleic acid molecule is complementary to a nucleotide sequence present in an emerging strain of influenza virus.
Claim 15. A method of stimulating the immune system of a subject to produce antibodies to influenza virus comprising administering an effective amount of at least one isolated influenza virus Replikin- peptide having from 7 to about 50 amino acids comprising (1) at least one lysine residue located six to ten amino acid residues from a second lysine residue; (2) at least one histidine residue; and (3) at least 6% lysine residues.
Claim 16. The method of claim 15 wherein the at least one Replikin peptide is present in influenza virus hemagglutinin protein.
Claim 17. The method of claim 15 wherein at least one of the at least one Replikin peptide is conserved in an emerging strain of influenza virus for at least two consecutive years, including the current year.
Claim 18. A method of selecting an influenza virus peptide for inclusion in an influenza virus vaccine comprising
   (1) obtaining at least one isolate of each strain of a plurality of strains of influenza virus,
   (2) analyzing the hemagglutinin amino acid sequence of the at least one isolate of each strain of the plurality of strains of influenza virus for the presence and concentration of Replikin sequences,
   (3) comparing the concentration of Replikin sequences in the hemagglutinin amino acid sequence of the at least one isolate of each strain of the plurality of strains of influenza virus to the concentration of Replikin sequences observed in the hemagglutinin amino acid sequence of each of the strains at at least one earlier time period to provide the concentration of Replikins for at least two time periods, said at least one earlier time period being within about six months to about three years prior to step (1),
   (4) identifying the strain of influenza virus having the highest increase in concentration of Replikin sequences during the at least two time periods,
   (5) selecting at least one Replikin sequence present in the strain of influenza virus peptide identified in step (4) as an isolated peptide for inclusion in an influenza virus vaccine.
Claim 19. The method of claim 18 wherein the Replikin sequence is conserved for at least about two consecutive years within the strain of influenza virus having the highest increase in Replikin sequence concentration during the at least two time periods.
Claim 20. The method of claim 18 wherein in step (5) a plurality of Replikin sequences is selected.
Claim 21. A method of making an influenza virus vaccine comprising
   (1) identifying a strain of influenza virus as an emerging strain,
   (2) selecting at least one Replikin sequence present in the emerging strain as a peptide template for influenza virus vaccine manufacture,
   (3) synthesizing peptides having the amino acid sequence of the at least one Replikin sequence selected in step (2), and
   (4) combining a therapeutically effective amount of the peptides of step (4) with a pharmaceutically acceptable carrier and/or adjuvant.
Claim 22. The method of claim 21 wherein the at least one Replikin selected in step (2) is conserved in the influenza virus hemagglutinin sequence for at least two consecutive years, including the current year.
Claim 23. A method of identifying an emerging strain of influenza virus comprising
   (1) obtaining at least one isolate of each strain of a plurality of strains of influenza virus,
   (2) analyzing the hemagglutinin amino acid sequence of the at least one isolate of each strain of the plurality of strains of influenza virus for the presence and concentration of Replikin sequences,
   (3) comparing the concentration of Replikin sequences in the hemagglutinin amino acid sequence of the at least one isolate of each strain of the plurality of strains of influenza virus to the concentration of Replikin sequences observed in the hemagglutinin amino acid sequence of each of the strains at at least one earlier time period to provide the concentration of Replikins for at least two time periods, said at least one earlier time period being within about six months to about three years prior to step (1), and
   (4) identifying the strain of influenza virus having the highest increase in concentration of Replikin sequences during the at least two time periods.
Claim 24. An influenza virus vaccine comprising at least one isolated Replikin present in the hemagglutinin protein of an emerging strain of influenza virus and a pharmaceutically acceptable carrier and/or adjuvant.
Claim 25. The influenza virus vaccine of claim 24 wherein the vaccine comprises a plurality of isolated Replikins.
Claim 26. The vaccine of claim 24 wherein the at least one isolated Replikin is conserved for at least two consecutive years in the emerging strain.
Claim 27. A method of preventing or treating influenza virus infection comprising administering to a patient in need thereof a vaccine comprising at least one isolated Replikin present in the hemagglutinin protein of an emerging strain of influenza virus and a pharmaceutically acceptable carrier and/or adjuvant.
Claim 28. The method of claim 27 wherein the vaccine is administered prior to the onset of flu season.
Claim 29. The method of claim 27 further comprising administering an antiviral agent.
Claim 30. the method of claim 27 wherein the antiviral agent is gancyclovir.
Claim 31. The method of claim 27 wherein the vaccine comprises a Replikin that has been conserved in the emerging strain for at least about one year.
Claim 32. A malaria vaccine comprising at least one isolated *Plasmodium ƒalciparum* replikin and a pharmaceutically acceptable carrier.
Claim 33. The vaccine of claim 32 comprising a plurality of *Plasmodium ƒalciparum* replikins.
Claim 34. The vaccine of claim 33 comprising overlapping Replikins.
Claim 35. The vaccine of claim 33 wherein the Replikin is present on the merozoite surface or within the parasitophorous vacuole of the *Plasmodium ƒalciparum.*
Claim 36. An isolated *Plasmodium ƒalciparum* peptide comprising
   (1) at least one lysine residue located six to ten residues from a second lysine residue; (2) at least one histidine residue; and (3) at least 6% lysine residues.
Claim 37. The peptide of claim 36 comprising overlapping replikins.
Claim 38. An antibody that specifically binds to a Plasmodium falciparum peptide sequence having (1) at least one lysine residue located six to ten residues from a second lysine residue; (2) at least one histidine residue; and (3) at least 6% lysine residues.
Claim 39. An antibody cocktail comprising a plurality of antibodies, wherein each of the plurality of antibodies specifically binds to a *Plasmodium ƒalciparum* peptide sequence having (1) at least one lysine residue located six to ten residues from a second lysine residue; (2) at least one histidine residue; and (3) at least 6% lysine residues.
Claim 40. A composition comprising the antibody of claim 38 or antibody cocktail of claim 39 and a pharmaceutical composition.
Claim 41. A method of stimulating the immune system of a subject to produce antibodies to *Plasmodium ƒalciparum* comprising administering to the subject an effective amount of at least one *Plasmodium ƒalciparum* Replikin and a pharmaceutically acceptable carrier.
Claim 42. The method of claim 41 wherein the Replikin is located on the merozoite surface or within the parasitophorous vacuole of the *Plasmodium ƒalciparum.*
Claim 43. A method of preventing or treating malaria comprising administering to a patient a therapeutically effective amount of a vaccine comprising at least one *Plasmodium ƒalciparum* Replikin and a pharmaceutically acceptable carrier.
Claim 44. A method of detecting the presence of a contaminating organism in a body sample or environmental sample comprising
   1) isolating nucleic acids from the body sample or environmental sample;
   2) screening the nucleic acids for the presence of a replikin structure; and
   3) correlating the presence of a Replikin structure with the presence of the contaminating organism.
Claim 45. The method of claim 44 wherein the body sample is blood.
Claim 46. A method for increasing the replication rate of an organism comprising transforming a gene encoding an enzyme having a replication function in the organism with at least one Replikin structure.
Claim 47. The method of claim 46 wherein the organism is a food plant.
Claim 48. The method of claim 46 wherein the organism is a rice plant.
Claim 49. An isolated *Bacillus anthracis* peptide comprising from 7 to about 50 amino acids including
   (1) at least one lysine residue located six to ten residues from a second lysine residue; (2) at least one histidine residue; and (3) at least 6% lysine residues.
Claim 50. The peptide of claim 49 wherein the peptide has the amino acid sequence as set forth in SEQ ID NO. 91.
Claim 51. The peptide of claim 49 wherein the peptide has the amino acid sequence as set forth in SEQ ID NO. 92.
Claim 52. The peptide of claim 49 wherein the peptide has the amino acid sequence as set forth in SEQ ID NO. 93.
Claim 53 The peptide of claim 49 wherein the peptide has the amino acid sequence as set forth in SEQ ID NO. 94.
Claim 54. The peptide of claim 49 wherein the peptide has the amino acid sequence as set forth in SEQ ID NO. 95.
Claim 55 The peptide of claim 50 wherein the peptide has the amino acid sequence as set forth in SEQ ID NO. 96.
Claim 56. The peptide of claim 49 wherein the peptide has the amino acid sequence as set forth in SEQ ID NO. 97.
Claim 57. The peptide of claim 49 wherein the peptide has the amino acid sequence as set forth in SEQ ID NO. 98.
Claim 58. An isolated small pox virus peptide comprising from 7 to about 50 amino acids including
   (1) at least one lysine residue located six to ten residues from a second lysine residue; (2) at least one histidine residue; and (3) at least 6% lysine residues.
Claim 59. The peptide of claim 58 wherein the peptide has the amino acid sequence as set forth in SEQ ID NO. 99.
Claim 60. The peptide of claim 58 wherein the peptide has the amino acid sequence as set forth in SEQ ID NO. 100.
Claim 61. The peptide of claim 58 wherein the peptide has the amino acid sequence as set forth in SEQ ID NO. 101.
Claim 62. The peptide of claim 58 wherein the peptide has the amino acid sequence as set forth in SEQ ID NO. 102.
Claim 63. The peptide of claim 58 wherein the peptide has the amino acid sequence as set forth in SEQ ID NO. 103.
Claim 64. An antibody that specifically binds to a *Bacillus anthracis* peptide or polypeptide sequence comprising from 7 to about 50 amino acids including (1) at least one lysine residue located six to ten amino acid residues from a second lysine residue; (2) at least one histidine residue; and (3) at least 6% lysine residues.
Claim 65. An antibody cocktail comprising a plurality of antibodies, wherein each of said antibodies specifically binds to a *Bacillus anthracis* peptide or polypeptide sequence comprising from 7 to about 50 amino acids including (1) at least one lysine residue located six to ten amino acid residues from a second lysine residue; (2) at least one histidine residue; and (3) at least 6% lysine residues.
Claim 66. An antibody that specifically binds to a small pox virus peptide or polypeptide sequence comprising from 7 to about 50 amino acids including (1) at least one lysine residue located six to ten amino acid residues from a second lysine residue; (2) at least one histidine residue; and (3) at least 6% lysine residues.
Claim 67. An antibody cocktail comprising a plurality of antibodies, wherein each of said antibodies specifically binds to a small pox virus peptide or polypeptide sequence comprising from 7 to about 50 amino acids including (1) at least one lysine residue located six to ten amino acid residues from a second lysine residue; (2) at least one histidine residue; and (3) at least 6% lysine residues.
Claim 68. A method of stimulating the immune system of a subject to produce antibodies to *Bacillus anthracis* comprising administering an effective amount of at least one *Bacillus anthracis* peptide comprising from 7 to about 50 amino acids including (1) at least one lysine residue located six to ten amino acid residues from a second lysine residue; (2) at least one histidine residue; and (3) at least 6% lysine residues.
Claim 69. The method of claim 68 comprising administering at least one peptide selected from the group consisting of SEQ ID NO. 91, SEQ ID NO. 92, SEQ ID NO. 93, SEQ ID NO. 94, SEQ ID NO. 95, SEQ ID NO. 96, SEQ ID NO. 97, SEQ ID NO. 98. and combinations thereof.
Claim 70. A method of stimulating the immune system of a subject to produce antibodies to small pox virus comprising administering an effective amount of at least one small pox virus peptide comprising from 7 to about 50 amino acids including (1) at least one lysine residue located six to ten amino acid residues from a second lysine residue; (2) at least one histidine residue; and (3) at least 6% lysine residues.
Claim 71. The method of claim 70 wherein the at least one peptide is selected from the group consisting of SEQ ID NO. 99, SEQ ID NO. 100, SEQ ID NO. 101, SEQ ID NO. 102, SEQ ID NO. 103 and combinations thereof.
Claim 72. An antisense nucleic acid molecule complementary to a mRNA encoding a *Bacillus anthracis* polypeptide comprising a replikin sequence comprising from 7 to about 50 amino acids including
   (1) at least one lysine residue located six to ten residues from a second lysine residue; (2) at least one histidine residue; and (3) at least 6% lysine residues.
Claim 73. An antisense nucleic acid molecule complementary to the coding strand of the gene or to the mRNA encoding the *Bacillus anthracis* Anthrax Lethal Factor Protein pX01-107 peptide, wherein said antisense nucleic acid molecule is complementary to a nucleotide sequence encoding the peptide of SEQ ID NO. 91, SEQ ID NO. 92, SEQ ID NO. 93, SEQ ID NO. 94, SEQ ID NO. 95, SEQ ID NO. 96, SEQ ID NO. 97, or SEQ ID NO. 98.
Claim 74. An antisense nucleic acid molecule complementary to a mRNA encoding a *Small Pox Virus* polypeptide comprising a replikin sequence comprising from 7 to about 50 amino acids including
   (1) at least one lysine residue located six to ten residues from a second lysine residue; (2) at least one histidine residue; and (3) at least 6% lysine residues.
Claim 75. An antisense nucleic acid molecule complementary to the coding strand of the gene or to the mRNA encoding the Small Pox Virus Surface Antigen S Precursor Protein, wherein said antisense nucleic acid molecule is complementary to a nucleotide sequence encoding the peptide of SEQ ID NO. 99, SEQ ID NO. 100, SEQ ID NO. 101, SEQ ID NO. 102, or SEQ ID NO. 103.
Claim 76. An isolated peptide comprising from 7 to about 50 amino acids including
   (1) at least one lysine residue located six to ten residues from a second lysine residue; (2) at least one histidine residue; and (3) at least 6% lysine residues.
Claim 77. The peptide of claim 76 wherein said peptide is a recognin.
Claim 78. The peptide of claim 76 wherein said peptide is a replikin.
Claim 79. The peptide of claim 76 wherein said peptide is an alga peptide.
Claim 80. The peptide of claim 76 wherein said peptide is a yeast peptide.
Claim 81. The peptide of claim 76 wherein said peptide is an amoeba peptide.
Claim 82. The peptide of claim 76 wherein said peptide is a virus peptide.
Claim 83. The peptide of claim 76 wherein said peptide is plant peptide.
Claim 84. The peptide of claim 76 wherein said peptide is a replication-associated peptide.
Claim 85. The peptide of claim 76 wherein said peptide is a bacterial peptide.
Claim 86. The peptide of claim 76 wherein said peptide is a tumor virus associated peptide.
Claim 87. The peptide of claim 76 wherein said peptide is a transforming-associated peptide.
Claim 88. The peptide of claim 76 wherein said peptide is a cancer cell associated peptide.
Claim 89. The peptide of claim 76 wherein said peptide is a glioma peptide.
Claim 90. An antibody that specifically binds to a peptide sequence comprising from 7 to 50 amino acids and including (1) at least one lysine residue located six to ten residues from a second lysine residue; (2) at least one histidine residue; and (3) at least 6% lysine residues.
Claim 91. An antibody cocktail comprising a plurality of antibodies, wherein each of the antibodies specifically binds to a peptide sequence comprising from 7 to 50 amino acids and including (1) at least one lysine residue located six to ten residues from a second lysine residue; (2) at least one histidine residue; and (3) at least 6% lysine residues.
Claim 92. An isolated peptide selected from the group of peptides having an amino acid sequence consisting of any one of SEQ ID NO. 1 through SEQ ID NO. 90.
Claim 93. A method of identifying a protein or peptide containing a replikin sequence or a recognin sequence comprising scanning the amino acid sequence of the protein or peptide for a subsequence comprising 7 to 50 amino acids which includes (1) at least one lysine residue located six to ten residues from a second lysine residue; (2) at least one histidine residue; and (3) at least 6% lysine residues.
Claim 94. The method of claim 93 wherein the amino acid sequence is scanned visually.
Claim 95. The method of claim 93 wherein the scanning of the amino acid sequence is performed by a computer.
Claim 96. A method of identifying a nucleotide sequence containing a replikin sequence or a recognin sequence comprising scanning the nucleotide sequence for a subsequence comprising 21 to 150 nucleotides which includes codons for (1) at least one lysine residue located within eighteen to thirty nucleotides from a second codon encoding a lysine residue; and (2) at least one histidine residue; and (3) encodes at least 6% lysine residues.
Claim 97. The method of claim 96 wherein the subsequence is in frame with the coding sequence of a protein or peptide.
Claim 98. The method of claim 96 wherein the scanning of the nucleotide sequence is performed by a computer.
Claim 99. The method of claim 96 wherein the nucleotide sequence is visually scanned.
Claim 100. A computer readable medium having stored thereon instructions that, when executed by a computer, cause the computer to identify nucleotide sequences encoding replikins and/or recognins according to the method of:
   with reference to a data bank of nucleotide sequences, scanning said nucleotide sequences for nucleotide sequences containing a replikin sequence or a recognin sequence comprising 21 to 150 nucleotide which includes codons for (1) at least one lysine residue located within eighteen to thirty nucleotides from a second codon encoding a lysine residue; and (2) at least one histidine residue; and (3) encodes at least 6% lysine residues.
Claim 101. A computer readable medium having stored thereon instructions that, when executed by a computer, cause the computer to identify proteins or peptides containing a replikin sequence or a recognin sequence, according to the method of:
   with reference to a data bank of protein or peptide sequences, scanning said protein or peptide sequences for a replikin sequence or a recognin sequence comprising 7 to 50 amino acids which includes (1) at least one lysine located within six to ten amino acids from a second lysine residue; and (2) at least one histidine residue; and (3) at least 6% of lysine residues.
Claim 102. A computer readable medium having stored thereon instructions that, when executed by a computer, cause the computer to identify proteins, peptides, carbohydrates or lipids containing a subsequence comprising at least three predetermined structural characteristics, according to the method of:
   with reference to a data bank of proteins, peptides, carbohydrate or lipid sequences, scanning the sequence of said proteins, peptides, carbohydrates, or lipids, respectively, for a subsequence comprising 7 to 50 amino acids, carbohydrates or lipids, which includes (1) at least one first amino acid, carbohydrate or lipid residue located within six to ten amino acid, carbohydrate or lipid residues, respectively, from a second of said first amino acid, carbohydrate or lipid residue; and (2) at least one second amino acid, carbohydrate or lipid residue which is different from said first amino acid, carbohydrate or lipid residue; and (3) at least 6% of said first amino acid, carbohydrate or lipid residues.
Claim 103. A computer readable medium having stored thereon instructions that, when executed by a computer, cause the computer to identify sequences, according to the method of:
   with reference to a data bank of nucleic acid sequences, scanning said nucleic acid sequences for a subsequence comprising about 21 to about 150 nucleotides which includes (1) at least one codon encoding a first amino acid residue located within eighteen to thirty nucleotides from a second codon encoding the first amino residue; and (2) at least one codon encoding a second amino acid residue which is different from said first amino acid residue; and (3) encodes at least 6% of said first amino acid residues.
Claim 104. A process for stimulating the immune system of a subj ect to produce antibodies that bind specifically to replikins, said process comprising administering to the subject an effective amount of a dosage of a composition comprising at least one replikin peptide.
Claim 105. A process for stimulating the immune system of a subject to produce antibodies that bind specifically to replikins, said process comprising administering to the subject an effective amount of a dosage of a composition comprising at least one non-immune-based organic agent that specifically targets replikin sequences.
Claim 106. The process of claim 105 wherein the agent is nucleic acid.
Claim 107. The process of claim 106 wherein the nucleic acid is in antisense configuration.
Claim 108. The process of claim 104 wherein the dosage is administered as an approximately 1 mg dosage form.
Claim 109. A method of identifying a compound comprising a sequence of covalently linked units selected from the group consisting of amino acids, carbohydrates, lipids, and combinations thereof, said method comprising scanning the amino acid sequence of said compound for a subsequence comprising 7 to 50 amino acids which includes
   (1) at least one first amino acid, carbohydrate or lipid residue located six to ten residues from a second of said first amino acid, carbohydrate or lipid residue; (2) at least one second amino acid, lipid or carbohydrate residue which is different from said first amino acid, carbohydrate or lipid residue; and (3) at least 6% of said first amino acid, carbohydrate or lipid residue.
Claim 110. A method of identifying a sequence of covalently linked nucleotides, said method comprising scanning the sequence for a subsequence comprising 21 to about 150 nucleotides which includes codons for (1) at least one first amino acid residue located within eighteen to thirty nucleotides from a second codon encoding the first amino acid residue; and (2) at least one second amino acid residue which is different from said first amino acid residue; and which (3) encodes at least 6% of said first amino acid residue.
Claim 111. The peptide according to claim 79 wherein said peptide has the sequence as set forth in SEQ ID NO: 34.
Claim 112. The peptide according to claim 79 wherein said peptide has the sequence as set forth in SEQ ID NO: 35.
Claim 113. The peptide according to claim 80 wherein said peptide has the sequence as set forth in SEQ ID NO: 36.
Claim 114. The peptide according to claim 80 wherein said peptide has the sequence as set forth in SEQ ID NO: 37.
Claim 115. The peptide according to claim 80 wherein said peptide has the sequence as set forth in SEQ ID NO: 2.
Claim 116. The peptide according to claim 81 wherein said peptide has the sequence as set forth in SEQ ID NO: 41.
Claim 117. The peptide according to claim 82 wherein said peptide has the sequence as set forth in SEQ ID NO: 9.
Claim 118. The peptide according to claim 82 wherein said peptide has the sequence as set forth in SEQ ID NO: 11.
Claim 119. The peptide according to claim 82 wherein said peptide has the sequence as set forth in SEQ ID NO: 12.
Claim 120. The peptide according to claim 82 wherein said peptide has the sequence as set forth in SEQ ID NO: 3.
Claim 121. The peptide according to claim 82 wherein said peptide has the sequence as set forth in SEQ ID NO: 5.
Claim 122. The method according to claim 82 wherein said peptide has the sequence as set forth in SEQ ID NO: 7.
Claim 123. The peptide according to claim 82 wherein said peptide has the sequence as set forth in SEQ ID NO: 47.
Claim 124. The peptide according to claim 82 wherein said peptide has the sequence as set forth in SEQ ID NO: 13.
Claim 125. The peptide according to claim 82 wherein said peptide has the sequence as set forth in SEQ ID NO: 14.
Claim 126. The peptide according to claim 82 wherein said peptide has the sequence as set forth in SEQ ID NO: 15.
Claim 127. The peptide according to claim 82 wherein said peptide has the sequence as set forth in SEQ ID NO: 16.
Claim 128 The peptide according to claim 82 wherein said peptide has the sequence as set forth in SEQ ID NO: 17.
Claim 129. The peptide according to claim 83 wherein said peptide has the sequence as set forth in SEQ ID NO: 44.
Claim 130. The peptide according to claim 83 wherein said peptide has the sequence as set forth in SEQ ID NO: 45.
Claim 131. The peptide according to claim 83 wherein said peptide has the sequence as set forth in SEQ ID NO: 46.
Claim 132. The peptide according to claim 84 wherein said peptide has the sequence as set forth in SEQ ID NO: 2.
Claim 133. The peptide according to claim 84 wherein said peptide has the sequence as set forth in SEQ ID NO: 8.
Claim 134. The peptide according to claim 84 wherein said peptide has the sequence as set forth in SEQ ID NO: 9.
Claim 135. The peptide according to claim 84 wherein said peptide has the sequence as set forth in SEQ ID NO: 11.
Claim 136. The peptide according to claim 84 wherein said peptide has the sequence as set forth in SEQ ID NO: 12.
Claim 137. The peptide according to claim 85 wherein said peptide has the sequence as set forth in SEQ ID NO: 42.
Claim 138. The peptide according to claim 85 wherein said peptide has the sequence as set forth in SEQ ID NO: 10.
Claim 139. The peptide according to claim 85 wherein said peptide has the sequence as set forth in SEQ ID NO: 43.
Claim 140. The peptide according to claim 86 wherein said peptide has the sequence as set forth in SEQ ID NO: 48.
Claim 141. The peptide according to claim 86 wherein said peptide has the sequence as set forth in SEQ ID NO: 49.
Claim 142. The peptide according to claim 86 wherein said peptide has the sequence as set forth in SEQ ID NO: 50.
Claim 143. The peptide according to claim 86 wherein said peptide has the sequence as set forth in SEQ ID NO: 51.
Claim 144. The peptide according to claim 86 wherein said peptide has the sequence as set forth in SEQ ID NO: 52.
Claim 145. The peptide according to claim 86 wherein said peptide has the sequence as set forth in SEQ ID NO: 53.
Claim 146. The peptide according to claim 86 wherein said peptide has the sequence as set forth in SEQ ID NO: 54.
Claim 147. The peptide according to claim 86 wherein said peptide has the sequence as set forth in SEQ ID NO: 55.
Claim 148. The peptide according to claim 86 wherein said peptide has the sequence as set forth in SEQ ID NO: 56.
Claim 149. The peptide according to claim 86 wherein said peptide has the sequence as set forth in SEQ ID NO: 57.
Claim 150. The peptide according to claim 86 wherein said peptide has the sequence as set forth in SEQ ID NO: 58.
Claim 151. The peptide according to claim 86 wherein said peptide has the sequence as set forth in SEQ ID NO: 59.
Claim 152. The peptide according to claim 86 wherein said peptide has the sequence as set forth in SEQ ID NO: 60.
Claim 153, The peptide according to claim 86 wherein said peptide has the sequence as set forth in SEQ ID NO: 61.
Claim 154. The peptide according to claim 86 wherein said peptide has the sequence as set forth in SEQ ID NO: 62.
Claim 155. The peptide according to claim 86 wherein said peptide has the sequence as set forth in SEQ ID NO: 63.
Claim 156. The peptide according to claim 86 wherein said peptide has the sequence as set forth in SEQ ID NO: 64.
Claim 157. The peptide according to claim 86 wherein said peptide has the sequence as set forth in SEQ ID NO: 65.
Claim 158. The peptide according to claim 86 wherein said peptide has the sequence as set forth in SEQ ID NO: 18.
Claim 159. The peptide according to claim 87 wherein said peptide has the sequence as set forth in SEQ ID NO: 66.
Claim 160. The peptide according to claim 87 wherein said peptide has the sequence as set forth in SEQ ID NO: 67.
Claim 161. The peptide according to claim 87 wherein said peptide has the sequence as set forth in SEQ ID NO: 68.
Claim 162. The peptide according to claim 87 wherein said peptide has the sequence as set forth in SEQ ID NO: 69.
Claim 163. The peptide according to claim 87 wherein said peptide has the sequence as set forth in SEQ ID NO: 70.
Claim 164. The peptide according to claim 87 wherein said peptide has the sequence as set forth in SEQ ID NO: 71
Claim 165. The peptide according to claim 87 wherein said peptide has the sequence as set forth in SEQ ID NO: 72.
Claim 166. The peptide according to claim 87 wherein said peptide has the sequence as set forth in SEQ ID NO: 73.
'Claim 167. The peptide according to claim 87 wherein said peptide has the sequence as set forth in SEQ ID NO: 74.
Claim 168. The peptide according to claim 87 wherein said peptide has the sequence as set forth in SEQ ID NO: 75.
Claim 169. The peptide according to claim 87 wherein said peptide has the sequence as set forth in SEQ ID NO: 76.
Claim 170. The peptide according to claim 87 wherein said peptide has the sequence as set forth in SEQ ID NO: 77.
Claim 171. The peptide according to claim 88 wherein said peptide has the sequence as set forth in SEQ ID NO: 78.
Claim 172. The peptide according to claim 88 wherein said peptide has the sequence as set forth in SEQ ID NO: 79.
Claim 173. The peptide according to claim 88 wherein said peptide has the sequence as set forth in SEQ ID NO: 80.
Claim 174. The peptide according to claim 88 wherein said peptide has the sequence as set forth in SEQ ID NO: 81.
Claim 175. The peptide according to claim 88 wherein said peptide has the sequence as set forth in SEQ ID NO: 82.
Claim 176. The peptide according to claim 88 wherein said peptide has the sequence as set forth in SEQ ID NO: 83.
Claim 177. The peptide according to claim 88 wherein said peptide has the sequence as set forth in SEQ ID NO: 84.
Claim 178. The peptide according to claim 88 wherein said peptide has the sequence as set forth in SEQ ID NO: 85.
Claim 179. The peptide according to claim 88 wherein said peptide has the sequence as set forth in SEQ ID NO: 86.
Claim 180. The peptide according to claim 88 wherein said peptide has the sequence as set forth in SEQ ID NO: 87.
Claim 181. The peptide according to claim 88 wherein said peptide has the sequence as set forth in SEQ ID NO: 88.
Claim 182. The peptide according to claim 88 wherein said peptide has the sequence as set forth in SEQ ID NO: 89.
Claim 183. The peptide according to claim 84 wherein said peptide has the sequence set forth in SEQ ID NO. 10.

## Claims

1. An isolated or synthesized plant peptide, wherein said peptide has replication, transformation, oxidation-reduction, or conservation functions, or any combination thereof, in said plant and consists of 7 to about 50 amino acids including
(1) at least one lysine residue located six to ten amino acid residues from a second lysine residue;
(2) at least one histidine residue; and
(3) at least 6% lysine residues.

2. The isolated or synthesized plant peptide of claim 1, wherein said peptide has a lysine residue on one end and a lysine residue or a histidine residue on the other end.

3. The isolated or synthesized plant peptide of claims 1 or 2, wherein said plant peptide is an algal peptide.

4. The isolated or synthesized peptide of claims 1, 2, or 3, wherein said peptide comprises at least one amino acid sequence of SEQ ID NO(s): 34, 35, 37, or 44-46.

5. The peptide of claims 1, 2, or 3, wherein said peptide consists of the amino acid sequence of SEQ ID NO(s): SEQ ID NO(s): 34, 35, 37, or 44-46.

6. The isolated or synthesized peptide of claims 1, 2, 3, 4, or 5, wherein said peptide is conserved.

7. The isolated or synthesized peptide of claim 6, wherein said peptide is conserved for at least two years.

8. The isolated or synthesized peptide of claim 6, wherein the lysine residues and histidine residue that define the peptide of claim 1 are conserved.

9. The isolated or synthesized peptide of claim 8, wherein the peptide is conserved in the same or different strains.

10. The isolated or synthesized peptide of claim 6, wherein said peptide is conserved for at least two years in the same or different strains.

11. An antibody that specifically binds to at least one peptide sequence of claims 1-10.

12. A composition comprising the antibody of claim 11 or a plurality of antibodies of claim 11 and a pharmaceutical composition.

13. A vaccine comprising at least one isolated or synthesized peptide of claims 1-10.

14. An antisense nucleic acid molecule complementary to an mRNA encoding a at least one peptide of claims 1-10.

15. The peptide of claims 1, 2, 3, 4, 5, 6, 7, 8, 9, or 10, wherein said peptide is Cladophora peptide, a Cladophora prolifera peptide, an Isolepsis peptide, an Isolepsis prolifera peptide, a Caulerpa peptide, a Caulerpa Mexicana peptide, a Culerpa verticillata peptide, a Laurencia peptide, a Laurancia obtura peptide, a Sargassum peptide, a Sargassum natans peptide, a Penicillos peptide, a Penicillos capitatus peptide, a Halimeda peptide, a Halimeda tuna peptide, a Chlamydomonas reinhardtii peptide, a Pyrobotris stellata peptide, a Guillardia theta peptide, an Odontella sinensis peptide, a Prototheca peptide, a Chlorella vulgaris peptide, a Monomastix peptide, a red algae peptide, a Codium fragile peptide, a rice peptide, an Arabidopsis peptide, or a Marchantia peptide.

16. Use of a composition comprising at least one peptide of claims 1-10 or at least one non-immune-based organic agent that specifically targets at least one peptide of claims 1-10 for the preparation of a pharmaceutical composition for stimulating the immune system of a subject to produce antibodies that bind specifically to at least one peptide of claims 1-10.

17. The use of claim 16, wherein the at least one non-immune-based organic agent is nucleic acid.

18. The use of claim 17, wherein the nucleic acid is in antisense configuration.

19. The use of claim 16, wherein the dosage is administered as an approximately 1 mg dosage form.

20. A method for increasing the replication rate of an organism comprising transforming a gene encoding an enzyme having a replication function in the organism with at least one Replikin structure encoding at least one peptide of claims 1-10.

21. The method of claim 20, wherein the organism is a food plant or a rice plant.


SEQUENCE LISTING

<110> BOGOCH, SAMUEL
      BOGOCH, ELENORE S.

<120> REPLIKIN PEPTIDES AND METHODS OF USE

<130> 09425-46976

<140>
<141>

<150> 60/303,396
<151> 2001-07-09

<150> 60/278,761
<151> 2001-03-27

<150> 09/146,755
<151> 1998-09-04

<150> 09/817,144
<151> 2001-03-27

<150> 08/198,139
<151> 1994-02-17

<160> 535

<170> PatentIn 2.1

<210> 1
<211> 10
<212> PRT
<213> Artificial Sequence

<220>
<223> Description of Artificial Sequence: Synthetic
      glioma replikin

<400> 1
Lys Ala Gly Val Ala Phe Leu His Lys Lys
 1               5                  10


<210> 2
<211> 13
<212> PRT
<213> Saccharomyces cerevisiae

<400> 2
His Ser Ile Lys Arg Glu Leu Gly Ile Ile Phe Asp Lys
 1               5                  10


<210> 3
<211> 10

<212> PRT
<213> Gemini vinis virus

<400> 3
His Lys Gln Lys Ile Val Ala Pro Val Lys
1               5                   10


<210> 4
<211> 16
<212> PRT
<213> Unknown Organism

<220>
<223> Description of Unknown Organism: Virus recognin

<400> 4
Tyr Lys Ala Gly Val Ala Phe Leu His Lys Lys Asn Asp Ile Asp Glu
1               5                   10                  15


<210> 5
<211> 10
<212> PRT
<213> Human immunodeficiency virus type 1

<400> 5
Lys Cys Phe Asn Cys Gly Lys Glu Gly His
1               5                   10


<210> 6
<211> 11
<212> PRT
<213> Human immunodeficiency virus type 1

<400> 6
Lys Val Tyr Leu Ala Trp Val Pro Ala His Lys
1               5                   10


<210> 7
<211> 10
<212> PRT
<213> Human immunodeficiency virus type 2

<400> 7
Lys Cys Trp Asn Cys Gly Lys Glu Gly His
1               5                   10


<210> 8
<211> 11
<212> PRT
<213> Maize streak virus

<400> 8
Lys Tyr Ile Val Cys Ala Arg Glu Ala His Lys
 1               5                   10


<210> 9
<211> 17
<212> PRT
<213> Maize streak virus

<400> 9
Lys Glu Lys Lys Pro Ser Lys Asp Glu Ile Met Arg Asp Ile Ile Ser
 1               5                   10                  15

His


<210> 10
<211> 9
<212> PRT
<213> Staphylococcus aureus

<400> 10
Lys Lys Glu Lys Thr Thr His Asn Lys
 1               5


<210> 11
<211> 10
<212> PRT
<213> Bovine herpesvirus 4

<400> 11
His Lys Ile Asn Ile Thr Asn Gly Gln Lys
 1               5                   10


<210> 12
<211> 10
<212> PRT
<213> Meleagrid herpesvirus 1

<400> 12
His Lys Asp Leu Tyr Arg Leu Leu Met Lys
 1               5                   10


<210> 13
<211> 15
<212> PRT
<213> Unknown Organsim

<220>
<223> Description of Unknown Organism: Virus recognin

<400> 13
Lys Phe Arg Ile Asn Ala Lys Asn Tyr Phe Leu Thr Tyr Pro His
 1               5                   10                  15

<210> 14
<211> 19
<212> PRT
<213> Unknown Organism

<220>
<223> Description of Unknown Organism: Virus recognin

<400> 14
Lys Asn Leu Glu Thr Pro Val Asn Lys Leu Phe Ile Arg Ile Cys Arg
 1               5                   10                  15

Glu Phe His


<210> 15
<211> 14
<212> PRT
<213> Unknown Organism

<220>
<223> Description of Unknown Organism: Virus recognin

<400> 15
His Pro Asn Ile Gln Ala Ala Lys Ser Ser Thr Asp Val Lys
 1               5                   10


<210> 16
<211> 19
<212> PRT
<213> Unknown Organism

<220>
<223> Description of Unknown Organism: Virus recognin

<400> 16
Lys Ser Ser Thr Asp Val Lys Ala Tyr Met Asp Lys Asp Gly Asp Val
 1               5                   10                  15

Leu Asp His


<210> 17
<211> 21
<212> PRT
<213> Unknown Organism

<220>
<223> Description of Unknown Organism: Virus recognin

<400> 17
Lys Ala Ser Ala Leu Asn Ile Leu Arg Glu Lys Ala Pro Lys Asp Phe
 1               5                   10                  15

Val Leu Gln Phe His
            20


<210> 18
<211> 13
<212> PRT
<213> Hepatitis C virus

<400> 18
His Tyr Pro Pro Lys Pro Gly Cys Ile Val Pro Ala Lys
1               5                   10


<210> 19
<211> 4
<212> PRT
<213> Homo sapiens

<400> 19
Tyr Lys Ala Gly
1


<210> 20
<211> 6
<212> PRT
<213> Homo sapiens

<400> 20
Tyr Lys Ala Gly Val Ala
1               5


<210> 21
<211> 7
<212> PRT
<213> Homo sapiens

<400> 21
Tyr Lys Ala Gly Val Ala Phe
1               5


<210> 22
<211> 7
<212> PRT
<213> Homo sapiens

<400> 22
Tyr Lys Ala Gly Val Ala Phe
1               5


<210> 23
<211> 9
<212> PRT
<213> Homo sapiens

<400> 23
Ala Gly Val Ala Phe His Lys Lys Asn
1               5

<210> 24
<211> 4
<212> PRT
<213> Homo sapiens

<400> 24
Gly Val Ala Phe
1

<210> 25
<211> 3
<212> PRT
<213> Homo sapiens

<400> 25
Val Ala Phe
1

<210> 26
<211> 7
<212> PRT
<213> Homo sapiens

<400> 26
Val Ala Phe Leu His Lys Lys
1               5

<210> 27
<211> 7
<212> PRT
<213> Homo sapiens

<400> 27
Val Ala Phe Leu His Lys Lys
1               5

<210> 28
<211> 9
<212> PRT
<213> Homo sapiens

<400> 28
Val Ala Phe Leu His Lys Lys Asn Asp
1               5

<210> 29
<211> 8

<212> PRT
<213> Homo sapiens

<400> 29
Val Ala Phe His Lys Lys Asn Asp
1               5


<210> 30
<211> 4
<212> PRT
<213> Homo sapiens

<400> 30
Ala Phe Leu His
1


<210> 31
<211> 8
<212> PRT
<213> Homo sapiens

<400> 31
His Lys Lys Asn Asp Ile Asp Glu
1               5


<210> 32
<211> 6
<212> PRT
<213> Homo sapiens

<400> 32
Lys Lys Asn Asp Ile Asp
1               5


<210> 33
<211> 6
<212> PRT
<213> Homo sapiens

<400> 33
Lys Asn Asp Ile Asp Glu
1               5


<210> 34
<211> 8
<212> PRT
<213> Caldophera prolifera

<400> 34
Lys Ala Ser Lys Phe Thr Lys His
1               5

<210> 35
<211> 12
<212> PRT
<213> Isolepis prolifera

<400> 35
Lys Ala Gln Ala Glu Thr Gly Glu Ile Lys Gly His
1               5                   10


<210> 36
<211> 10
<212> PRT
<213> Schizosaccharomyces pombe

<400> 36
Lys Ser Phe Lys Tyr Pro Lys Lys His Lys
1               5                   10


<210> 37
<211> 10
<212> PRT
<213> Oryza sativa

<400> 37
Lys Lys Ala Tyr Gly Asn Glu Leu His Lys
1               5                   10


<210> 38
<211> 9
<212> PRT
<213> Penicillium marneffei

<400> 38
Lys Val Asp Ile Val Thr His Gln Lys
1               5


<210> 39
<211> 12
<212> PRT
<213> Diseula dcstructiva

<400> 39
Lys Leu Glu Glu Asp Ala Ala Tyr His Arg Lys Lys
1               5                   10


<210> 40
<211> 17
<212> PRT
<213> Ophiostoma novo-ulmi

<400> 40
Lys Val Ile Leu Pro Leu Arg Gly Asn Ile Lys Gly Ile Phe Phe Lys
1               5                   10                  15

His

<210> 41
<211> 11
<212> PRT
<213> Entamoeba invadens

<400> 41
Lys Leu Ile Leu Lys Gly Asp Leu Asn Lys His
1               5                   10

<210> 42
<211> 8
<212> PRT
<213> Helicobacter pylori

<400> 42
Lys Ser Val His Ala Phe Leu Lys
1               5

<210> 43
<211> 9
<212> PRT
<213> Mycoplasma pulmonis

<400> 43
Lys Val His Phe Phe Gln Leu Lys Lys
1               5

<210> 44
<211> 9
<212> PRT
<213> Arabidopsis thaliana

<400> 44
Lys Asp His Asp Phe Asp Gly Asp Lys
1               5

<210> 45
<211> 11
<212> PRT
<213> Arabidopsis thaliana

<400> 45
Lys Met Lys Gly Leu Lys Gln Lys Lys Ala His
1               5                   10

<210> 46
<211> 12
<212> PRT
<213> Arabidopsis thaliana

<400> 46
Lys Glu Leu Ser Ser Thr Thr Gln Glu Lys Ser His
1               5                   10


<210> 47
<211> 9
<212> PRT
<213> Feline immunodeficiency virus

<400> 47
His Leu Lys Asp Tyr Lys Leu Val Lys
1               5


<210> 48
<211> 7
<212> PRT
<213> Rous sarcoma virus

<400> 48
Lys Lys Leu Arg His Glu Lys
1               5


<210> 49
<211> 7
<212> PRT
<213> Avian sarcoma virus

<400> 49
Lys Lys Leu Arg His Asp Lys
1               5


<210> 50
<211> 7
<212> PRT
<213> Homo sapiens

<400> 50
Lys Lys Leu Arg His Asp Lys
1               5


<210> 51
<211> 7
<212> PRT
<213> Avian sarcoma virus

<400> 51
Lys Lys Leu Arg His Glu Lys
1               5


<210> 52
<211> 7

<212> PRT
<213> Homo sapiens

<400> 52
Lys Lys Leu Arg His Glu Lys
1               5


<210> 53
<211> 8
<212> PRT
<213> Homo sapiens

<400> 53
Lys Gln Ala His Glu Leu Ala Lys
1               5


<210> 54
<211> 8
<212> PRT
<213> Polyama virus

<400> 54
Lys Thr His Arg Phe Ser Lys His
1               5


<210> 55
<211> 8
<212> PRT
<213> Sindbis virus

<400> 55
Lys Asn Leu His Glu Lys Ile Lys
1               5


<210> 56
<211> 9
<212> PRT
<213> Human papilloamavirus type 71

<400> 56
Lys His Arg Pro Leu Leu Gln Leu Lys
1               5


<210> 57
<211> 7
<212> PRT
<213> Avian encephalomyelitis virus

<400> 57
Lys Ser Pro Asn His Val Lys
1               5

<210> 58
<211> 8
<212> PRT
<213> Feline sarcoma virus

<400> 58
Lys Asn Ile His Leu Glu Lys Lys
1               5


<210> 59
<211> 8
<212> PRT
<213> Homo sapiens

<400> 59
Lys Asn Ile His Leu Glu Lys Lys
1               5


<210> 60
<211> 10
<212> PRT
<213> Polyoma virus

<400> 60
Lys Pro His Leu Ala Gln Ser Leu Glu Lys
1               5                   10


<210> 61
<211> 9
<212> PRT
<213> Polyoma virus

<400> 61
Lys Gln His Arg Glu Leu Lys Asp Lys
1               5


<210> 62
<211> 9
<212> PRT
<213> Polyoma virus

<400> 62
Lys Gln His Arg Glu Leu Lys Asp Lys
1               5


<210> 63
<211> 12
<212> PRT
<213> Murine leukemia virus

<400> 63
Lys Val Pro Val Leu Ile Ser Pro Thr Leu Lys His
1               5                   10

<210> 64
<211> 13
<212> PRT
<213> Human T-cell lymphotropic virus type 2

<400> 64
Lys Ser Leu Leu Leu Glu Val Asp Lys Asp Ile Ser His
1               5                   10


<210> 65
<211> 13
<212> PRT
<213> Homo sapiens

<400> 65
Lys Ala Gly Ile Thr Ile Met Val Lys Arg Glu Tyr His
1               5                   10


<210> 66
<211> 8
<212> PRT
<213> Homo sapiens

<400> 66
Lys Ser Gly Lys His Leu Gly Lys
1               5


<210> 67
<211> 9
<212> PRT
<213> Homo sapiens

<400> 67
Lys Arg Arg Glu Gln Leu Lys His Lys
1               5


<210> 68
<211> 10
<212> PRT
<213> Homo sapiens

<400> 68
Lys Ser Phe Glu Val Ile Lys Val Ile His
1               5                   10


<210> 69
<211> 8
<212> PRT
<213> Homo sapiens

<400> 69
Lys Lys Lys His Thr Val Lys Lys
1               5

<210> 70
<211> 9
<212> PRT
<213> Homo sapiens

<400> 70
Lys Ala Gln Lys Asp His Leu Ser Lys
1               5

<210> 71
<211> 10
<212> PRT
<213> Homo sapiens

<400> 71
His Leu Lys Arg Val Lys Asp Leu Lys Lys
1               5                   10

<210> 72
<211> 11
<212> PRT
<213> Homo sapiens

<400> 72
Lys Tyr Gly Ser Pro Lys His Arg Leu Ile Lys
1               5                   10

<210> 73
<211> 13
<212> PRT
<213> Papilloma virus type 11

<400> 73
Lys Leu Lys His Ile Leu Gly Lys Ala Arg Phe Ile Lys
1               5                   10

<210> 74
<211> 12
<212> PRT
<213> Homo sapiens

<400> 74
Lys Gly Asp His Val Lys His Tyr Lys Ile Arg Lys
1               5                   10

<210> 75
<211> 13
<212> PRT

<213> Homo sapiens

<400> 75
Lys Glu Lys Leu Arg Asp Val Met Val Asp Arg His Lys
1               5                   10


<210> 76
<211> 15
<212> PRT
<213> Homo sapiens

<400> 76
Lys Leu Gln Ala Arg Gln Gln Gln Leu Leu Lys Lys Ile Glu His
1               5                   10                  15


<210> 77
<211> 14
<212> PRT
<213> Homo sapiens

<400> 77
Lys Lys Gly Asn Arg Val Ser Pro Thr Met Lys Val Thr His
1               5                   10


<210> 78
<211> 9
<212> PRT
<213> Homo sapiens

<400> 78
Lys Glu Ile Pro Leu His Phe Arg Lys
1               5


<210> 79
<211> 8
<212> PRT
<213> Homo sapiens

<400> 79
Lys Lys Lys Pro His Ile Lys Lys
1               5


<210> 80
<211> 9
<212> PRT
<213> Homo sapiens

<400> 80
Lys Thr Arg His Asp Pro Leu Ala Lys
1               5


<210> 81

<211> 10
<212> PRT
<213> Homo sapiens

<400> 81
Lys His His Pro Lys Asp Asn Leu Ile Lys
1               5                   10


<210> 82
<211> 10
<212> PRT
<213> Homo sapiens

<400> 82
Lys His Lys Arg Lys Lys Phe Arg Gln Lys
1               5                   10


<210> 83
<211> 10
<212> PRT
<213> Homo sapiens

<400> 83
Lys Ala Gly Val Ala Phe Leu His Lys Lys
1               5                   10


<210> 84
<211> 10
<212> PRT
<213> Homo sapiens

<400> 84
Lys His Lys Arg Lys Lys Phe Arg Gln Lys
1               5                   10


<210> 85
<211> 10
<212> PRT
<213> Homo sapiens

<400> 85
Lys Lys Lys Ser Lys Lys His Lys Asp Lys
1               5                   10


<210> 86
<211> 11
<212> PRT
<213> Homo sapiens

<400> 86
His Lys Ser Glu Lys Pro Ala Leu Pro Arg Lys
1               5                   10

<210> 87
<211> 14
<212> PRT
<213> Homo sapiens

<400> 87
Lys Lys Lys Lys Pro Ser Arg Leu Lys Gly Asp Asn Glu Lys
1               5                   10


<210> 88
<211> 16
<212> PRT
<213> Homo sapiens

<400> 88
Lys Thr Lys Lys Gly Asn Arg Val Ser Pro Thr Met Lys Val Thr His
1               5                   10                  15


<210> 89
<211> 18
<212> PRT
<213> Homo sapiens

<400> 89
Lys His Lys Glu Lys Met Ser Lys Asp Gly Lys Lys Lys Lys Lys Lys
1               5                   10                  15

Ser Lys


<210> 90
<211> 9
<212> PRT
<213> Legionella sp.

<400> 90
Lys Ile His Leu Ile Ser Val Lys Lys
1               5


<210> 91
<211> 10
<212> PRT
<213> Bacillus anthracis

<400> 91
His Val Lys Lys Glu Lys Glu Lys Asn Lys
1               5                   10


<210> 92
<211> 9
<212> PRT
<213> Bacillus anthracis

<400> 92
Lys His Ile Val Lys Ile Glu Val Lys
1               5

<210> 93
<211> 16
<212> PRT
<213> Bacillus anthracis

<400> 93
Lys Lys Lys Lys Ile Lys Asp Ile Tyr Gly Lys Asp Ala Leu Leu His
1               5                   10                  15

<210> 94
<211> 8
<212> PRT
<213> Bacillus anthracis

<400> 94
Lys Trp Glu Lys Ile Lys Gln His
1               5

<210> 95
<211> 18
<212> PRT
<213> Bacillus anthracis

<400> 95
Lys Lys Leu Gln Ile Pro Pro Pro Ile Glu Pro Lys Lys Asp Asp Ile
1               5                   10                  15

Ile His

<210> 96
<211> 31
<212> PRT
<213> Bacillus anthracis

<400> 96
His Asn Arg Tyr Ala Ser Asn Ile Val Glu Ser Ala Tyr Leu Leu Ile
1               5                   10                  15

Leu Asn Glu Trp Lys Asn Asn Ile Gln Ser Asp Leu Ile Lys Lys
            20                  25                  30

<210> 97
<211> 24
<212> PRT
<213> Bacillus anthracis

<400> 97
His Ala Val Asp Asp Tyr Ala Gly Tyr Leu Leu Asp Lys Asn Gln Ser
1               5                   10                  15

Asp Leu Val Thr Asn Ser Lys Lys
            20

<210> 98
<211> 13
<212> PRT
<213> Bacillus anthracis

<400> 98
His Ala Glu Arg Leu Lys Val Gln Lys Asn Ala Pro Lys
1               5                   10

<210> 99
<211> 10
<212> PRT
<213> Variola virus

<400> 99
Lys His Tyr Asn Asn Ile Thr Trp Tyr Lys
1               5                   10

<210> 100
<211> 12
<212> PRT
<213> Variola virus

<400> 100
Lys Tyr Ser Gln Thr Gly Lys Glu Leu Ile Ile His
1               5                   10

<210> 101
<211> 17
<212> PRT
<213> Variola virus

<400> 101
His Tyr Asp Asp Val Arg Ile Lys Asn Asp Ile Val Val Ser Arg Cys
1               5                   10                  15

Lys

<210> 102
<211> 11
<212> PRT
<213> Variola virus

<400> 102
His Arg Phe Lys Leu Ile Leu Asp Ser Lys Ile
1               5                   10

<210> 103
<211> 11
<212> PRT
<213> Variola virus

<400> 103
Lys Glu Arg Gly His Asn Tyr Tyr Phe Glu Lys
1               5                   10


<210> 104
<211> 8
<212> PRT
<213> Influenza B virus

<400> 104
Lys Ser His Phe Ala Asn Leu Lys
1               5


<210> 105
<211> 11
<212> PRT
<213> Influenza B virus

<400> 105
Lys Ser His Phe Ala Asn Leu Lys Gly Thr Lys
1               5                   10


<210> 106
<211> 19
<212> PRT
<213> Influenza B virus

<400> 106
Lys Ser His Phe Ala Asn Leu Lys Gly Thr Lys Thr Arg Gly Lys Leu
1               5                   10                  15

Cys Pro Lys


<210> 107
<211> 9
<212> PRT
<213> Influenza B virus

<400> 107
His Glu Lys Tyr Gly Gly Leu Asn Lys
1               5


<210> 108
<211> 11
<212> PRT
<213> Influenza B virus

<400> 108
His Glu Lys Tyr Gly Gly Leu Asn Lys Ser Lys
1               5                   10

<210> 109
<211> 20
<212> PRT
<213> Influenza B virus

<400> 109
His Glu Lys Tyr Gly Gly Leu Asn Lys Ser Lys Pro Tyr Tyr Thr Gly
1               5                   10                  15

Glu His Ala Lys
            20

<210> 110
<211> 13
<212> PRT
<213> Influenza B virus

<400> 110
His Ala Lys Ala Ile Gly Asn Cys Pro Ile Trp Val Lys
1               5                   10

<210> 111
<211> 23
<212> PRT
<213> Influenza B virus

<400> 111
His Ala Lys Ala Ile Gly Asn Cys Pro Ile Trp Val Lys Thr Pro Leu
1               5                   10                  15

Lys Leu Ala Asn Gly Thr Lys
            20

<210> 112
<211> 29
<212> PRT
<213> Influenza B virus

<400> 112
His Ala Lys Ala Ile Gly Asn Cys Pro Ile Trp Val Lys Thr Pro Leu
1               5                   10                  15

Lys Leu Ala Asn Gly Thr Lys Tyr Arg Pro Pro Ala Lys
            20                  25

<210> 113
<211> 32
<212> PRT
<213> Influenza B virus

<400> 113
His Ala Lys Ala Ile Gly Asn Cys Pro Ile Trp Val Lys Thr Pro Leu
1               5                   10                  15

Lys Leu Ala Asn Gly Thr Lys Tyr Arg Pro Pro Ala Lys Leu Leu Lys
            20                  25                  30


<210> 114
<211> 13
<212> PRT
<213> Influenza B virus

<400> 114
His Phe Ala Asn Leu Lys Gly Thr Lys Thr Arg Gly Lys
1               5                   10


<210> 115
<211> 17
<212> PRT
<213> Influenza B virus

<400> 115
His Phe Ala Asn Leu Lys Gly Thr Lys Thr Arg Gly Lys Leu Cys Pro
1               5                   10                  15

Lys


<210> 116
<211> 16
<212> PRT
<213> Influenza B virus

<400> 116
His Ser Asp Asn Glu Ile Gln Met Val Lys Leu Tyr Gly Asp Ser Lys
1               5                   10                  15


<210> 117
<211> 21
<212> PRT
<213> Influenza B virus

<400> 117
His Ser Asp Asn Glu Ile Gln Asp Lys Met Val Lys Leu Tyr Gly Asp
1               5                   10                  15

Ser Lys Pro Gln Lys
            20


<210> 118
<211> 19
<212> PRT
<213> Influenza B virus

<400> 118
His Ser Asp Asn Glu Ile Gln Met Val Lys Leu Tyr Gly Asp Ser Lys
1               5                   10                  15

Pro Gln Lys


<210> 119
<211> 9
<212> PRT
<213> Influenza B virus

<220>
<221> MOD_RES
<222> (2)
<223> ala or val

<400> 119
Lys Xaa Ser Ile Leu His Glu Val Lys
1               5


<210> 120
<211> 15
<212> PRT
<213> Influenza B virus

<400> 120
Lys Cys Thr Gly Thr Ile Pro Ser Ala Lys Ala Ser Ile Leu His
1               5                   10                  15


<210> 121
<211> 18
<212> PRT
<213> Influenza B virus

<400> 121
Lys Cys Thr Gly Thr Ile Pro Ser Ala Lys Ala Ser Ile Leu His Glu
1               5                   10                  15

Val Lys


<210> 122
<211> 16
<212> PRT
<213> Influenza B virus

<400> 122
Lys Tyr Gly Gly Leu Asn Lys Ser Lys Pro Tyr Tyr Thr Gly Glu His
1               5                   10                  15


<210> 123
<211> 26
<212> PRT

<213> Influenza B virus

<400> 123
Lys Val Trp Cys Ala Ser Gly Arg Ser Lys Val Ile Lys Gly Ser Leu
1               5                   10                  15

Pro Leu Ile Gly Glu Ala Asp Cys Leu His
            20                  25


<210> 124
<211> 10
<212> PRT
<213> Influenza B virus

<400> 124
Lys Pro Tyr Tyr Thr Gly Glu His Ala Lys
1               5                   10


<210> 125
<211> 18
<212> PRT
<213> Influenza B virus

<400> 125
Lys Cys Met Gly Thr Ile Pro Ser Ala Lys Ala Ser Ile Leu His Glu
1               5                   10                  15

Val Lys


<210> 126
<211> 15
<212> PRT
<213> Influenza B virus

<400> 126
His Asn Val Ile Asn Ala Glu Lys Ala Pro Gly Gly Pro Tyr Lys
1               5                   10                  15


<210> 127
<211> 16
<212> PRT
<213> Influenza B virus

<400> 127
His Ser Asp Asn Glu Thr Gln Met Ala Lys Leu Tyr Gly Asp Ser Lys
1               5                   10                  15


<210> 128
<211> 18
<212> PRT
<213> Influenza B virus

<400> 128
His Gly Val Ala Val Ala Ala Asp Leu Lys Ser Thr Gln Glu Ala Ile
1               5                   10                  15

Asn Lys


<210> 129
<211> 29
<212> PRT
<213> Influenza B virus

<400> 129
His Gly Val Ala Val Ala Ala Asp Leu Lys Ser Thr Gln Glu Ala Ile
1               5                   10                  15

Asn Lys Asp Thr Ile Ser Thr Gln Glu Ala Ile Asn Lys
            20                  25


<210> 130
<211> 21
<212> PRT
<213> Influenza B virus

<400> 130
Lys Leu Tyr Gly Asp Ser Lys Pro Gln Lys Phe Thr Ser Ser Ala Asn
1               5                   10                  15

Gly Val Thr Thr His
            20


<210> 131
<211> 19
<212> PRT
<213> Influenza B virus

<400> 131
His Ser Asp Asn Glu Thr Gln Met Ala Lys Leu Tyr Gly Asp Ser Lys
1               5                   10                  15

Pro Gln Lys


<210> 132
<211> 13
<212> PRT
<213> Influenza B virus

<400> 132
His Phe Ala Asn Leu Lys Gly Thr Gln Thr Arg Gly Lys
1               5                   10


<210> 133
<211> 12

<212> PRT
<213> Influenza B virus

<400> 133
Lys Pro Arg Ser Ala Leu Lys Cys Lys Gly Phe His
1               5                   10


<210> 134
<211> 22
<212> PRT
<213> Influenza B virus

<220>
<221> MOD_RES
<222> (15)
<223> gly or ala

<400> 134
Lys Ser Lys Pro Tyr Tyr Thr Gly Glu His Ala Lys Ala Ile Xaa Asn
1               5                   10                  15

Cys Pro Ile Trp Val Lys
            20


<210> 135
<211> 16
<212> PRT
<213> Influenza virus

<220>
<221> MOD_RES
<222> (3)
<223> val or ile

<220>
<221> MOD_RES
<222> (13)
<223> arg or lys

<220>
<221> MOD_RES
<222> (14)
<223> ser or thr

<220>
<221> MOD_RES
<222> (15)
<223> thr or ala

<400> 135
His Pro Xaa Thr Ile Gly Glu Cys Pro Lys Tyr Val Xaa Xaa Xaa Lys
1               5                   10                  15


<210> 136
<211> 21

<212> PRT
<213> Influenza virus

<220>
<221> MOD_RES
<222> (10)
<223> glu or gly

<220>
<221> MOD_RES
<222> (13)
<223> lys or arg

<220>
<221> MOD_RES
<222> (14)
<223> asn or ser

<220>
<221> MOD_RES
<222> (17)
<223> lys or arg

<400> 136
His Asp Ser Asn Val Lys Asn Leu Tyr Xaa Lys Val Xaa Xaa Gln Leu
1               5                   10                  15

Xaa Asn Asn Ala Lys
            20


<210> 137
<211> 17
<212> PRT
<213> Influenza virus

<220>
<221> MOD_RES
<222> (10)
<223> glu or gly

<220>
<221> MOD_RES
<222> (13)
<223> lys or arg

<220>
<221> MOD_RES
<222> (14)
<223> asn or ser

<400> 137
His Asp Ser Asn Val Lys Asn Leu Tyr Xaa Lys Val Xaa Xaa Gln Leu
1               5                   10                  15

Lys

<210> 138
<211> 36
<212> PRT
<213> Influenza virus

<220>
<221> MOD_RES
<222> (4)..(5)
<223> asn or asp

<220>
<221> MOD_RES
<222> (6)
<223> ala, thr or glu

<220>
<221> MOD_RES
<222> (12)
<223> arg or lys

<220>
<221> MOD_RES
<222> (31)
<223> glu or lys

<400> 138
His Lys Cys Xaa Xaa Xaa Cys Met Glu Ser Val Xaa Asn Gly Thr Tyr
1               5                   10                  15

Asp Tyr Pro Lys Tyr Ser Glu Glu Ser Lys Leu Asn Arg Glu Xaa Ile
            20                  25                  30

Asp Gly Val Lys
        35


<210> 139
<211> 26
<212> PRT
<213> Influenza virus

<220>
<221> MOD_RES
<222> (4)..(5)
<223> asn or asp

<220>
<221> MOD_RES
<222> (6)
<223> ala, thr or glu

<220>
<221> MOD_RES
<222> (12)
<223> arg or lys

<400> 139
His Lys Cys Xaa Xaa Xaa Cys Met Glu Ser Val Xaa Asn Gly Thr Tyr
1               5                   10                  15

Asp Tyr Pro Lys Tyr Ser Glu Glu Ser Lys
            20                  25


<210> 140
<211> 50
<212> PRT
<213> Influenza virus

<220>
<221> MOD_RES
<222> (4)
<223> glu or gly

<220>
<221> MOD_RES
<222> (21)
<223> asp or asn

<400> 140
His Gln Asn Xaa Gln Gly Ser Gly Tyr Ala Ala Asp Gln Lys Ser Thr
1               5                   10                  15

Gln Asn Ala Ile Xaa Gly Ile Thr Asn Lys Val Asn Ser Val Ile Glu
            20                  25                  30

Lys Met Asn Thr Gln Phe Thr Ala Val Gly Lys Glu Phe Asn Lys Leu
        35                  40                  45

Glu Lys
    50


<210> 141
<211> 33
<212> PRT
<213> Influenza virus

<220>
<221> MOD_RES
<222> (4)
<223> glu or gly

<220>
<221> MOD_RES
<222> (21)
<223> asp or asn

<400> 141
His Gln Asn Xaa Gln Gly Ser Gly Tyr Ala Ala Asp Gln Lys Ser Thr
1               5                   10                  15

Gln Asn Ala Ile Xaa Gly Ile Thr Asn Lys Val Asn Ser Val Ile Glu
            20                  25                  30

Lys

<210> 142
<211> 26
<212> PRT
<213> Influenza virus

<220>
<221> MOD_RES
<222> (4)
<223> glu or gly

<220>
<221> MOD_RES
<222> (21)
<223> asp or asn

<400> 142
His Gln Asn Xaa Gln Gly Ser Gly Tyr Ala Ala Asp Gln Lys Ser Thr
1               5                   10                  15

Gln Asn Ala Ile Xaa Gly Ile Thr Asn Lys
            20                  25

<210> 143
<211> 14
<212> PRT
<213> Influenza virus

<400> 143
Lys Phe Glu Ile Phe Pro Lys Thr Ser Ser Trp Pro Asn His
1               5                   10

<210> 144
<211> 27
<212> PRT
<213> Influenza virus

<220>
<221> MOD_RES
<222> (3)
<223> asn, ser or thr

<220>
<221> MOD_RES
<222> (9)
<223> asn or ser

<220>
<221> MOD_RES
<222> (13)
<223> val or thr

<400> 144
Lys Gly Xaa Ser Tyr Pro Lys Leu Xaa Lys Ser Tyr Xaa Asn Asn Lys
1               5                   10                  15

Gly Lys Glu Val Leu Val Leu Trp Gly Val His
            20                  25

<210> 145
<211> 18
<212> PRT
<213> Influenza virus

<220>
<221> MOD_RES
<222> (4)
<223> val or thr

<400> 145
Lys Ser Tyr Xaa Asn Asn Lys Gly Lys Glu Val Leu Val Leu Trp Gly
1               5                   10                  15

Val His

<210> 146
<211> 36
<212> PRT
<213> Influenza virus

<400> 146
His Lys Cys Asn Asn Glu Cys Met Glu Ser Val Lys Asn Gly Thr Tyr
1               5                   10                  15

Asp Tyr Pro Lys Tyr Ser Glu Glu Ser Lys Leu Asn Arg Glu Lys Ile
            20                  25                  30

Asp Gly Val Lys
        35

<210> 147
<211> 26
<212> PRT
<213> Influenza virus

<400> 147
His Lys Cys Asn Asn Glu Cys Met Glu Ser Val Lys Asn Gly Thr Tyr
1               5                   10                  15

Asp Tyr Pro Lys Tyr Ser Glu Glu Ser Lys
            20                  25

<210> 148
<211> 20
<212> PRT
<213> Influenza virus

<400> 148
His Lys Cys Asn Asn Glu Cys Met Glu Ser Val Lys Asn Gly Thr Tyr
1               5                   10                  15

Asp Tyr Pro Lys
            20


<210> 149
<211> 12
<212> PRT
<213> Influenza virus

<400> 149
His Lys Cys Asn Asn Glu Cys Met Glu Ser Val Lys
1               5                   10


<210> 150
<211> 34
<212> PRT
<213> Influenza virus

<220>
<221> MOD_RES
<222> (9)
<223> lys or arg

<220>
<221> MOD_RES
<222> (16)
<223> glu or gly

<400> 150
His Asn Gly Lys Ser Ser Phe Tyr Xaa Asn Leu Leu Trp Leu Thr Xaa
1               5                   10                  15

Lys Asn Gly Leu Tyr Pro Asn Leu Ser Lys Ser Tyr Val Asn Asn Lys
            20                  25                  30

Glu Lys


<210> 151
<211> 32
<212> PRT
<213> Influenza virus

<220>
<221> MOD_RES
<222> (9)
<223> lys or arg

<220>
<221> MOD_RES
<222> (16)
<223> glu or gly

<400> 151
His Asn Gly Lys Ser Ser Phe Tyr Xaa Asn Leu Leu Trp Leu Thr Xaa
1               5                   10                  15

Lys Asn Gly Leu Tyr Pro Asn Leu Ser Lys Ser Tyr Val Asn Asn Lys
            20                  25                  30


<210> 152
<211> 26
<212> PRT
<213> Influenza virus

<220>
<221> MOD_RES
<222> (9)
<223> lys or arg

<220>
<221> MOD_RES
<222> (16)
<223> glu or gly

<400> 152
His Asn Gly Lys Ser Ser Phe Tyr Xaa Asn Leu Leu Trp Leu Thr Xaa
1               5                   10                  15

Lys Asn Gly Leu Tyr Pro Asn Leu Ser Lys
            20                  25


<210> 153
<211> 17
<212> PRT
<213> Influenza virus

<220>
<221> MOD_RES
<222> (9)
<223> lys or arg

<220>
<221> MOD_RES
<222> (16)
<223> glu or gly

<400> 153
His Asn Gly Lys Ser Ser Phe Tyr Xaa Asn Leu Leu Trp Leu Thr Xaa
1               5                   10                  15

Lys


<210> 154
<211> 40
<212> PRT
<213> Influenza virus

<400> 154
Lys Ser Ser Phe Tyr Lys Asn Leu Leu Trp Leu Thr Glu Lys Asn Gly
1               5                   10                  15

Leu Tyr Pro Asn Leu Ser Lys Ser Tyr Val Asn Asn Lys Glu Lys Glu
            20                  25                  30

Val Leu Val Leu Trp Gly Val His
        35                  40


<210> 155
<211> 35
<212> PRT
<213> Influenza virus

<400> 155
Lys Asn Leu Leu Trp Leu Thr Glu Lys Asn Gly Leu Tyr Pro Asn Leu
1               5                   10                  15

Ser Lys Ser Tyr Val Asn Asn Lys Glu Lys Glu Val Leu Val Leu Trp
            20                  25                  30

Gly Val His
        35


<210> 156
<211> 27
<212> PRT
<213> Influenza virus

<400> 156
Lys Asn Gly Leu Tyr Pro Asn Leu Ser Lys Ser Tyr Val Asn Asn Lys
1               5                   10                  15

Glu Lys Glu Val Leu Val Leu Trp Gly Val His
            20                  25


<210> 157
<211> 18
<212> PRT
<213> Influenza virus

<220>
<221> MOD_RES
<222> (4)
<223> val or ala

<220>
<221> MOD_RES
<222> (12)
<223> lys or not present

<220>
<221> MOD_RES

<222> (13)
<223> val or not present

<400> 157
Lys Ser Tyr Xaa Asn Asn Lys Glu Lys Glu Val Xaa Xaa Leu Trp Gly
1               5                   10                  15

Val His


<210> 158
<211> 12
<212> PRT
<213> Influenza virus

<400> 158
Lys Glu Ser Ser Trp Pro Asn His Thr Val Thr Lys
1               5                   10


<210> 159
<211> 44
<212> PRT
<213> Influenza virus

<220>
<221> MOD_RES
<222> (4)
<223> thr or asn

<400> 159
His Glu Thr Xaa Lys Gly Val Thr Ala Ala Cys Pro Tyr Ala Gly Ala
1               5                   10                  15

Ser Ser Phe Tyr Arg Asn Leu Leu Trp Leu Val Lys Lys Glu Asn Ser
            20                  25                  30

Tyr Pro Lys Leu Ser Lys Ser Tyr Val Asn Asn Lys
        35                  40


<210> 160
<211> 38
<212> PRT
<213> Influenza virus

<220>
<221> MOD_RES
<222> (4)
<223> thr or asn

<400> 160
His Glu Thr Xaa Lys Gly Val Thr Ala Ala Cys Pro Tyr Ala Gly Ala
1               5                   10                  15

Ser Ser Phe Tyr Arg Asn Leu Leu Trp Leu Val Lys Lys Glu Asn Ser
            20                  25                  30

Tyr Pro Lys Leu Ser Lys
        35

<210> 161
<211> 22
<212> PRT
<213> Influenza virus

<400> 161
Lys Phe Glu Ile Phe Pro Lys Thr Ser Ser Trp Pro Asn Glu Val Leu
 1               5                   10                  15

Val Leu Trp Gly Val His
            20

<210> 162
<211> 8
<212> PRT
<213> Influenza virus

<400> 162
Lys Glu Arg Ser Trp Pro Lys His
 1               5

<210> 163
<211> 21
<212> PRT
<213> Influenza virus

<400> 163
Lys Leu Ser Lys Ser Tyr Val Asn Asn Lys Glu Lys Glu Val Leu Val
 1               5                   10                  15

Leu Trp Gln Val His
            20

<210> 164
<211> 15
<212> PRT
<213> Influenza virus

<400> 164
Lys Asn Asn Lys Glu Lys Glu Val Leu Val Leu Trp Gln Val His
 1               5                   10                  15

<210> 165
<211> 34
<212> PRT
<213> Influenza virus

<220>
<221> MOD_RES
<222> (2)

<223> lys or asn

<220>
<221> MOD_RES
<222> (3)
<223> gly or gln

<220>
<221> MOD_RES
<222> (9)
<223> arg or lys

<220>
<221> MOD_RES
<222> (20)
<223> lys or ser

<220>
<221> MOD_RES
<222> (23)
<223> asn or thr

<400> 165
His Xaa Xaa Lys Ser Ser Phe Tyr Xaa Asn Leu Leu Trp Leu Thr Glu
1               5                   10                  15

Lys Asn Gly Xaa Tyr Pro Xaa Leu Ser Lys Ser Tyr Ala Asn Asn Lys
            20                  25                  30

Glu Lys


<210> 166
<211> 17
<212> PRT
<213> Influenza virus

<220>
<221> MOD_RES
<222> (2)
<223> lys or asn

<220>
<221> MOD_RES
<222> (3)
<223> gly or gln

<220>
<221> MOD_RES
<222> (9)
<223> arg or lys

<400> 166
His Xaa Xaa Lys Ser Ser Phe Tyr Xaa Asn Leu Leu Trp Leu Thr Glu
1               5                   10                  15

Lys

<210> 167
<211> 9
<212> PRT
<213> Influenza virus

<400> 167
His Ala Lys Lys Ser Ser Phe Tyr Lys
1               5

<210> 168
<211> 11
<212> PRT
<213> Influenza virus

<400> 168
His Asn Gly Lys Leu Cys Arg Leu Lys Gly Lys
1               5                   10

<210> 169
<211> 9
<212> PRT
<213> Influenza virus

<220>
<221> MOD_RES
<222> (7)
<223> gln or gly

<400> 169
His Tyr Lys Leu Asn Asn Xaa Lys Lys
1               5

<210> 170
<211> 25
<212> PRT
<213> Influenza virus

<400> 170
His Asp Ile Tyr Arg Asp Glu Ala Ile Asn Asn Arg Phe Gln Ile Gln
1               5                   10                  15

Gly Val Lys Leu Thr Gln Gly Tyr Lys
            20                  25

<210> 171
<211> 11
<212> PRT
<213> Influenza virus

<400> 171
Lys Gly Asn Gly Cys Phe Glu Ile Phe His Lys
1               5                   10

<210> 172
<211> 18
<212> PRT
<213> Influenza virus

<400> 172
Lys Leu Asn Arg Leu Ile Glu Lys Thr Asn Asp Lys Tyr His Gln Ile
1               5                   10                  15

Glu Lys


<210> 173
<211> 14
<212> PRT
<213> Influenza virus

<400> 173
Lys Leu Asn Arg Leu Ile Glu Lys Thr Asn Asp Lys Tyr His
1               5                   10


<210> 174
<211> 13
<212> PRT
<213> Influenza virus

<400> 174
Lys Cys His Thr Asp Lys Gly Ser Leu Ser Thr Thr Lys
1               5                   10


<210> 175
<211> 16
<212> PRT
<213> Influenza virus

<400> 175
Lys Ile Asn Asn Gly Asp Tyr Ala Lys Leu Tyr Ile Trp Gly Val His
1               5                   10                  15


<210> 176
<211> 17
<212> PRT
<213> Influenza virus

<400> 176
His Asn Gly Lys Leu Cys Arg Lys Gly Ile Ala Pro Leu Gln Leu Gly
1               5                   10                  15

Lys


<210> 177
<211> 38
<212> PRT

<213> Influenza virus

<400> 177
His Glu Thr Asn Arg Gln Val Thr Ala Ala Cys Pro Tyr Ala Gly Ala
1               5                   10                  15

Asn Ser Phe Phe Arg Asn Leu Ile Trp Leu Val Lys Lys Glu Ser Ser
            20                  25                  30

Tyr Pro Lys Leu Ser Lys
        35


<210> 178
<211> 35
<212> PRT
<213> Influenza virus

<400> 178
His Glu Thr Asn Arg Gln Val Thr Ala Ala Cys Pro Tyr Ala Gly Ala
1               5                   10                  15

Asn Ser Phe Phe Arg Asn Leu Ile Trp Leu Val Lys Lys Glu Ser Ser
            20                  25                  30

Tyr Pro Lys
        35


<210> 179
<211> 31
<212> PRT
<213> Influenza virus

<400> 179
His Pro Pro Thr Ser Thr Asp Gln Gln Ser Leu Tyr Gln Asn Ala Asp
1               5                   10                  15

Ala Tyr Ile Phe Val Gly Ser Ser Lys Tyr Asn Arg Lys Phe Lys
            20                  25                  30


<210> 180
<211> 35
<212> PRT
<213> Influenza virus

<400> 180
His Pro Pro Thr Ser Thr Asp Gln Gln Ser Leu Tyr Gln Asn Ala Asp
1               5                   10                  15

Ala Tyr Ile Phe Val Gly Ser Ser Lys Tyr Asn Arg Lys Phe Lys Pro
            20                  25                  30

Glu Ile Ala
        35

<210> 181
<211> 25
<212> PRT
<213> Influenza virus

<400> 181
His Asp Ile Tyr Arg Asp Glu Ala Ile Asn Asn Arg Phe Gln Ile Gln
1               5                   10                  15

Gly Val Lys Ile Thr Gln Gly Tyr Lys
            20                  25


<210> 182
<211> 43
<212> PRT
<213> Influenza virus

<400> 182
His Gln Asn Glu Gln Gly Ser Gly Tyr Ala Ala Asp Gln Lys Ser Thr
1               5                   10                  15

Gln Asn Ala Ile Asp Gly Ile Thr Asn Lys Val Asn Ser Val Ile Glu
            20                  25                  30

Lys Met Asn Thr Gln Phe Thr Ala Val Gly Lys
        35                  40


<210> 183
<211> 33
<212> PRT
<213> Influenza virus

<400> 183
His Gln Asn Glu Gln Gly Ser Gly Tyr Ala Ala Asp Gln Lys Ser Thr
1               5                   10                  15

Gln Asn Ala Ile Asp Gly Ile Thr Asn Lys Val Asn Ser Val Ile Glu
            20                  25                  30

Lys


<210> 184
<211> 50
<212> PRT
<213> Influenza virus

<400> 184
His Gln Asn Glu Gln Gly Ser Gly Tyr Ala Ala Asp Gln Lys Ser Thr
1               5                   10                  15

Gln Asn Ala Ile Asn Gly Ile Thr Asn Lys Val Asn Ser Val Ile Glu
            20                  25                  30

Lys Met Asn Thr Gln Phe Thr Ala Val Gly Lys Glu Phe Asn Lys Leu
        35                  40                  45

Glu Lys
    50


<210> 185
<211> 18
<212> PRT
<213> Influenza virus

<400> 185
His Asn Gly Lys Leu Cys Arg Leu Lys Gly Ile Ala Pro Leu Gln Leu
 1               5                  10                  15

Gly Lys


<210> 186
<211> 12
<212> PRT
<213> Influenza virus

<400> 186
His Lys Cys Asn Asn Glu Cys Met Glu Ser Val Lys
 1               5                  10


<210> 187
<211> 14
<212> PRT
<213> Influenza virus

<400> 187
Lys Phe Glu Ile Phe Pro Lys Ala Ser Ser Trp Pro Asn His
 1               5                  10


<210> 188
<211> 21
<212> PRT
<213> Influenza virus

<400> 188
His Asp Ser Asn Val Lys Asn Leu Tyr Glu Lys Val Arg Ser Gln Leu
 1               5                  10                  15

Arg Asn Asn Ala Lys
            20


<210> 189
<211> 22
<212> PRT
<213> Influenza virus

<400> 189
Lys Val Asn Ser Val Ile Lys Lys Met Asn Thr Gln Phe Ala Ala Val
 1               5                  10                  15

Gly Lys Glu Phe Asn His
            20

<210> 190
<211> 8
<212> PRT
<213> Influenza virus

<400> 190
Lys His Asn Gly Lys Leu Cys Lys
1               5

<210> 191
<211> 28
<212> PRT
<213> Influenza virus

<400> 191
Lys Lys Gly Thr Ser Tyr Pro Lys Leu Ser Lys Ser Tyr Thr His Asn
1               5                   10                  15

Lys Gly Lys Glu Val Leu Val Leu Trp Gly Val His
            20                  25

<210> 192
<211> 27
<212> PRT
<213> Influenza virus

<400> 192
Lys Gly Thr Ser Tyr Pro Lys Leu Ser Lys Ser Tyr Thr His Asn Lys
1               5                   10                  15

Gly Lys Glu Val Leu Val Leu Trp Gly Val His
            20                  25

<210> 193
<211> 21
<212> PRT
<213>·Influenza virus

<400> 193
Lys Leu Ser Lys Ser Tyr Thr His Asn Lys Gly Lys Glu Val Leu Val
1               5                   10                  15

Leu Trp Gly Val His
            20

<210> 194
<211> 18
<212> PRT
<213> Influenza virus

<400> 194
Lys Ser Tyr Thr His Asn Lys Gly Lys Glu Val Leu Val Leu Trp Gly
1               5                   10                  15

Val His


<210> 195
<211> 10
<212> PRT
<213> Influenza virus

<400> 195
Lys Gly Val Thr Ala Ser Cys Ser His Lys
1               5                   10


<210> 196
<211> 34
<212> PRT
<213> Influenza virus

<400> 196
Lys Gly Val Thr Ala Ser Cys Ser His Lys Gly Arg Ser Ser Phe Tyr
1               5                   10                  15

Arg Asn Leu Leu Trp Leu Thr Glu Lys Asn Gly Leu Tyr Pro Asn Leu
            20                  25                  30

Ser Lys


<210> 197
<211> 27
<212> PRT
<213> Influenza virus

<400> 197
Lys Gly Asn Ser Tyr Pro Lys Leu Ser Lys Ser Tyr Val Asn Asn Lys
1               5                   10                  15

Glu Lys Glu Val Leu Val Leu Trp Gly Ile His
            20                  25


<210> 198
<211> 8
<212> PRT
<213> Influenza virus

<400> 198
Lys Glu Phe Asn His Leu Glu Lys
1               5


<210> 199
<211> 39

<212> PRT
<213> Influenza virus

<400> 199
His Pro Pro Thr Ser Thr Asp Gln Gln Ser Leu Tyr Gln Asn Ala Asp
1               5                   10                  15

Ala Tyr Val Phe Val Gly Ser Ser Lys Tyr Asn Lys Lys Phe Lys Pro
            20                  25                  30

Glu Ile Ala Thr Arg Pro Lys
        35


<210> 200
<211> 31
<212> PRT
<213> Influenza virus

<400> 200
His Pro Pro Thr Ser Thr Asp Gln Gln Ser Leu Tyr Gln Asn Ala Asp
1               5                   10                  15

Ala Tyr Val Phe Val Gly Ser Ser Lys Tyr Asn Lys Lys Phe Lys
            20                  25                  30


<210> 201
<211> 31
<212> PRT
<213> Influenza virus

<400> 201
His Glu Gly Lys Ser Ser Phe Tyr Arg Asn Leu Leu Trp Leu Thr Glu
1               5                   10                  15

Lys Glu Gly Ser Tyr Pro Lys Leu Lys Asn Ser Tyr Val Asn Lys
            20                  25                  30


<210> 202
<211> 23
<212> PRT
<213> Influenza virus

<400> 202
His Glu Gly Lys Ser Ser Phe Tyr Arg Asn Leu Leu Trp Leu Thr Glu
1               5                   10                  15

Lys Glu Gly Ser Tyr Pro Lys
            20


<210> 203
<211> 26
<212> PRT
<213> Influenza virus

<400> 203
His Lys Cys Asp Asn Glu Cys Met Glu Ser Val Arg Asn Gly Thr Tyr
1               5                   10                  15

Asp Tyr Pro Lys Tyr Ser Glu Glu Ser Lys
            20                  25


<210> 204
<211> 12
<212> PRT
<213> Influenza virus

<400> 204
Lys Glu Ser Ser Trp Pro Asn His Thr Val Thr Lys
1               5                   10


<210> 205
<211> 35
<212> PRT
<213> Influenza virus

<400> 205
Lys Asn Leu Leu Trp Leu Thr Glu Lys Asn Gly Leu Tyr Pro Asn Leu
1               5                   10                  15

Ser Lys Ser Tyr Val Asn Asn Lys Glu Lys Glu Ile Leu Val Leu Trp
            20                  25                  30

Gly Val His
        35


<210> 206
<211> 27
<212> PRT
<213> Influenza virus

<220>
<221> MOD_RES
<222> (9)
<223> lys or met

<220>
<221> MOD_RES
<222> (10)
<223> asn or not present

<220>
<221> MOD_RES
<222> (16)
<223> glu or gly

<220>
<221> MOD_RES
<222> (17)
<223> not present or lys

<400> 206
His Asn Gly Lys Ser Ser Phe Tyr Xaa Xaa Leu Leu Trp Leu Thr Xaa
1               5                   10                  15

Xaa Lys Asn Gly Leu Tyr Pro Asn Leu Ser Lys
            20                  25


<210> 207
<211> 17
<212> PRT
<213> Influenza virus

<400> 207
His Asn Gly Lys Ser Ser Phe Tyr Lys Asn Leu Leu Trp Leu Thr Glu
1               5                   10                  15

Lys


<210> 208
<211> 55
<212> PRT
<213> Influenza virus

<400> 208
His Thr Val Thr Lys Gly Val Thr Ala Ser Cys Ser His Asn Gly Lys
1               5                   10                  15

Ser Ser Phe Tyr Lys Asn Leu Leu Trp Leu Thr Glu Lys Asn Gly Leu
            20                  25                  30

Tyr Pro Asn Leu Ser Lys Ser Tyr Val Asn Asn Lys Glu Lys Glu Val
        35                  40                  45

Leu Val Leu Trp Gly Val His
    50                  55


<210> 209
<211> 38
<212> PRT
<213> Influenza virus

<220>
<221> MOD_RES
<222> (5)
<223> lys or gly

<220>
<221> MOD_RES
<222> (8)
<223> thr or ser

<220>
<221> MOD_RES
<222> (21)

<223> lys or met

<220>
<221> MOD_RES
<222> (22)
<223> asn or not present

<220>
<221> MOD_RES
<222> (28)
<223> glu or gly

<220>
<221> MOD_RES
<222> (30)
<223> asn present or not or lys

<400> 209
His Thr Val Thr Xaa Gly Val Xaa Ala Ser Cys Ser His Asn Gly Lys
1               5                   10                  15

Ser Ser Phe Tyr Xaa Xaa Leu Leu Trp Leu Thr Xaa Lys Xaa Gly Leu
            20                  25                  30

Tyr Pro Asn Leu Ser Lys
        35


<210> 210
<211> 29
<212> PRT
<213> Influenza virus

<400> 210
His Thr Val Thr Lys Gly Val Thr Ala Ser Cys Ser His Asn Gly Lys
1               5                   10                  15

Ser Ser Phe Tyr Lys Asn Leu Leu Trp Leu Thr Glu Lys
            20                  25


<210> 211
<211> 48
<212> PRT
<213> Influenza virus

<400> 211
Lys Tyr Val Arg Ser Thr Lys Leu Arg Met Val Thr Gly Leu Arg Asn
1               5                   10                  15

Ile Pro Ser Ile Gln Ser Arg Gly Leu Phe Gly Ala Ile Ala Gly Phe
            20                  25                  30

Ile Glu Gly Gly Trp Thr Gly Met Ile Asp Gly Trp Tyr Gly Tyr His
        35                  40                  45

<210> 212
<211> 43
<212> PRT
<213> Influenza virus

<400> 212
His Gln Asn Glu Gln Gly Ser Gly Tyr Ala Ala Asp Gln Lys Ser Thr
1               5                   10                  15

Gln Asn Ala Ile Asn Gly Ile Thr Asn Lys Val Asn Ser Ile Ile Glu
            20                  25                  30

Lys Met Asn Thr Gln Phe Thr Ala Val Gly Lys
        35                  40


<210> 213
<211> 33
<212> PRT
<213> Influenza virus

<400> 213
His Gln Asn Glu Gln Gly Ser Gly Tyr Ala Ala Asp Gln Lys Ser Thr
1               5                   10                  15

Gln Asn Ala Ile Asn Gly Ile Thr Asn Lys Val Asn Ser Ile Ile Glu
            20                  25                  30

Lys


<210> 214
<211> 26
<212> PRT
<213> Influenza virus

<400> 214
His Gln Asn Glu Gln Gly Ser Gly Tyr Ala Ala Asp Gln Lys Ser Thr
1               5                   10                  15

Gln Asn Ala Ile Asn Gly Ile Thr Asn Lys
            20                  25


<210> 215
<211> 23
<212> PRT
<213> Influenza virus

<400> 215
His Ser Gly Ala Arg Ser Phe Tyr Arg Asn Leu Leu Trp Ile Val Lys
1               5                   10                  15

Lys Gly Asn Ser Tyr Pro Lys
            20

<210> 216
<211> 26
<212> PRT
<213> Influenza virus

<400> 216
His Ser Gly Ala Arg Ser Phe Tyr Arg Asn Leu Leu Trp Ile Val Lys
1               5                   10                  15

Lys Gly Asn Ser Tyr Pro Lys Leu Asn Lys
            20                  25


<210> 217
<211> 32
<212> PRT
<213> Influenza virus

<400> 217
His Ser Gly Ala Arg Ser Phe Tyr Arg Asn Leu Leu Trp Ile Val Lys
1               5                   10                  15

Lys Gly Asn Ser Tyr Pro Lys Leu Asn Lys Ser Tyr Thr Asn Asp Lys
            20                  25                  30


<210> 218
<211> 34
<212> PRT
<213> Influenza virus

<400> 218
His Ser Gly Ala Arg Ser Phe Tyr Arg Asn Leu Leu Trp Ile Val Lys
1               5                   10                  15

Lys Gly Asn Ser Tyr Pro Lys Leu Asn Lys Ser Tyr Thr Asn Asp Lys
            20                  25                  30

Gly Lys


<210> 219
<211> 16
<212> PRT
<213> Influenza virus

<400> 219
His Thr Val Ser Lys Gly Val Thr Thr Ser Cys Ser His Asn Gly Lys
1               5                   10                  15


<210> 220
<211> 12
<212> PRT
<213> Influenza virus

<400> 220
Lys Ala Thr Ser Trp Pro Asn His Glu Thr Thr Lys
1               5                   10

<210> 221
<211> 12
<212> PRT
<213> Influenza virus

<400> 221
Lys Gln Val Thr Thr Ser Cys Ser His Asn Gln Lys
1               5                   10

<210> 222
<211> 27
<212> PRT
<213> Influenza virus

<400> 222
Lys Gly Asn Ser Tyr Pro Lys Leu Asn Lys Ser Tyr Thr Asn Asp Lys
1               5                   10                  15

Gly Lys Glu Val Leu Val Ile Trp Gly Val His
            20                  25

<210> 223
<211> 21
<212> PRT
<213> Influenza virus

<400> 223
Lys Leu Asn Lys Ser Tyr Thr Asn Asp Lys Gly Lys Glu Val Leu Val
1               5                   10                  15

Ile Trp Gly Val His
            20

<210> 224
<211> 18
<212> PRT
<213> Influenza virus

<400> 224
Lys Ser Tyr Thr Asn Asp Lys Gly Lys Glu Val Leu Val Ile Trp Gly
1               5                   10                  15

Val His

<210> 225
<211> 35
<212> PRT
<213> Influenza virus

<220>
<221> MOD_RES
<222> (16)
<223> glu or gln

<220>
<221> MOD_RES
<222> (29)
<223> val or ala

<400> 225
His Asn Gln Lys Ser Ser Phe Tyr Arg Asn Leu Leu Trp Leu Thr Xaa
1               5                   10                  15

Lys Asn Gly Leu Tyr Pro Asn Leu Ser Lys Ser Tyr Xaa Ala Asn Asn
            20                  25                  30

Lys Glu Lys
        35


<210> 226
<211> 16
<212> PRT
<213> Influenza virus

<400> 226
His Pro Ile Thr Ile Gly Glu Cys Pro Lys Tyr Val Arg Ser Ala Lys
1               5                   10                  15


<210> 227
<211> 43
<212> PRT
<213> Influenza virus

<400> 227
His Gln Asn Glu Gln Gly Ser Gly Tyr Ala Ala Asp Gln Lys Ser Thr
1               5                   10                  15

Gln Asn Ala Ile Asn Gly Ile Thr Asn Lys Val Asn Ser Val Ile Glu
            20                  25                  30

Lys Met Asn Thr Gln Phe Thr Ala Val Gly Lys
        35                  40


<210> 228
<211> 33
<212> PRT
<213> Influenza virus

<400> 228
His Gln Asn Glu Gln Gly Ser Gly Tyr Ala Ala Asp Gln Lys Ser Thr
1               5                   10                  15

Gln Asn Ala Ile Asn Gly Ile Thr Asn Lys Val Asn Ser Val Ile Glu
            20                  25                  30

Lys

<210> 229
<211> 34
<212> PRT
<213> Influenza virus

<400> 229
His Asn Gly Lys Ser Ser Phe Tyr Arg Asn Leu Leu Trp Leu Thr Glu
1               5                   10                  15

Lys Asn Gly Leu Tyr Pro Asn Leu Ser Lys Ser Tyr Val Asn Asn Lys
            20                  25                  30

Glu Lys

<210> 230
<211> 11
<212> PRT
<213> Influenza virus

<400> 230
Lys His Phe Glu Lys Val Lys Ile Leu Pro Lys
1               5                   10

<210> 231
<211> 14
<212> PRT
<213> Influenza virus

<400> 231
Lys His Leu Leu Ser Ser Val Lys His Phe Glu Lys Val Lys
1               5                   10

<210> 232
<211> 13
<212> PRT
<213> Influenza virus

<220>
<221> MOD_RES
<222> (3)
<223> lys, gln or met

<220>
<221> MOD_RES
<222> (4)
<223> asp or asn

<400> 232
His Ala Xaa Xaa Ile Leu Glu Lys Thr His Asn Gly Lys
1               5                   10

<210> 233
<211> 16
<212> PRT
<213> Influenza virus

<220>
<221> MOD_RES
<222> (3)
<223> lys, gln or met

<220>
<221> MOD_RES
<222> (4)
<223> asp or asn

<220>
<221> MOD_RES
<222> (16)
<223> lys or arg

<400> 233
His Ala Xaa Xaa Ile Leu Glu Lys Thr His Asn Gly Lys Leu Cys Xaa
1               5                   10                  15


<210> 234
<211> 19
<212> PRT
<213> Influenza virus

<400> 234
His Asn Val His Pro Leu Thr Ile Gly Glu Cys Pro Lys Tyr Val Lys
1               5                   10                  15

Ser Glu Lys


<210> 235
<211> 16
<212> PRT
<213> Influenza virus

<400> 235
His Pro Leu Thr Ile Gly Glu Cys Pro Lys Tyr Val Lys Ser Glu Lys
1               5                   10                  15


<210> 236
<211> 18
<212> PRT
<213> Influenza virus

<400> 236
Lys His Leu Leu Ser Ser Val Lys His Phe Glu Lys Val Lys Ile Leu
1               5                   10                  15

Pro Lys

<210> 237
<211> 38
<212> PRT
<213> Influenza virus

<400> 237
Lys Arg Gln Ser Ser Gly Ile Met Lys Thr Glu Gly Thr Leu Glu Asn
1               5                   10                  15

Cys Glu Thr Lys Cys Gln Thr Pro Leu Gly Ala Ile Asn Thr Thr Leu
            20                  25                  30

Pro Phe His Asn Val His
        35


<210> 238
<211> 27
<212> PRT
<213> Influenza virus

<220>
<221> MOD_RES
<222> (7)
<223> val or ile

<220>
<221> MOD_RES
<222> (10)
<223> gly or arg

<220>
<221> MOD_RES
<222> (26)
<223> val or ile

<400> 238
Lys Gly Ser Asn Tyr Pro Xaa Ala Lys Xaa Ser Tyr Asn Asn Thr Ser
1               5                   10                  15

Gly Glu Gln Met Leu Ile Ile Trp Gln Xaa His
            20                  25


<210> 239
<211> 36
<212> PRT
<213> Influenza virus

<400> 239
His Thr Thr Leu Gly Gln Ser Arg Ala Cys Ala Val Ser Gly Asn Pro
1               5                   10                  15

Ser Phe Phe Arg Asn Met Val Trp Leu Thr Glu Lys Gly Ser Asn Tyr
            20                  25                  30

Pro Val Ala Lys
        35


<210> 240
<211> 7
<212> PRT
<213> Influenza virus

<400> 240
Lys His Phe Glu Lys Val Lys
1               5


<210> 241
<211> 38
<212> PRT
<213> Influenza virus

<400> 241
Lys Ile Ser Lys Arg Gly Ser Ser Gly Ile Met Lys Thr Glu Gly Thr
1               5                   10                  15

Leu Glu Asn Cys Glu Thr Lys Cys Gln Thr Pro Leu Gly Ala Ile Asn
            20                  25                  30

Thr Thr Leu Pro Phe His
        35


<210> 242
<211> 35
<212> PRT
<213> Influenza virus

<400> 242
Lys Arg Gly Ser Ser Gly Ile Met Lys Thr Glu Gly Thr Leu Glu Asn
1               5                   10                  15

Cys Glu Thr Lys Cys Gln Thr Pro Leu Gly Ala Ile Asn Thr Thr Leu
            20                  25                  30

Pro Phe His
        35


<210> 243
<211> 27
<212> PRT
<213> Influenza virus

<400> 243
Lys Thr Glu Gly Thr Leu Glu Asn Cys Glu Thr Lys Cys Gln Thr Pro
1               5                   10                  15

Leu Gly Ala Ile Asn Thr Thr Leu Pro Phe His
            20                  25

<210> 244
<211> 38
<212> PRT
<213> Influenza virus

<400> 244
Lys Ile Ser Lys Arg Gly Ser Ser Gly Ile Met Lys Thr Glu Gly Thr
1               5                   10                  15

Leu Glu Asn Cys Glu Thr Lys Cys Gln Thr Pro Leu Gly Ala Ile Asn
            20                  25                  30

Thr Thr Leu Pro Phe His
        35


<210> 245
<211> 30
<212> PRT
<213> Influenza virus

<220>
<221> MOD_RES
<222> (29)
<223> val or ile

<400> 245
Lys Thr Glu Gly Thr Leu Glu Asn Cys Glu Thr Lys Cys Gln Thr Pro
1               5                   10                  15

Leu Gly Ala Ile Asn Thr Thr Leu Pro Phe His Asn Xaa His
            20                  25                  30


<210> 246
<211> 38
<212> PRT
<213> Influenza virus

<400> 246
Lys Ile Ser Lys Arg Gly Ser Ser Gly Ile Met Lys Thr Glu Gly Thr
1               5                   10                  15

Leu Glu Asn Cys Glu Thr Lys Cys Gln Thr Pro Leu Gly Ala Ile Asn
            20                  25                  30

Thr Thr Leu Pro Phe His
        35


<210> 247
<211> 27
<212> PRT
<213> Influenza virus

<220>
<221> MOD_RES

<222> (2)
<223> glu or gly

<400> 247
Lys Xaa Ser Asn Tyr Pro Val Ala Lys Gly Ser Tyr Asn Asn Thr Ser
1               5                   10                  15

Gly Glu Gln Met Leu Ile Ile Trp Gly Val His
            20                  25


<210> 248
<211> 16
<212> PRT
<213> Influenza virus

<400> 248
His Pro Leu Thr Ile Gly Glu Cys Pro Lys Tyr Val Lys Ser Glu Lys
1               5                   10                  15


<210> 249
<211> 16
<212> PRT
<213> Influenza virus

<400> 249
Lys Cys Gln Thr Pro Leu Gly Ala Ile Lys Thr Thr Leu Pro Phe His
1               5                   10                  15


<210> 250
<211> 58
<212> PRT
<213> Influenza virus

<220>
<221> MOD_RES
<222> (21)
<223> phe or ile

<220>
<221> MOD_RES
<222> (47)
<223> asn or ser

<400> 250
His His Ser Asn Asp Gln Gly Ser Gly Tyr Ala Ala Asp Lys Glu Ser
1               5                   10                  15

Thr Gln Lys Ala Xaa Asp Gly Ile Thr Asn Lys Val Asn Ser Val Ile
            20                  25                  30

Glu Lys Met Asn Thr Gln Phe Glu Ala Val Gly Lys Leu Phe Xaa Asn
        35                  40                  45

Leu Glu Lys Leu Glu Asn Leu Asn Lys Lys
    50                  55

<210> 251
<211> 57
<212> PRT
<213> Influenza virus

<220>
<221> MOD_RES
<222> (20)
<223> phe or ile

<220>
<221> MOD_RES
<222> (46)
<223> asn or ser

<400> 251
His Ser Asn Asp Gln Gly Ser Gly Tyr Ala Ala Asp Lys Glu Ser Thr
1               5                   10                  15

Gln Lys Ala Xaa Asp Gly Ile Thr Asn Lys Val Asn Ser Val Ile Glu
            20                  25                  30

Lys Met Asn Thr Gln Phe Glu Ala Val Gly Lys Leu Phe Xaa Asn Leu
        35                  40                  45

Glu Lys Leu Glu Asn Leu Asn Lys Lys
    50                  55


<210> 252
<211> 26
<212> PRT
<213> Influenza virus

<220>
<221> MOD_RES
<222> (20)
<223> phe or ile

<400> 252
His Ser Asn Asp Gln Gly Ser Gly Tyr Ala Ala Asp Lys Glu Ser Thr
1               5                   10                  15

Gln Lys Ala Xaa Asp Gly Ile Thr Asn Lys
            20                  25


<210> 253
<211> 21
<212> PRT
<213> Influenza virus

<400> 253
His Asp Ser Asn Val Arg Asn Leu Tyr Asp Lys Val Arg Met Gln Leu
1               5                   10                  15

Arg Asp Asn Ala Lys
            20


<210> 254
<211> 30
<212> PRT
<213> Influenza virus

<400> 254
His Lys Cys Asp Asp Glu Cys Met Asn Ser Val Lys Asn Gly Thr Tyr
1               5                   10                  15

Asp Tyr Pro Lys Leu Asn Arg Asn Glu Ile Lys Gly Val Lys
            20                  25                  30


<210> 255
<211> 27
<212> PRT
<213> Influenza virus

<400> 255
His Lys Cys Asp Asp Glu Cys Met Asn Ser Val Lys Asn Gly Thr Tyr
1               5                   10                  15

Asp Tyr Pro Lys Leu Asn Arg Asn Glu Ile Lys
            20                  25


<210> 256
<211> 20
<212> PRT
<213> Influenza virus

<400> 256
His Lys Cys Asp Asp Glu Cys Met Asn Ser Val Lys Asn Gly Thr Tyr
1               5                   10                  15

Asp Tyr Pro Lys
            20


<210> 257
<211> 12
<212> PRT
<213> Influenza virus

<400> 257
His Lys Cys Asp Asp Glu Cys Met Asn Ser Val Lys
1               5                   10


<210> 258
<211> 27
<212> PRT
<213> Influenza virus

<400> 258
Lys Gly Ser Asn Tyr Pro Val Ala Lys Gly Ser Tyr Asn Asn Thr Asn
1               5                   10                  15

Gly Glu Gln Ile Leu Ile Ile Trp Gly Val His
            20                  25


<210> 259
<211> 43
<212> PRT
<213> Influenza virus

<400> 259
His Ser Asn Asp Gln Gly Ser Gly Tyr Ala Ala Asp Lys Glu Ser Thr
1               5                   10                  15

Gln Lys Ala Val Asp Gly Ile Thr Asn Lys Val Asn Ser Val Ile Glu
            20                  25                  30

Lys Met Asn Thr Gln Phe Glu Ala Val Gly Lys
        35                  40


<210> 260
<211> 35
<212> PRT
<213> Influenza virus

<400> 260
Lys Arg Gly Ser Ser Gly Ile Met Lys Thr Glu Gly Thr Leu Glu Asn
1               5                   10                  15

Cys Glu Thr Lys Cys Gln Thr Pro Leu Gly Ala Ile Asn Thr Thr Leu
            20                  25                  30

Pro Phe His
        35


<210> 261
<211> 16
<212> PRT
<213> Influenza virus

<400> 261
His Pro Leu Thr Ile Gly Glu Cys Pro Lys Tyr Val Lys Ser Glu Lys
1               5                   10                  15


<210> 262
<211> 16
<212> PRT
<213> Influenza virus

<400> 262
His Ala Lys Asp Ile Leu Glu Lys Thr His Asn Gly Lys Leu Cys Lys
1               5                   10                  15

<210> 263
<211> 25
<212> PRT
<213> Influenza virus

<400> 263
His Asp Val Tyr Arg Asp Glu Ala Leu Asn Asn Arg Phe Gln Ile Lys
1               5                   10                  15

Gly Val Glu Leu Lys Ser Gly Tyr Lys
            20                  25


<210> 264
<211> 19
<212> PRT
<213> Influenza virus

<400> 264
His Thr Ile Asp Leu Thr Asp Ser Glu Met Asn Lys Leu Phe Glu Arg
1               5                   10                  15

Thr Arg Lys


<210> 265
<211> 7
<212> PRT
<213> Influenza virus

<400> 265
Lys Phe His Gln Ile Glu Lys
1               5


<210> 266
<211> 11
<212> PRT
<213> Influenza virus

<220>
<221> MOD_RES
<222> (8)
<223> gly or gln

<400> 266
Lys Thr Asn Glu Lys Phe His Xaa Ile Glu Lys
1               5                   10


<210> 267
<211> 14
<212> PRT
<213> Influenza virus

<220>

<221> MOD_RES
<222> (5)
<223> val or leu

<400> 267
Lys Leu Asn Arg Xaa Ile Glu Lys Thr Asn Glu Lys Phe His
1               5                   10


<210> 268
<211> 25
<212> PRT
<213> Influenza virus

<400> 268
His Gln Ile Glu Lys Glu Phe Ser Glu Val Glu Gly Arg Ile Gln Asp
1               5                   10                  15

Leu Glu Lys Tyr Val Glu Asp Thr Lys
            20                  25


<210> 269
<211> 8
<212> PRT
<213> Influenza virus

<400> 269
Lys Ile Cys Asn Asn Pro His Lys
1               5


<210> 270
<211> 14
<212> PRT
<213> Influenza virus

<400> 270
Lys Leu Asn Arg Val Ile Lys Lys Thr Asn Glu Lys Phe His
1               5                   10


<210> 271
<211> 24
<212> PRT
<213> Influenza virus

<220>
<221> MOD_RES
<222> (3)
<223> ile or val

<220>
<221> MOD_RES
<222> (17)
<223> gly or gln

<220>

<221> MOD_RES
<222> (20)
<223> arg or lys

<220>
<221> MOD_RES
<222> (22)
<223> gln or gly

<400> 271
His Asp Xaa Tyr Arg Asp Glu Ala Leu Asn Asn Arg Phe Gln Ile Lys
1               5                   10                  15

Xaa Val Glu Xaa Ser Xaa Tyr Lys
            20


<210> 272
<211> 25
<212> PRT
<213> Influenza virus

<400> 272
His Gln Ile Glu Lys Glu Phe Ser Glu Val Glu Gly Arg Ile Gln Asp
1               5                   10                  15

Leu Glu Lys Tyr Val Glu Asp Thr Lys
            20                  25


<210> 273
<211> 25
<212> PRT
<213> Influenza virus

<400> 273
Lys Tyr Val Glu Asp Thr Lys Ile Asp Leu Trp Ser Tyr Asn Ala Glu
1               5                   10                  15

Leu Leu Val Ala Leu Glu Asn Gln His
            20                  25


<210> 274
<211> 49
<212> PRT
<213> Influenza virus

<400> 274
Lys Tyr Val Lys Gln Asn Ser Leu Lys Leu Ala Thr Gly Met Arg Asn
1               5                   10                  15

Val Pro Glu Lys Gln Thr Arg Gly Leu Phe Gly Ala Ile Ala Gly Phe
            20                  25                  30

Ile Glu Asn Gly Trp Glu Gly Met Ile Asp Gly Trp Tyr Gly Phe Arg
        35                  40                  45

His

<210> 275
<211> 39
<212> PRT
<213> Influenza virus

<400> 275
Lys Glu Phe Ser Glu Val Glu Gly Arg Ile Gln Asp Leu Glu Lys Tyr
1               5                   10                  15

Val Glu Asp Thr Lys Ile Asp Leu Trp Ser Tyr Asn Ala Glu Leu Leu
            20                  25                  30

Val Ala Leu Glu Asn Gln His
        35

<210> 276
<211> 33
<212> PRT
<213> Influenza virus

<220>
<221> MOD_RES
<222> (4)
<223> ser or glu

<220>
<221> MOD_RES
<222> (5)
<223> glu or gln

<220>
<221> MOD_RES
<222> (7)
<223> thr or ser

<220>
<221> MOD_RES
<222> (9)
<223> gln or tyr

<220>
<221> MOD_RES
<222> (13)
<223> leu or gln

<220>
<221> MOD_RES
<222> (18)
<223> ala or asn

<220>
<221> MOD_RES
<222> (20)
<223> ile or leu

<220>
<221> MOD_RES
<222> (22)
<223> gln or gly

<220>
<221> MOD_RES
<222> (24)
<223> asn or thr

<220>
<221> MOD_RES
<222> (25)
<223> gly or asn

<220>
<221> MOD_RES
<222> (27)
<223> leu or val

<220>
<221> MOD_RES
<222> (29)
<223> arg or ser

<220>
<221> MOD_RES
<222> (32)
<223> glu or cys

<400> 276
His Gln Asn Xaa Xaa Gly Xaa Gly Xaa Ala Ala Asp Xaa Lys Ser Thr
  1               5                  10                  15

Gln Xaa Ala Xaa Asp Xaa Ile Xaa Xaa Lys Xaa Asn Xaa Val Ile Xaa
             20                  25                  30

Lys


<210> 277
<211> 18
<212> PRT
<213> Influenza virus

<220>
<221> MOD_RES
<222> (4)
<223> gly or gln

<220>
<221> MOD_RES
<222> (6)
<223> gln or arg

<220>
<221> MOD_RES

<222> (14)
<223> val or ile

<220>
<221> MOD_RES
<222> (17)
<223> ser or thr

<400> 277
His Cys Asp Xaa Phe Xaa Asn Glu Lys Trp Asp Leu Phe Xaa Glu Arg
1               5                   10                  15

Xaa Lys


<210> 278
<211> 20
<212> PRT
<213> Influenza virus

<400> 278
His Thr Ile Asp Leu Thr Asp Ser Glu Met Asn Lys Lys Leu Phe Glu
1               5                   10                  15

Arg Thr Arg Lys
            20


<210> 279
<211> 28
<212> PRT
<213> Influenza virus

<400> 279
Lys Ser Gly Ser Thr Tyr Pro Val Leu Lys Val Thr Met Pro Asn Asn
1               5                   10                  15

Asp Asn Phe Asp Lys Leu Tyr Ile Trp Gly Val His
            20                  25


<210> 280
<211> 34
<212> PRT
<213> Influenza virus

<400> 280
Lys Leu Asn Trp Leu Thr Lys Ser Gly Asn Thr Tyr Pro Val Leu Asn
1               5                   10                  15

Val Thr Met Pro Asn Asn Asp Asn Phe Asp Lys Leu Val Ile Trp Gly
            20                  25                  30

Val His


<210> 281
<211> 19

<212> PRT
<213> Influenza virus

<400> 281
His Thr Ile Asp Leu Thr Asp Ser Glu Met Asn Lys Leu Phe Glu Lys
1               5                   10                  15

Thr Arg Lys


<210> 282
<211> 18
<212> PRT
<213> Influenza virus

<400> 282
Lys Leu Asn Arg Leu Ile Glu Lys Thr Asn Glu Lys Phe His Gln Thr
1               5                   10                  15

Glu Lys


<210> 283
<211> 47
<212> PRT
<213> Influenza virus

<400> 283
His Thr Gly Lys Ser Ser Val Met Arg Ser Asp Ala Pro Ile Asp Phe
1               5                   10                  15

Cys Asn Ser Glu Cys Ile Thr Pro Asn Gln Ser Ile Pro Asn Asp Lys
            20                  25                  30

Pro Phe Gln Asn Val Asn Lys Ile Thr Tyr Gly Ala Cys Pro Lys
        35                  40                  45


<210> 284
<211> 39
<212> PRT
<213> Influenza virus

<400> 284
His Thr Gly Lys Ser Ser Val Met Arg Ser Asp Ala Pro Ile Asp Phe
1               5                   10                  15

Cys Asn Ser Glu Cys Ile Thr Pro Asn Gln Ser Ile Pro Asn Asp Lys
            20                  25                  30

Pro Phe Gln Asn Val Asn Lys
        35


<210> 285
<211> 33
<212> PRT
<213> Influenza virus

<400> 285
His Pro Ser Thr Asp Ser Asp Gln Thr Ser Leu Tyr Val Arg Ala Ser
1               5                   10                  15

Gly Arg Val Thr Val Ser Thr Lys Arg Ser Gln Gln Thr Val Ile Pro
            20                  25                  30

Lys


<210> 286
<211> 25
<212> PRT
<213> Influenza virus

<400> 286
Lys Tyr Val Glu Asp Thr Lys Ile Asp Leu Trp Ser Tyr Asn Ala Glu
1               5                   10                  15

Leu Leu Val Ala Leu Glu Asn Gln His
            20                  25


<210> 287
<211> 26
<212> PRT
<213> Influenza virus

<400> 287
Lys Leu Phe Glu Arg Thr Arg Lys Gln Leu Arg Glu Asn Ala Glu Asp
1               5                   10                  15

Met Gly Asn Gly Cys Phe Lys Ile Tyr His
            20                  25


<210> 288
<211> 16
<212> PRT
<213> Influenza virus

<400> 288
Lys Arg Arg Ser Ile Lys Ser Phe Phe Ser Arg Leu Asn Trp Leu His
1               5                   10                  15


<210> 289
<211> 16
<212> PRT
<213> Influenza virus

<220>
<221> MOD_RES
<222> (12)
<223> val or arg

<400> 289
His Pro Val Thr Ile Gly Glu Cys Pro Lys Tyr Xaa Lys Ser Thr Lys
1               5                   10                  15

<210> 290
<211> 30
<212> PRT
<213> Influenza virus

<400> 290
Lys Gly Asn Ser Tyr Pro Lys Leu Ser Lys Leu Ser Lys Ser Tyr Ile
1               5                   10                  15

Ile Asn Lys Lys Lys Glu Val Leu Val Ile Trp Gly Ile His
            20                  25                  30

<210> 291
<211> 24
<212> PRT
<213> Influenza virus

<220>
<221> MOD_RES
<222> (9)
<223> val or tyr

<400> 291
Lys Leu Ser Lys Leu Ser Lys Ser Xaa Ile Ile Asn Lys Lys Lys Glu
1               5                   10                  15

Val Leu Val Ile Trp Gly Ile His
            20

<210> 292
<211> 21
<212> PRT
<213> Influenza virus

<220>
<221> MOD_RES
<222> (6)
<223> val or tyr

<400> 292
Lys Leu Ser Lys Ser Xaa Ile Ile Asn Lys Lys Lys Glu Val Leu Val
1               5                   10                  15

Ile Trp Gly Ile His
            20

<210> 293
<211> 46
<212> PRT
<213> Plasmodium falciparum

<400> 293
Lys Glu Glu Glu Glu Lys Glu Lys Glu Lys Glu Lys Glu Lys Glu Glu
1               5                   10                  15

Lys Glu Lys Glu Glu Lys Glu Lys Glu Glu Lys Glu Lys Glu Lys Glu
            20                  25                  30

Glu Lys Glu Lys Glu Lys Glu Glu Lys Glu Glu Glu Lys Lys
        35                  40                  45


<210> 294
<211> 48
<212> PRT
<213> Plasmodium falciparum

<400> 294
Lys Glu Glu Glu Glu Lys Glu Lys Glu Lys Glu Lys Glu Lys Glu Glu
1               5                   10                  15

Lys Glu Lys Glu Glu Lys Glu Lys Glu Glu Lys Glu Lys Glu Lys Glu
            20                  25                  30

Glu Lys Glu Lys Glu Lys Glu Glu Lys Glu Glu Glu Lys Lys Glu Lys
        35                  40                  45


<210> 295
<211> 47
<212> PRT
<213> Plasmodium falciparum

<400> 295
Lys Glu Glu Glu Glu Lys Glu Lys Glu Lys Glu Lys Glu Lys Glu Glu
1               5                   10                  15

Lys Glu Lys Glu Glu Lys Glu Lys Glu Lys Glu Glu Lys Glu Lys Glu
            20                  25                  30

Glu Lys Glu Lys Glu Glu Lys Glu Glu Lys Glu Glu Glu Lys Lys
        35                  40                  45


<210> 296
<211> 10
<212> PRT
<213> Plasmodium falciparum

<400> 296
Lys Glu Glu Glu Glu Lys Glu Lys Glu Lys
1               5                   10


<210> 297
<211> 19
<212> PRT
<213> Plasmodium falciparum

<400> 297
His Lys Lys Leu Ile Lys Ala Leu Lys Lys Asn Ile Glu Ser Ile Gln
1               5                   10                  15

Asn Lys Lys


<210> 298
<211> 19
<212> PRT
<213> Plasmodium falciparum

<400> 298
His Lys Lys Leu Ile Lys Ala Leu Lys Lys Asn Ile Glu Ser Ile Gln
1               5                   10                  15

Asn Lys Met


<210> 299
<211> 10
<212> PRT
<213> Plasmodium falciparum

<400> 299
His Lys Lys Leu Ile Lys Ala Leu Lys Lys
1               5                   10


<210> 300
<211> 9
<212> PRT
<213> Plasmodium falciparum

<400> 300
His Lys Lys Leu Ile Lys Ala Leu Lys
1               5


<210> 301
<211> 23
<212> PRT
<213> Plasmodium falciparum

<400> 301
Lys Ala Thr Tyr Ser Phe Val Asn Thr Lys Lys Lys Ile Ile Ser Leu
1               5                   10                  15

Lys Ser Gln Gly His Lys Lys
            20


<210> 302
<211> 22
<212> PRT
<213> Plasmodium falciparum

<400> 302
Lys Ala Thr Tyr Ser Phe Val Asn Thr Lys Lys Lys Ile Ile Ser Leu
1               5                   10                  15

Lys Ser Gln Gly His Lys
            20


<210> 303
<211> 21
<212> PRT
<213> Plasmodium falciparum

<400> 303
Lys Ala Thr Tyr Ser Phe Val Asn Thr Lys Lys Lys Ile Ile Ser Leu
1               5                   10                  15

Lys Ser Gln Gly His
            20


<210> 304
<211> 29
<212> PRT
<213> Plasmodium falciparum

<400> 304
His Thr Tyr Val Lys Gly Lys Lys Ala Pro Ser Asp Pro Gln Cys Ala
1               5                   10                  15

Asp Ile Lys Glu Glu Cys Lys Glu Leu Leu Lys Glu Lys
            20                  25


<210> 305
<211> 11
<212> PRT
<213> Plasmodium falciparum

<400> 305
Lys Ile Ile Ser Leu Lys Ser Gln Gly His Lys
1               5                   10


<210> 306
<211> 21
<212> PRT
<213> Plasmodium falciparum

<400> 306
Lys Lys Lys Lys Phe Glu Pro Leu Lys Asn Gly Asn Val Ser Glu Thr
1               5                   10                  15

Ile Lys Leu Ile His
            20


<210> 307

<211> 20
<212> PRT
<213> Plasmodium falciparum

<400> 307
Lys Lys Lys Phe Glu Pro Leu Lys Asn Gly Asn Val Ser Glu Thr Ile
1               5                   10                  15

Lys Leu Ile His
            20


<210> 308
<211> 19
<212> PRT
<213> Plasmodium falciparum

<400> 308
Lys Lys Phe Glu Pro Leu Lys Asn Gly Asn Val Ser Glu Thr Ile Lys
1               5                   10                  15

Leu Ile His


<210> 309
<211> 13
<212> PRT
<213> Plasmodium falciparum

<400> 309
Lys Asn Gly Asn Val Ser Glu Thr Ile Lys Leu Ile His
1               5                   10


<210> 310
<211> 11
<212> PRT
<213> Plasmodium falciparum

<400> 310
Lys Leu Ile His Leu Gly Asn Lys Asp Lys Lys
1               5                   10


<210> 311
<211> 36
<212> PRT
<213> Plasmodium falciparum

<400> 311
Lys Val Lys Lys Ile Gly Val Thr Leu Lys Lys Phe Glu Pro Leu Lys
1               5                   10                  15

Asn Gly Asn Val Ser Glu Thr Ile Lys Leu Ile His Leu Gly Asn Lys
            20                  25                  30

Asp Lys Lys His
        35

<210> 312
<211> 59
<212> PRT
<213> Plasmodium falciparum

<400> 312
His Leu Ile Tyr Lys Asn Lys Ser Tyr Asn Pro Leu Leu Leu Ser Cys
1               5                   10                  15

Val Lys Lys Met Asn Met Leu Lys Glu Asn Val Asp Tyr Ile Gln Asn
            20                  25                  30

Gln Asn Leu Phe Lys Glu Leu Met Asn Gln Lys Ala Thr Tyr Ser Phe
        35                  40                  45

Val Asn Thr Lys Lys Lys Ile Ile Ser Leu Lys
    50                  55


<210> 313
<211> 52
<212> PRT
<213> Plasmodium falciparum

<400> 313
His Leu Ile Tyr Lys Asn Lys Ser Tyr Asn Pro Leu Leu Leu Ser Cys
1               5                   10                  15

Val Lys Lys Met Asn Met Leu Lys Glu Asn Val Asp Tyr Ile Gln Asn
            20                  25                  30

Gln Asn Leu Phe Lys Glu Leu Met Asn Gln Lys Ala Thr Tyr Ser Phe
        35                  40                  45

Val Asn Thr Lys
    50


<210> 314
<211> 43
<212> PRT
<213> Plasmodium falciparum

<400> 314
His Leu Ile Tyr Lys Asn Lys Ser Tyr Asn Pro Leu Leu Leu Ser Cys
1               5                   10                  15

Val Lys Lys Met Asn Met Leu Lys Glu Asn Val Asp Tyr Ile Gln Asn
            20                  25                  30

Gln Asn Leu Phe Lys Glu Leu Met Asn Gln Lys
        35                  40


<210> 315
<211> 38

<212> PRT
<213> Plasmodium falciparum

<400> 315
His Leu Ile Tyr Lys Asn Lys Ser Tyr Asn Pro Leu Leu Leu Ser Cys
1               5                   10                  15

Val Lys Lys Met Asn Met Leu Lys Glu Asn Val Asp Tyr Ile Gln Lys
            20                  25                  30

Asn Gln Asn Leu Phe Lys
        35


<210> 316
<211> 24
<212> PRT
<213> Plasmodium falciparum

<400> 316
His Leu Ile Tyr Lys Asn Lys Ser Tyr Asn Pro Leu Leu Leu Ser Cys
1               5                   10                  15

Val Lys Lys Met Asn Met Leu Lys
            20


<210> 317
<211> 47
<212> PRT
<213> Plasmodium falciparum

<400> 317
Lys Ser Ala Asn Asn Ser Ala Asn Asn Gly Lys Lys Asn Asn Ala Glu
1               5                   10                  15

Glu Met Lys Asn Leu Val Asn Phe Leu Gln Ser His Lys Lys Leu Ile
            20                  25                  30

Lys Ala Leu Lys Lys Asn Ile Glu Ser Ile Gln Asn Lys Lys His
        35                  40                  45


<210> 318
<211> 37
<212> PRT
<213> Plasmodium falciparum

<400> 318
Lys Lys Asn Asn Ala Glu Glu Met Lys Asn Leu Val Asn Phe Leu Gln
1               5                   10                  15

Ser His Lys Lys Leu Ile Lys Ala Leu Lys Lys Asn Ile Glu Ser Ile
            20                  25                  30

Gln Asn Lys Lys His
        35

<210> 319
<211> 29
<212> PRT
<213> Plasmodium falciparum

<400> 319
Lys Asn Leu Val Asn Phe Leu Gln Ser His Lys Lys Leu Ile Lys Ala
1               5                   10                  15

Leu Lys Lys Asn Ile Glu Ser Ile Gln Asn Lys Lys His
            20                  25


<210> 320
<211> 19
<212> PRT
<213> Plasmodium falciparum

<400> 320
Lys Lys Leu Ile Lys Ala Leu Lys Lys Asn Ile Glu Ser Ile Gln Asn
1               5                   10                  15

Lys Lys His


<210> 321
<211> 18
<212> PRT
<213> Plasmodium falciparum

<400> 321
Lys Leu Ile Lys Ala Leu Lys Lys Asn Ile Glu Ser Ile Gln Asn Lys
1               5                   10                  15

Lys His


<210> 322
<211> 12
<212> PRT
<213> Plasmodium falciparum

<400> 322
Lys Lys Asn Ile Glu Ser Ile Gln Asn Lys Lys His
1               5                   10


<210> 323
<211> 11
<212> PRT
<213> Plasmodium falciparum

<400> 323
Lys Asn Ile Glu Ser Ile Gln Asn Lys Lys His
1               5                   10

<210> 324
<211> 17
<212> PRT
<213> Plasmodium falciparum

<400> 324
Lys Asn Asn Ala Glu Glu Met Lys Asn Leu Val Asn Phe Leu Gln Ser
1               5                   10                  15

His


<210> 325
<211> 23
<212> PRT
<213> Plasmodium falciparum

<400> 325
Lys Lys Leu Ile Lys Ala Leu Lys Lys Asn Ile Glu Ser Ile Gln Asn
1               5                   10                  15

Lys Lys Gln Gly His Lys Lys
            20


<210> 326
<211> 19
<212> PRT
<213> Plasmodium falciparum

<400> 326
Lys Lys Asn Asn Ala Glu Glu Met Lys Asn Leu Val Asn Phe Leu Gln
1               5                   10                  15

Ser His Lys


<210> 327
<211> 17
<212> PRT
<213> Plasmodium falciparum

<400> 327
Lys Asn Asn Ala Glu Glu Met Lys Asn Leu Val Asn Phe Leu Gln Ser
1               5                   10                  15

His


<210> 328
<211> 22
<212> PRT
<213> Plasmodium falciparum

<400> 328
Lys Leu Ile Lys Ala Leu Lys Lys Asn Ile Glu Ser Ile Gln Asn Lys
1               5                   10                  15

Lys Gln Gly His Lys Lys
            20

<210> 329
<211> 28
<212> PRT
<213> Plasmodium falciparum

<400> 329
Lys Val Lys Lys Ile Gly Val Thr Leu Lys Lys Phe Glu Pro Leu Lys
1               5                   10                  15

Asn Gly Asn Val Ser Glu Thr Ile Lys Leu Ile His
            20                  25

<210> 330
<211> 13
<212> PRT
<213> Plasmodium falciparum

<400> 330
Lys Asn Gly Asn Val Ser Glu Thr Ile Lys Leu Ile His
1               5                   10

<210> 331
<211> 11
<212> PRT
<213> Plasmodium falciparum

<400> 331
Lys Leu Ile His Leu Gly Asn Lys Asp Lys Lys
1               5                   10

<210> 332
<211> 28
<212> PRT
<213> Plasmodium falciparum

<400> 332
Lys Ser Ala Asn Asn Ser Ala Asn Asn Gly Lys Lys Asn Asn Ala Glu
1               5                   10                  15

Glu Met Lys Asn Leu Val Asn Phe Leu Gln Ser His
            20                  25

<210> 333
<211> 18
<212> PRT
<213> Plasmodium falciparum

<400> 333
Lys Lys Asn Asn Ala Glu Glu Met Lys Asn Leu Val Asn Phe Leu Gln
1               5                   10                  15

Ser His

<210> 334
<211> 19
<212> PRT
<213> Plasmodium falciparum

<400> 334
Lys Lys Leu Ile Lys Ala Leu Lys Lys Asn Ile Glu Ser Ile Gln Asn
1               5                   10                  15

Lys Lys His

<210> 335
<211> 15
<212> PRT
<213> Plasmodium falciparum

<400> 335
Lys Ala Leu Lys Lys Asn Ile Glu Ser Ile Gln Asn Lys Lys His
1               5                   10                  15

<210> 336
<211> 12
<212> PRT
<213> Plasmodium falciparum

<400> 336
Lys Lys Asn Ile Glu Ser Ile Gln Asn Lys Lys His
1               5                   10

<210> 337
<211> 27
<212> PRT
<213> Plasmodium falciparum

<400> 337
Lys Glu Leu Met Asn Gln Lys Ala Thr Tyr Ser Phe Val Asn Thr Lys
1               5                   10                  15

Lys Lys Ile Ile Ser Leu Lys Ser Gln Gly His
            20                  25

<210> 338
<211> 7
<212> PRT
<213> Plasmodium falciparum

<400> 338
Lys Ser Gln Gly His Lys Lys
1               5

<210> 339
<211> 12
<212> PRT
<213> Plasmodium falciparum

<400> 339
Lys Lys Lys Ile Ile Ser Leu Lys Ser Gln Gly His
1               5                   10


<210> 340
<211> 11
<212> PRT
<213> Plasmodium falciparum

<400> 340
Lys Lys Ile Ile Ser Leu Lys Ser Gln Gly His
1               5                   10


<210> 341
<211> 12
<212> PRT
<213> Plasmodium falciparum

<400> 341
Lys Lys Asn Ile Glu Ser Ile Gln Asn Lys Lys His
1               5                   10


<210> 342
<211> 11
<212> PRT
<213> Plasmodium falciparum

<400> 342
Lys Asn Ile Glu Ser Ile Gln Asn Lys Lys His
1               5                   10


<210> 343
<211> 29
<212> PRT
<213> Plasmodium falciparum

<400> 343
His Thr Tyr Val Lys Gly Lys Lys Ala Pro Ser Asp Pro Gln Cys Ala
1               5                   10                  15

Asp Ile Lys Glu Glu Cys Lys Glu Leu Leu Lys Glu Lys
            20                  25


<210> 344
<211> 27
<212> PRT
<213> Plasmodium falciparum

<400> 344
His Thr Tyr Val Lys Gly Lys Lys Ala Pro Ser Asp Pro Gln Cys Ala
1               5                   10                  15

Asp Ile Lys Glu Glu Cys Lys Glu Leu Leu Lys
            20                  25

<210> 345
<211> 29
<212> PRT
<213> Plasmodium falciparum

<400> 345
His Glu Asn Val Leu Ser Ala Ala Leu Glu Asn Thr Gln Ser Glu Glu
1               5                   10                  15

Glu Lys Lys Glu Val Ile Asp Val Ile Glu Glu Val Lys
            20                  25

<210> 346
<211> 48
<212> PRT
<213> Plasmodium falciparum

<400> 346
Lys Glu Asn Val Val Thr Thr Ile Leu Glu Lys Val Glu Glu Thr Thr
1               5                   10                  15

Ala Glu Ser Val Thr Thr Phe Ser Asn Ile Leu Glu Glu Ile Gln Glu
            20                  25                  30

Asn Thr Ile Thr Asn Asp Thr Ile Glu Glu Lys Leu Glu Glu Leu His
        35                  40                  45

<210> 347
<211> 14
<212> PRT
<213> Plasmodium falciparum

<400> 347
His Tyr Leu Gln Gln Met Lys Glu Lys Phe Ser Lys Glu Lys
1               5                   10

<210> 348
<211> 42
<212> PRT
<213> Plasmodium falciparum

<400> 348
His Tyr Leu Gln Gln Met Lys Glu Lys Phe Ser Lys Glu Lys Asn Asn
1               5                   10                  15

Asn Val Ile Glu Val Thr Asn Lys Ala Glu Lys Lys Gly Asn Val Gln
            20                  25                  30

Val Thr Asn Lys Thr Glu Lys Thr Thr Lys
        35                  40


<210> 349
<211> 48
<212> PRT
<213> Plasmodium falciparum

<400> 349
His Tyr Leu Gln Gln Met Lys Glu Lys Phe Ser Lys Glu Lys Asn Asn
 1               5                  10                  15

Asn Val Ile Glu Val Thr Asn Lys Ala Glu Lys Lys Gly Asn Val Gln
            20                  25                  30

Val Thr Asn Lys Thr Glu Lys Thr Thr Lys Val Asp Lys Asn Asn Lys
        35                  40                  45


<210> 350
<211> 57
<212> PRT
<213> Plasmodium falciparum

<400> 350
His Tyr Leu Gln Gln Met Lys Glu Lys Phe Ser Lys Glu Lys Asn Asn
 1               5                  10                  15

Asn Val Ile Glu Val Thr Asn Lys Ala Glu Lys Lys Gly Asn Val Gln
            20                  25                  30

Val Thr Asn Lys Thr Glu Lys Thr Thr Lys Val Asp Lys Asn Asn Lys
        35                  40                  45

Val Pro Lys Lys Arg Arg Thr Gln Lys
    50                  55


<210> 351
<211> 59
<212> PRT
<213> Plasmodium falciparum

<400> 351
His Tyr Leu Gln Gln Met Lys Glu Lys Phe Ser Lys Glu Lys Asn Asn
 1               5                  10                  15

Asn Val Ile Glu Val Thr Asn Lys Ala Glu Lys Lys Gly Asn Val Gln
            20                  25                  30

Val Thr Asn Lys Thr Glu Lys Thr Thr Lys Val Asp Lys Asn Asn Lys
        35                  40                  45

Val Pro Lys Lys Arg Arg Thr Gln Lys Ser Lys
    50                  55

<210> 352
<211> 52
<212> PRT
<213> Plasmodium falciparum

<400> 352
His Val Asp Glu Val Met Lys Tyr Val Gln Lys Ile Asp Lys Glu Val
1               5                   10                  15

Asp Lys Glu Val Ser Lys Ala Leu Glu Ser Lys Asn Asp Val Thr Asn
            20                  25                  30

Val Leu Lys Gln Asn Gln Asp Phe Phe Ser Lys Val Lys Asn Phe Val
        35                  40                  45

Lys Lys Tyr Lys
    50

<210> 353
<211> 50
<212> PRT
<213> Plasmodium falciparum

<400> 353
His Val Asp Glu Val Met Lys Tyr Val Gln Lys Ile Asp Lys Glu Val
1               5                   10                  15

Asp Lys Glu Val Ser Lys Ala Leu Glu Ser Lys Asn Asp Val Thr Asn
            20                  25                  30

Val Leu Lys Gln Asn Gln Asp Phe Phe Ser Lys Val Lys Asn Phe Val
        35                  40                  45

Lys Lys
    50

<210> 354
<211> 43
<212> PRT
<213> Plasmodium falciparum

<400> 354
His Val Asp Glu Val Met Lys Tyr Val Gln Lys Ile Asp Lys Glu Val
1               5                   10                  15

Asp Lys Glu Val Ser Lys Ala Leu Glu Ser Lys Asn Asp Val Thr Asn
            20                  25                  30

Val Leu Lys Gln Asn Gln Asp Phe Phe Ser Lys
        35                  40

<210> 355
<211> 35
<212> PRT
<213> Plasmodium falciparum

<400> 355
His Val Asp Glu Val Met Lys Tyr Val Gln Lys Ile Asp Lys Glu Val
1               5                   10                  15

Asp Lys Glu Val Ser Lys Ala Leu Glu Ser Lys Asn Asp Val Thr Asn
            20                  25                  30

Val Leu Lys
        35


<210> 356
<211> 27
<212> PRT
<213> Plasmodium falciparum

<400> 356
His Val Asp Glu Val Met Lys Tyr Val Gln Lys Ile Asp Lys Glu Val
1               5                   10                  15

Asp Lys Glu Val Ser Lys Ala Leu Glu Ser Lys
            20                  25


<210> 357
<211> 22
<212> PRT
<213> Plasmodium falciparum

<400> 357
His Val Asp Glu Val Met Lys Tyr Val Gln Lys Ile Asp Lys Glu Val
1               5                   10                  15

Asp Lys Glu Val Ser Lys
            20


<210> 358
<211> 18
<212> PRT
<213> Plasmodium falciparum

<400> 358
His Val Asp Glu Val Met Lys Tyr Val Gln Lys Ile Asp Lys Glu Val
1               5                   10                  15

Asp Lys


<210> 359
<211> 14
<212> PRT
<213> Plasmodium falciparum

<400> 359
His Val Asp Glu Val Met Lys Tyr Val Gln Lys Ile Asp Lys
1               5                   10

<210> 360
<211> 39
<212> PRT
<213> Plasmodium falciparum

<400> 360
Lys Asp Glu Val Ile Asp Leu Ile Val Gln Lys Glu Lys Arg Ile Glu
1               5                   10                  15

Lys Val Lys Ala Lys Lys Lys Lys Leu Glu Lys Lys Val Glu Glu Gly
            20                  25                  30

Val Ser Gly Leu Lys Lys His
        35

<210> 361
<211> 23
<212> PRT
<213> Plasmodium falciparum

<400> 361
Lys Val Lys Ala Lys Lys Lys Lys Leu Glu Lys Lys Val Glu Glu Gly
1               5                   10                  15

Val Ser Gly Leu Lys Lys His
            20

<210> 362
<211> 21
<212> PRT
<213> Plasmodium falciparum

<400> 362
Lys Ala Lys Lys Lys Lys Leu Glu Lys Lys Val Glu Glu Gly Val Ser
1               5                   10                  15

Gly Leu Lys Lys His
            20

<210> 363
<211> 19
<212> PRT
<213> Plasmodium falciparum

<400> 363
Lys Lys Lys Lys Leu Glu Lys Lys Val Glu Glu Gly Val Ser Gly Leu
1               5                   10                  15

Lys Lys His

<210> 364
<211> 18
<212> PRT
<213> Plasmodium falciparum

<400> 364
Lys Lys Lys Leu Glu Lys Lys Val Glu Glu Gly Val Ser Gly Leu Lys
1               5                   10                  15

Lys His


<210> 365
<211> 17
<212> PRT
<213> Plasmodium falciparum

<400> 365
Lys Lys Leu Glu Lys Lys Val Glu Glu Gly Val Ser Gly Leu Lys Lys
1               5                   10                  15

His


<210> 366
<211> 16
<212> PRT
<213> Plasmodium falciparum

<400> 366
Lys Leu Glu Lys Lys Val Glu Glu Gly Val Ser Gly Leu Lys Lys His
1               5                   10                  15


<210> 367
<211> 13
<212> PRT
<213> Plasmodium falciparum

<400> 367
Lys Lys Val Glu Glu Gly Val Ser Gly Leu Lys Lys His
1               5                   10


<210> 368
<211> 12
<212> PRT
<213> Plasmodium falciparum

<400> 368
Lys Val Glu Glu Gly Val Ser Gly Leu Lys Lys His
1               5                   10


<210> 369
<211> 59

<212> PRT
<213> Plasmodium falciparum

<400> 369
His Val Glu Gln Asn Val Tyr Val Asp Val Asp Val Pro Ala Met Lys
1               5                   10                  15

Asp Gln Phe Leu Gly Ile Leu Asn Glu Ala Gly Gly Leu Lys Glu Met
            20                  25                  30

Phe Phe Asn Leu Glu Asp Val Phe Lys Ser Glu Ser Asp Val Ile Thr
        35                  40                  45

Val Glu Glu Ile Lys Asp Glu Pro Val Gln Lys
    50                  55


<210> 370
<211> 53
<212> PRT
<213> Plasmodium falciparum

<400> 370
His Ile Lys Gly Leu Glu Glu Asp Asp Leu Glu Glu Val Asp Asp Leu
1               5                   10                  15

Lys Gly Ser Ile Leu Asp Met Leu Lys Gly Asp Met Glu Leu Gly Asp
            20                  25                  30

Met Asp Lys Glu Ser Leu Glu Asp Val Thr Thr Lys Leu Gly Glu Arg
        35                  40                  45

Val Glu Ser Leu Lys
    50


<210> 371
<211> 44
<212> PRT
<213> Plasmodium falciparum

<400> 371
His Ile Lys Gly Leu Glu Glu Asp Asp Leu Glu Glu Val Asp Asp Leu
1               5                   10                  15

Lys Gly Ser Ile Leu Asp Met Leu Lys Gly Asp Met Glu Leu Gly Asp
            20                  25                  30

Met Asp Lys Glu Ser Leu Glu Asp Val Thr Thr Lys
        35                  40


<210> 372
<211> 35
<212> PRT
<213> Plasmodium falciparum

<400> 372
His Ile Lys Gly Leu Glu Glu Asp Asp Leu Glu Glu Val Asp Asp Leu
1               5                   10                  15

Lys Gly Ser Ile Leu Asp Met Leu Lys Gly Asp Met Glu Leu Gly Asp
            20                  25                  30

Met Asp Lys
        35


<210> 373
<211> 25
<212> PRT
<213> Plasmodium falciparum

<400> 373
His Ile Lys Gly Leu Glu Glu Asp Asp Leu Glu Glu Val Asp Asp Leu
1               5                   10                  15

Lys Gly Ser Ile Leu Asp Met Leu Lys
            20                  25


<210> 374
<211> 31
<212> PRT
<213> Plasmodium falciparum

<400> 374
His Ile Ile Ser Gly Asp Ala Asp Val Leu Ser Ser Ala Leu Gly Met
1               5                   10                  15

Asp Glu Glu Gln Met Lys Thr Arg Lys Lys Ala Gln Arg Pro Lys
            20                  25                  30


<210> 375
<211> 23
<212> PRT
<213> Plasmodium falciparum

<400> 375
His Asp Ile Thr Thr Thr Leu Asp Glu Val Val Glu Leu Lys Asp Val
1               5                   10                  15

Glu Glu Asp Lys Ile Glu Lys
            20


<210> 376
<211> 10
<212> PRT
<213> Plasmodium falciparum

<400> 376
Lys Lys Leu Glu Glu Val His Glu Leu Lys
1               5                   10

<210> 377
<211> 9
<212> PRT
<213> Plasmodium falciparum

<400> 377
Lys Leu Glu Glu Val His Glu Leu Lys
1               5


<210> 378
<211> 19
<212> PRT
<213> Plasmodium falciparum

<400> 378
Lys Thr Ile Glu Thr Asp Ile Leu Glu Glu Lys Lys Lys Glu Ile Glu
1               5                   10                  15

Lys Asp His


<210> 379
<211> 11
<212> PRT
<213> Plasmodium falciparum

<400> 379
Lys Lys Glu Ile Glu Lys Asp His Phe Glu Lys
1               5                   10


<210> 380
<211> 6
<212> PRT
<213> Plasmodium falciparum

<400> 380
Lys Asp His Phe Glu Lys
1               5


<210> 381
<211> 11
<212> PRT
<213> Plasmodium falciparum

<400> 381
Lys Phe Glu Glu Glu Ala Glu Glu Ile Lys His
1               5                   10


<210> 382
<211> 47
<212> PRT
<213> Plasmodium falciparum

<400> 382
Lys Asp Gly Asp Thr Lys Cys Thr Leu Glu Cys Ala Gln Gly Lys Lys
1               5                   10                  15

Cys Ile Lys His Lys Ser Asp His Asn His Lys Ser Asp His Asn His
            20                  25                  30

Lys Ser Asp Pro Asn His Lys Lys Lys Asn Asn Asn Asn Asn Lys
        35                  40                  45


<210> 383
<211> 40
<212> PRT
<213> Plasmodium falciparum

<400> 383
Lys Asp Gly Asp Thr Lys Cys Thr Leu Glu Cys Ala Gln Gly Lys Lys
1               5                   10                  15

Cys Ile Lys His Lys Ser Asp His Asn His Lys Ser Asp His Asn His
            20                  25                  30

Lys Ser Asp Pro Asn His Lys Lys
        35                  40


<210> 384
<211> 39
<212> PRT
<213> Plasmodium falciparum

<400> 384
Lys Asp Gly Asp Thr Lys Cys Thr Leu Glu Cys Ala Gln Gly Lys Lys
1               5                   10                  15

Cys Ile Lys His Lys Ser Asp His Asn His Lys Ser Asp His Asn His
            20                  25                  30

Lys Ser Asp Pro Asn His Lys
        35


<210> 385
<211> 33
<212> PRT
<213> Plasmodium falciparum

<400> 385
Lys Asp Gly Asp Thr Lys Cys Thr Leu Glu Cys Ala Gln Gly Lys Lys
1               5                   10                  15

Cys Ile Lys His Lys Ser Asp His Asn His Lys Ser Asp His Asn His
            20                  25                  30

Lys

<210> 386
<211> 27
<212> PRT
<213> Plasmodium falciparum

<400> 386
Lys Asp Gly Asp Thr Lys Cys Thr Leu Glu Cys Ala Gln Gly Lys Lys
1               5                   10                  15

Cys Ile Lys His Lys Ser Asp His Asn His Lys
            20                  25


<210> 387
<211> 21
<212> PRT
<213> Plasmodium falciparum

<400> 387
Lys Asp Gly Asp Thr Lys Cys Thr Leu Glu Cys Ala Gln Gly Lys Lys
1               5                   10                  15

Cys Ile Lys His Lys
            20


<210> 388
<211> 16
<212> PRT
<213> Plasmodium falciparum

<400> 388
Lys Asp Gly Asp Thr Lys Cys Thr Leu Glu Cys Ala Gln Gly Lys Lys
1               5                   10                  15


<210> 389
<211> 15
<212> PRT
<213> Plasmodium falciparum

<400> 389
Lys Asp Gly Asp Thr Lys Cys Thr Leu Glu Cys Ala Gln Gly Lys
1               5                   10                  15


<210> 390
<211> 23
<212> PRT
<213> Plasmodium falciparum

<400> 390
Lys Cys Ile Gln Ala Glu Cys Asn Tyr Lys Glu Cys Gly Glu Gln Lys
1               5                   10                  15

Cys Val Trp Asp Gly Ile His
            20

<210> 391
<211> 14
<212> PRT
<213> Plasmodium falciparum

<400> 391
Lys Glu Cys Gly Glu Gln Lys Cys Val Trp Asp Gly Ile His
1               5                   10

<210> 392
<211> 32
<212> PRT
<213> Plasmodium falciparum

<400> 392
His Ile Glu Cys Lys Cys Asn Asn Asp Tyr Val Leu Thr Asn Arg Tyr
1               5                   10                  15

Glu Cys Glu Pro Lys Asn Lys Cys Thr Ser Leu Glu Asp Thr Asn Lys
            20                  25                  30

<210> 393
<211> 39
<212> PRT
<213> Plasmodium falciparum

<400> 393
Lys Ser Asp His Asn His Lys Ser Asp His Asn His Lys Ser Asp His
1               5                   10                  15

Asn His Lys Ser Asp His Asn His Lys Ser Asp Pro Asn His Lys Lys
            20                  25                  30

Lys Asn Asn Asn Asn Asn Lys
        35

<210> 394
<211> 33
<212> PRT
<213> Plasmodium falciparum

<400> 394
Lys Ser Asp His Asn His Lys Ser Asp His Asn His Lys Ser Asp His
1               5                   10                  15

Asn His Lys Ser Asp Pro Asn His Lys Lys Lys Asn Asn Asn Asn Asn
            20                  25                  30

Lys

<210> 395
<211> 27

<212> PRT
<213> Plasmodium falciparum

<400> 395
Lys Ser Asp His Asn His Lys Ser Asp His Asn His Lys Ser Asp Pro
1               5                   10                  15

Asn His Lys Lys Lys Asn Asn Asn Asn Asn Lys
            20                  25


<210> 396
<211> 21
<212> PRT
<213> Plasmodium falciparum

<400> 396
Lys Ser Asp His Asn His Lys Ser Asp Pro Asn His Lys Lys Lys Asn
1               5                   10                  15

Asn Asn Asn Asn Lys
            20


<210> 397
<211> 18
<212> PRT
<213> Plasmodium falciparum

<400> 397
Lys Lys Lys Asn Asn Asn Asn Asn Lys Asp Asn Lys Ser Asp Pro Asn
1               5                   10                  15

His Lys


<210> 398
<211> 17
<212> PRT
<213> Plasmodium falciparum

<400> 398
Lys Lys Asn Asn Asn Asn Asn Lys Asp Asn Lys Ser Asp Pro Asn His
1               5                   10                  15

Lys


<210> 399
<211> 16
<212> PRT
<213> Plasmodium falciparum

<400> 399
Lys Asn Asn Asn Asn Asn Lys Asp Asn Lys Ser Asp Pro Asn His Lys
1               5                   10                  15

<210> 400
<211> 10
<212> PRT
<213> Plasmodium falciparum

<400> 400
Lys Asp Asn Lys Ser Asp Pro Asn His Lys
1               5                   10


<210> 401
<211> 7
<212> PRT
<213> Plasmodium falciparum

<400> 401
Lys Ser Asp Pro Asn His Lys
1               5


<210> 402
<211> 35
<212> PRT
<213> Plasmodium falciparum

<400> 402
His Ser Leu Tyr Ala Leu Gln Gln Asn Glu Glu Tyr Gln Lys Val Lys
1               5                   10                  15

Asn Glu Lys Asp Gln Asn Glu Ile Lys Lys Ile Lys Gln Leu Ile Glu
            20                  25                  30

Lys Asn Lys
        35


<210> 403
<211> 28
<212> PRT
<213> Plasmodium falciparum

<400> 403
His Ser Leu Tyr Ala Leu Gln Gln Asn Glu Glu Tyr Gln Lys Val Lys
1               5                   10                  15

Asn Glu Lys Asp Gln Asn Glu Ile Lys Lys Ile Lys
            20                  25


<210> 404
<211> 26
<212> PRT
<213> Plasmodium falciparum

<400> 404
His Ser Leu Tyr Ala Leu Gln Gln Asn Glu Glu Tyr Gln Lys Val Lys
1               5                   10                  15

Asn Glu Lys Asp Gln Asn Glu Ile Lys Lys
            20                  25

<210> 405
<211> 25
<212> PRT
<213> Plasmodium falciparum

<400> 405
His Ser Leu Tyr Ala Leu Gln Gln Asn Glu Glu Tyr Gln Lys Val Lys
1               5                   10                  15

Asn Glu Lys Asp Gln Asn Glu Ile Lys
            20                  25

<210> 406
<211> 11
<212> PRT
<213> Plasmodium falciparum

<400> 406
His Lys Leu Glu Asn Leu Glu Glu Met Asp Lys
1               5                   10

<210> 407
<211> 11
<212> PRT
<213> Plasmodium falciparum

<400> 407
Lys His Phe Asp Asp Asn Thr Asn Glu Gln Lys
1               5                   10

<210> 408
<211> 8
<212> PRT
<213> Plasmodium falciparum

<400> 408
Lys Lys Glu Asp Asp Glu Lys His
1               5

<210> 409
<211> 13
<212> PRT
<213> Plasmodium falciparum

<400> 409
Lys Glu Glu Asn Asn Lys Lys Glu Asp Asp Glu Lys His
1               5                   10

<210> 410
<211> 21
<212> PRT
<213> Plasmodium falciparum

<400> 410
Lys Thr Ser Ser Gly Ile Leu Asn Lys Glu Glu Asn Asn Lys Lys Glu
1               5                   10                  15

Asp Asp Glu Lys His
            20


<210> 411
<211> 7
<212> PRT
<213> Plasmodium falciparum

<400> 411
Lys Asn Ile His Ile Lys Lys
1               5


<210> 412
<211> 13
<212> PRT
<213> Plasmodium falciparum

<400> 412
His Ile Lys Lys Lys Glu Gly Ile Asp Ile Gly Tyr Lys
1               5                   10


<210> 413
<211> 21
<212> PRT
<213> Plasmodium falciparum

<400> 413
Lys Lys Met Trp Thr Cys Lys Leu Trp Asp Asn Lys Gly Asn Glu Ile
1               5                   10                  15

Thr Lys Asn Ile His
            20


<210> 414
<211> 30
<212> PRT
<213> Plasmodium falciparum

<400> 414
Lys Lys Gly Ile Gln Trp Asn Leu Leu Lys Lys Met Trp Thr Cys Lys
1               5                   10                  15

Leu Trp Asp Asn Lys Gly Asn Glu Ile Thr Lys Asn Ile His
            20                  25                  30

<210> 415
<211> 50
<212> PRT
<213> Plasmodium falciparum

<400> 415
Lys Glu Lys Lys Asp Ser Asn Glu Asn Arg Lys Lys Lys Gln Lys Glu
1               5                   10                  15

Asp Lys Lys Asn Pro Asn Lys Leu Lys Lys Ile Glu Tyr Thr Asn Lys
            20                  25                  30

Ile Thr His Phe Phe Lys Ala Lys Asn Asn Lys Gln Gln Asn Asn Val
        35                  40                  45

Thr His
    50


<210> 416
<211> 48
<212> PRT
<213> Plasmodium falciparum

<400> 416
Lys Lys Asp Ser Asn Glu Asn Arg Lys Lys Lys Gln Lys Glu Asp Lys
1               5                   10                  15

Lys Asn Pro Asn Lys Leu Lys Lys Ile Glu Tyr Thr Asn Lys Ile Thr
            20                  25                  30

His Phe Phe Lys Ala Lys Asn Asn Lys Gln Gln Asn Asn Val Thr His
        35                  40                  45


<210> 417
<211> 47
<212> PRT
<213> Plasmodium falciparum

<400> 417
Lys Asp Ser Asn Glu Asn Arg Lys Lys Lys Gln Lys Glu Asp Lys Lys
1               5                   10                  15

Asn Pro Asn Lys Leu Lys Lys Ile Glu Tyr Thr Asn Lys Ile Thr His
            20                  25                  30

Phe Phe Lys Ala Lys Asn Asn Lys Gln Gln Asn Asn Val Thr His
        35                  40                  45


<210> 418
<211> 39
<212> PRT
<213> Plasmodium falciparum

<400> 418
Lys Lys Gln Lys Glu Asp Lys Lys Asn Pro Asn Lys Leu Lys Lys Ile
1               5                   10                  15

Glu Tyr Thr Asn Lys Ile Thr His Phe Phe Lys Ala Lys Asn Asn Lys
            20                  25                  30

Gln Gln Asn Asn Val Thr His
        35


<210> 419
<211> 38
<212> PRT
<213> Plasmodium falciparum

<400> 419
Lys Gln Lys Glu Asp Lys Lys Asn Pro Asn Lys Leu Lys Lys Ile Glu
1               5                   10                  15

Tyr Thr Asn Lys Ile Thr His Phe Phe Lys Ala Lys Asn Asn Lys Gln
            20                  25                  30

Gln Asn Asn Val Thr His
        35


<210> 420
<211> 36
<212> PRT
<213> Plasmodium falciparum

<400> 420
Lys Glu Asp Lys Lys Asn Pro Asn Lys Leu Lys Lys Ile Glu Tyr Thr
1               5                   10                  15

Asn Lys Ile Thr His Phe Phe Lys Ala Lys Asn Asn Lys Gln Gln Asn
            20                  25                  30

Asn Val Thr His
        35


<210> 421
<211> 32
<212> PRT
<213> Plasmodium falciparum

<400> 421
Lys Asn Pro Asn Lys Leu Lys Lys Ile Glu Tyr Thr Asn Lys Ile Thr
1               5                   10                  15

His Phe Phe Lys Ala Lys Asn Asn Lys Gln Gln Asn Asn Val Thr His
            20                  25                  30


<210> 422
<211> 26

<212> PRT
<213> Plasmodium falciparum

<400> 422
Lys Lys Ile Glu Tyr Thr Asn Lys Ile Thr His Phe Phe Lys Ala Lys
1               5                   10                  15

Asn Asn Lys Gln Gln Asn Asn Val Thr His
            20                  25


<210> 423
<211> 25
<212> PRT
<213> Plasmodium falciparum

<400> 423
Lys Ile Glu Tyr Thr Asn Lys Ile Thr His Phe Phe Lys Ala Lys Asn
1               5                   10                  15

Asn Lys Gln Gln Asn Asn Val Thr His
            20                  25


<210> 424
<211> 19
<212> PRT
<213> Plasmodium falciparum

<400> 424
Lys Ile Thr His Phe Phe Lys Ala Lys Asn Asn Lys Gln Gln Asn Asn
1               5                   10                  15

Val Thr His


<210> 425
<211> 48
<212> PRT
<213> Plasmodium falciparum

<400> 425
His Lys Asn Asn Glu Asp Ile Lys Asn Asp Asn Ser Lys Asp Ile Lys
1               5                   10                  15

Asn Asp Asn Ser Lys Asp Ile Lys Asn Asp Asn Ser Lys Asp Ile Lys
            20                  25                  30

Asn Asp Asn Asn Glu Asp Ile Lys Asn Asp Asn Ser Lys Asp Ile Lys
        35                  40                  45


<210> 426
<211> 45
<212> PRT
<213> Plasmodium falciparum

<400> 426
His Lys Asn Asn Glu Asp Ile Lys Asn Asp Asn Ser Lys Asp Ile Lys
1               5                   10                  15

Asn Asp Asn Ser Lys Asp Ile Lys Asn Asp Asn Ser Lys Asp Ile Lys
            20                  25                  30

Asn Asp Asn Asn Glu Asp Ile Lys Asn Asp Asn Ser Lys
        35                  40                  45


<210> 427
<211> 40
<212> PRT
<213> Plasmodium falciparum

<400> 427
His Lys Asn Asn Glu Asp Ile Lys Asn Asp Asn Ser Lys Asp Ile Lys
1               5                   10                  15

Asn Asp Asn Ser Lys Asp Ile Lys Asn Asp Asn Ser Lys Asp Ile Lys
            20                  25                  30

Asn Asp Asn Asn Glu Asp Ile Lys
        35                  40


<210> 428
<211> 32
<212> PRT
<213> Plasmodium falciparum

<400> 428
His Lys Asn Asn Glu Asp Ile Lys Asn Asp Asn Ser Lys Asp Ile Lys
1               5                   10                  15

Asn Asp Asn Ser Lys Asp Ile Lys Asn Asp Asn Ser Lys Asp Ile Lys
            20                  25                  30


<210> 429
<211> 29
<212> PRT
<213> Plasmodium falciparum

<400> 429
His Lys Asn Asn Glu Asp Ile Lys Asn Asp Asn Ser Lys Asp Ile Lys
1               5                   10                  15

Asn Asp Asn Ser Lys Asp Ile Lys Asn Asp Asn Ser Lys
            20                  25


<210> 430
<211> 24
<212> PRT
<213> Plasmodium falciparum

<400> 430
His Lys Asn Asn Glu Asp Ile Lys Asn Asp Asn Ser Lys Asp Ile Lys
1               5                   10                  15

Asn Asp Asn Ser Lys Asp Ile Lys
            20


<210> 431
<211> 21
<212> PRT
<213> Plasmodium falciparum

<400> 431
His Lys Asn Asn Glu Asp Ile Lys Asn Asp Asn Ser Lys Asp Ile Lys
1               5                   10                  15

Asn Asp Asn Ser Lys
            20


<210> 432
<211> 16
<212> PRT
<213> Plasmodium falciparum

<400> 432
His Lys Asn Asn Glu Asp Ile Lys Asn Asp Asn Ser Lys Asp Ile Lys
1               5                   10                  15


<210> 433
<211> 8
<212> PRT
<213> Plasmodium falciparum

<400> 433
His Lys Asn Asn Glu Asp Ile Lys
1               5


<210> 434
<211> 31
<212> PRT
<213> Plasmodium falciparum

<400> 434
Lys Lys Tyr Asp Asp Leu Gln Asn Lys Tyr Asn Ile Leu Asn Lys Leu
1               5                   10                  15

Lys Asn Ser Leu Glu Glu Lys Asn Glu Glu Leu Lys Lys Tyr His
            20                  25                  30


<210> 435
<211> 30
<212> PRT
<213> Plasmodium falciparum

<400> 435
Lys Tyr Asp Asp Leu Gln Asn Lys Tyr Asn Ile Leu Asn Lys Leu Lys
1               5                   10                  15

Asn Ser Leu Glu Glu Lys Asn Glu Glu Leu Lys Lys Tyr His
            20                  25                  30


<210> 436
<211> 23
<212> PRT
<213> Plasmodium falciparum

<400> 436
Lys Tyr Asn Ile Leu Asn Lys Leu Lys Asn Ser Leu Glu Glu Lys Asn
1               5                   10                  15

Glu Glu Leu Lys Lys Tyr His
            20


<210> 437
<211> 17
<212> PRT
<213> Plasmodium falciparum

<400> 437
Lys Leu Lys Asn Ser Leu Glu Glu Lys Asn Glu Glu Leu Lys Lys Tyr
1               5                   10                  15

His


<210> 438
<211> 15
<212> PRT
<213> Plasmodium falciparum

<400> 438
Lys Asn Ser Leu Glu Glu Lys Asn Glu Glu Leu Lys Lys Tyr His
1               5                   10                  15


<210> 439
<211> 9
<212> PRT
<213> Plasmodium falciparum

<400> 439
Lys Asn Glu Glu Leu Lys Lys Tyr His
1               5


<210> 440
<211> 35
<212> PRT
<213> Plasmodium falciparum

<400> 440
His Met Gly Asn Asn Gln Asp Ile Asn Glu Asn Val Tyr Asn Ile Lys
1               5                   10                  15

Pro Gln Glu Phe Lys Glu Glu Glu Glu Glu Asp Ile Ser Met Val Asn
            20                  25                  30

Thr Lys Lys
        35


<210> 441
<211> 17
<212> PRT
<213> Plasmodium falciparum

<400> 441
Lys Asn Ser Asn Glu Leu Lys Arg Ile Asn Asp Asn Phe Phe Lys Leu
1               5                   10                  15

His


<210> 442
<211> 55
<212> PRT
<213> Plasmodium falciparum

<400> 442
Lys Pro Cys Leu Tyr Lys Lys Cys Lys Ile Ser Gln Cys Leu Tyr Lys
1               5                   10                  15

Lys Cys Lys Ile Ser Gln Val Trp Trp Cys Met Pro Val Lys Asp Thr
            20                  25                  30

Phe Asn Thr Tyr Glu Arg Asn Asn Val Leu Asn Ser Lys Ile Glu Asn
        35                  40                  45

Asn Ile Glu Lys Ile Pro His
    50                  55


<210> 443
<211> 40
<212> PRT
<213> Plasmodium falciparum

<400> 443
His Ile Asn Asn Glu Tyr Thr Asn Lys Asn Pro Lys Asn Cys Leu Leu
1               5                   10                  15

Tyr Lys Asn Glu Glu Arg Asn Tyr Asn Asp Asn Asn Ile Lys Asp Tyr
            20                  25                  30

Ile Asn Ser Met Asn Phe Lys Lys
        35                  40

<210> 444
<211> 39
<212> PRT
<213> Plasmodium falciparum

<400> 444
His Ile Asn Asn Glu Tyr Thr Asn Lys Asn Pro Lys Asn Cys Leu Leu
1               5                   10                  15

Tyr Lys Asn Glu Glu Arg Asn Tyr Asn Asp Asn Asn Ile Lys Asp Tyr
            20                  25                  30

Ile Asn Ser Met Asn Phe Lys
        35


<210> 445
<211> 18
<212> PRT
<213> Plasmodium falciparum

<400> 445
His Ile Asn Asn Glu Tyr Thr Asn Lys Asn Pro Lys Asn Cys Leu Leu
1               5                   10                  15

Tyr Lys


<210> 446
<211> 23
<212> PRT
<213> Plasmodium falciparum

<400> 446
Lys Asn Lys Thr Asn Gln Ser Lys Gly Val Lys Gly Glu Tyr Glu Lys
1               5                   10                  15

Lys Lys Glu Thr Asn Gly His
            20


<210> 447
<211> 21
<212> PRT
<213> Plasmodium falciparum

<400> 447
Lys Thr Asn Gln Ser Lys Gly Val Lys Gly Glu Tyr Glu Lys Lys Lys
1               5                   10                  15

Glu Thr Asn Gly His
            20


<210> 448
<211> 16
<212> PRT

<213> Plasmodium falciparum

<400> 448
Lys Gly Val Lys Gly Glu Tyr Glu Lys Lys Lys Glu Thr Asn Gly His
1               5                   10                  15


<210> 449
<211> 13
<212> PRT
<213> Plasmodium falciparum

<400> 449
Lys Gly Glu Tyr Glu Lys Lys Lys Glu Thr Asn Gly His
1               5                   10


<210> 450
<211> 28
<212> PRT
<213> Plasmodium falciparum

<400> 450
Lys Ser Gly Met Tyr Thr Asn Glu Gly Asn Lys Ser Cys Glu Cys Ser
1               5                   10                  15

Tyr Lys Lys Lys Ser Ser Ser Ser Asn Lys Val His
            20                  25


<210> 451
<211> 18
<212> PRT
<213> Plasmodium falciparum

<400> 451
Lys Ser Cys Glu Cys Ser Tyr Lys Lys Lys Ser Ser Ser Ser Asn Lys
1               5                   10                  15

Val His


<210> 452
<211> 11
<212> PRT
<213> Plasmodium falciparum

<400> 452
Lys Lys Lys Ser Ser Ser Ser Asn Lys Val His
1               5                   10


<210> 453
<211> 10
<212> PRT
<213> Plasmodium falciparum

<400> 453
Lys Lys Ser Ser Ser Ser Asn Lys Val His
1               5                   10


<210> 454
<211> 9
<212> PRT
<213> Plasmodium falciparum

<400> 454
Lys Ser Ser Ser Ser Asn Lys Val His
1               5


<210> 455
<211> 30
<212> PRT
<213> Plasmodium falciparum

<400> 455
His Ile Met Leu Lys Ser Gly Met Tyr Thr Asn Glu Gly Asn Lys Ser
1               5                   10                  15

Cys Glu Cys Ser Tyr Lys Lys Lys Ser Ser Ser Ser Asn Lys
            20                  25                  30


<210> 456
<211> 24
<212> PRT
<213> Plasmodium falciparum

<400> 456
His Ile Met Leu Lys Ser Gly Met Tyr Thr Asn Glu Gly Asn Lys Ser
1               5                   10                  15

Cys Glu Cys Ser Tyr Lys Lys Lys
            20


<210> 457
<211> 23
<212> PRT
<213> Plasmodium falciparum

<400> 457
His Ile Met Leu Lys Ser Gly Met Tyr Thr Asn Glu Gly Asn Lys Ser
1               5                   10                  15

Cys Glu Cys Ser Tyr Lys Lys
            20


<210> 458
<211> 22
<212> PRT
<213> Plasmodium falciparum

<400> 458
His Ile Met Leu Lys Ser Gly Met Tyr Thr Asn Glu Gly Asn Lys Ser
1               5                   10                  15

Cys Glu Cys Ser Tyr Lys
            20


<210> 459
<211> 50
<212> PRT
<213> Plasmodium falciparum

<400> 459
Lys Pro Leu Ala Lys Leu Arg Lys Arg Glu Lys Thr Gln Ile Asn Lys
1               5                   10                  15

Thr Lys Tyr Glu Arg Gly Asp Val Ile Ile Asp Asn Thr Glu Ile Gln
            20                  25                  30

Lys Ile Ile Ile Arg Asp Tyr His Glu Thr Leu Asn Val His Lys Leu
        35                  40                  45

Asp His
    50


<210> 460
<211> 43
<212> PRT
<213> Plasmodium falciparum

<400> 460
Lys Arg Glu Lys Thr Gln Ile Asn Lys Thr Lys Tyr Glu Arg Gly Asp
1               5                   10                  15

Val Ile Ile Asp Asn Thr Glu Ile Gln Lys Ile Ile Ile Arg Asp Tyr
            20                  25                  30

His Glu Thr Leu Asn Val His Lys Leu Asp His
        35                  40


<210> 461
<211> 40
<212> PRT
<213> Plasmodium falciparum

<400> 461
Lys Thr Gln Ile Asn Lys Thr Lys Tyr Glu Arg Gly Asp Val Ile Ile
1               5                   10                  15

Asp Asn Thr Glu Ile Gln Lys Ile Ile Ile Arg Asp Tyr His Glu Thr
            20                  25                  30

Leu Asn Val His Lys Leu Asp His
        35                  40

<210> 462
<211> 46
<212> PRT
<213> Plasmodium falciparum

<400> 462
Lys Pro Leu Ala Lys Leu Arg Lys Arg Glu Lys Thr Gln Ile Asn Lys
1               5                   10                  15

Thr Lys Tyr Glu Arg Gly Asp Val Ile Ile Asp Asn Thr Glu Ile Gln
            20                  25                  30

Lys Ile Ile Ile Arg Asp Tyr His Glu Thr Leu Asn Val His
        35                  40                  45


<210> 463
<211> 40
<212> PRT
<213> Plasmodium falciparum

<400> 463
Lys Pro Leu Ala Lys Leu Arg Lys Arg Glu Lys Thr Gln Ile Asn Lys
1               5                   10                  15

Thr Lys Tyr Glu Arg Gly Asp Val Ile Ile Asp Asn Thr Glu Ile Gln
            20                  25                  30

Lys Ile Ile Ile Arg Asp Tyr His
        35                  40


<210> 464
<211> 36
<212> PRT
<213> Plasmodium falciparum

<400> 464
Lys Leu Arg Lys Arg Glu Lys Thr Gln Ile Asn Lys Thr Lys Tyr Glu
1               5                   10                  15

Arg Gly Asp Val Ile Ile Asp Asn Thr Glu Ile Gln Lys Ile Ile Ile
            20                  25                  30

Arg Asp Tyr His
        35


<210> 465
<211> 33
<212> PRT
<213> Plasmodium falciparum

<400> 465
Lys Arg Glu Lys Thr Gln Ile Asn Lys Thr Lys Tyr Glu Arg Gly Asp
1               5                   10                  15

Val Ile Ile Asp Asn Thr Glu Ile Gln Lys Ile Ile Ile Arg Asp Tyr
            20                  25                  30

His

<210> 466
<211> 30
<212> PRT
<213> Plasmodium falciparum

<400> 466
Lys Thr Gln Ile Asn Lys Thr Lys Tyr Glu Arg Gly Asp Val Ile Ile
1               5                   10                  15

Asp Asn Thr Glu Ile Gln Lys Ile Ile Ile Arg Asp Tyr His
            20                  25                  30

<210> 467
<211> 41
<212> PRT
<213> Plasmodium falciparum

<400> 467
Lys Lys Asp Lys Glu Lys Lys Lys Asp Ser Asn Glu Asn Arg Lys Lys
1               5                   10                  15

Lys Gln Lys Glu Asp Lys Lys Asn Pro Asn Asp Asn Lys Leu Lys Lys
            20                  25                  30

Ile Glu Tyr Thr Asn Lys Ile Thr His
        35                  40

<210> 468
<211> 40
<212> PRT
<213> Plasmodium falciparum

<400> 468
Lys Asp Lys Glu Lys Lys Lys Asp Ser Asn Glu Asn Arg Lys Lys Lys
1               5                   10                  15

Gln Lys Glu Asp Lys Lys Asn Pro Asn Asp Asn Lys Leu Lys Lys Ile
            20                  25                  30

Glu Tyr Thr Asn Lys Ile Thr His
        35                  40

<210> 469
<211> 38
<212> PRT
<213> Plasmodium falciparum

<400> 469
Lys Glu Lys Lys Lys Asp Ser Asn Glu Asn Arg Lys Lys Lys Gln Lys
1               5                   10                  15

Glu Asp Lys Lys Asn Pro Asn Asp Asn Lys Leu Lys Lys Ile Glu Tyr
            20                  25                  30

Thr Asn Lys Ile Thr His
        35


<210> 470
<211> 36
<212> PRT
<213> Plasmodium falciparum

<400> 470
Lys Lys Lys Asp Ser Asn Glu Asn Arg Lys Lys Lys Gln Lys Glu Asp
1               5                   10                  15

Lys Lys Asn Pro Asn Asp Asn Lys Leu Lys Lys Ile Glu Tyr Thr Asn
            20                  25                  30

Lys Ile Thr His
        35


<210> 471
<211> 35
<212> PRT
<213> Plasmodium falciparum

<400> 471
Lys Lys Asp Ser Asn Glu Asn Arg Lys Lys Lys Gln Lys Glu Asp Lys
1               5                   10                  15

Lys Asn Pro Asn Asp Asn Lys Leu Lys Lys Ile Glu Tyr Thr Asn Lys
            20                  25                  30

Ile Thr His
        35


<210> 472
<211> 34
<212> PRT
<213> Plasmodium falciparum

<400> 472
Lys Asp Ser Asn Glu Asn Arg Lys Lys Lys Gln Lys Glu Asp Lys Lys
1               5                   10                  15

Asn Pro Asn Asp Asn Lys Leu Lys Lys Ile Glu Tyr Thr Asn Lys Ile
            20                  25                  30

Thr His

<210> 473
<211> 27
<212> PRT
<213> Plasmodium falciparum

<400> 473
Lys Lys Lys Gln Lys Glu Asp Lys Lys Asn Pro Asn Asp Asn Lys Leu
1               5                   10                  15

Lys Lys Ile Glu Tyr Thr Asn Lys Ile Thr His
            20                  25


<210> 474
<211> 26
<212> PRT
<213> Plasmodium falciparum

<400> 474
Lys Lys Gln Lys Glu Asp Lys Lys Asn Pro Asn Asp Asn Lys Leu Lys
1               5                   10                  15

Lys Ile Glu Tyr Thr Asn Lys Ile Thr His
            20                  25


<210> 475
<211> 25
<212> PRT
<213> Plasmodium falciparum

<400> 475
Lys Gln Lys Glu Asp Lys Lys Asn Pro Asn Asp Asn Lys Leu Lys Lys
1               5                   10                  15

Ile Glu Tyr Thr Asn Lys Ile Thr His
            20                  25


<210> 476
<211> 23
<212> PRT
<213> Plasmodium falciparum

<400> 476
Lys Glu Asp Lys Lys Asn Pro Asn Asp Asn Lys Leu Lys Lys Ile Glu
1               5                   10                  15

Tyr Thr Asn Lys Ile Thr His
            20


<210> 477
<211> 20
<212> PRT
<213> Plasmodium falciparum

<400> 477
Lys Lys Asn Pro Asn Asp Asn Lys Leu Lys Lys Ile Glu Tyr Thr Asn
1               5                   10                  15

Lys Ile Thr His
            20


<210> 478
<211> 19
<212> PRT
<213> Plasmodium falciparum

<400> 478
Lys Asn Pro Asn Asp Asn Lys Leu Lys Lys Ile Glu Tyr Thr Asn Lys
1               5                   10                  15

Ile Thr His


<210> 479
<211> 13
<212> PRT
<213> Plasmodium falciparum

<400> 479
Lys Leu Lys Lys Ile Glu Tyr Thr Asn Lys Ile Thr His
1               5                   10


<210> 480
<211> 11
<212> PRT
<213> Plasmodium falciparum

<400> 480
Lys Lys Ile Glu Tyr Thr Asn Lys Ile Thr His
1               5                   10


<210> 481
<211> 10
<212> PRT
<213> Plasmodium falciparum

<400> 481
Lys Ile Glu Tyr Thr Asn Lys Ile Thr His
1               5                   10


<210> 482
<211> 44
<212> PRT
<213> Plasmodium falciparum

<400> 482
His Gly Gln Ile Lys Ile Glu Asp Val Asn Asn Glu Asn Phe Asn Asn
1               5                   10                  15

Glu Gln Met Lys Asn Lys Tyr Asn Asp Glu Glu Lys Met Asp Ile Ser
            20                  25                  30

Lys Ser Lys Ser Leu Lys Ser Asp Phe Leu Glu Lys
        35                  40

<210> 483
<211> 38
<212> PRT
<213> Plasmodium falciparum

<400> 483
His Gly Gln Ile Lys Ile Glu Asp Val Asn Asn Glu Asn Phe Asn Asn
1               5                   10                  15

Glu Gln Met Lys Asn Lys Tyr Asn Asp Glu Glu Lys Met Asp Ile Ser
            20                  25                  30

Lys Ser Lys Ser Leu Lys
        35

<210> 484
<211> 35
<212> PRT
<213> Plasmodium falciparum

<400> 484
His Gly Gln Ile Lys Ile Glu Asp Val Asn Asn Glu Asn Phe Asn Asn
1               5                   10                  15

Glu Gln Met Lys Asn Lys Tyr Asn Asp Glu Glu Lys Met Asp Ile Ser
            20                  25                  30

Lys Ser Lys
        35

<210> 485
<211> 33
<212> PRT
<213> Plasmodium falciparum

<400> 485
His Gly Gln Ile Lys Ile Glu Asp Val Asn Asn Glu Asn Phe Asn Asn
1               5                   10                  15

Glu Gln Met Lys Asn Lys Tyr Asn Asp Glu Glu Lys Met Asp Ile Ser
            20                  25                  30

Lys

<210> 486
<211> 31
<212> PRT

<213> Plasmodium falciparum

<400> 486
Lys Lys Tyr Asp Asp Leu Gln Asn Lys Tyr Asn Ile Leu Asn Lys Leu
1               5                   10                  15

Lys Asn Ser Leu Glu Glu Lys Asn Glu Glu Leu Lys Lys Tyr His
            20                  25                  30


<210> 487
<211> 30
<212> PRT
<213> Plasmodium falciparum

<400> 487
Lys Tyr Asp Asp Leu Gln Asn Lys Tyr Asn Ile Leu Asn Lys Leu Lys
1               5                   10                  15

Asn Ser Leu Glu Glu Lys Asn Glu Glu Leu Lys Lys Tyr His
            20                  25                  30


<210> 488
<211> 23
<212> PRT
<213> Plasmodium falciparum

<400> 488
Lys Tyr Asn Ile Leu Asn Lys Leu Lys Asn Ser Leu Glu Glu Lys Asn
1               5                   10                  15

Glu Glu Leu Lys Lys Tyr His
            20


<210> 489
<211> 17
<212> PRT
<213> Plasmodium falciparum

<400> 489
Lys Leu Lys Asn Ser Leu Glu Glu Lys Asn Glu Glu Leu Lys Lys Tyr
1               5                   10                  15

His


<210> 490
<211> 15
<212> PRT
<213> Plasmodium falciparum

<400> 490
Lys Asn Ser Leu Glu Glu Lys Asn Glu Glu Leu Lys Lys Tyr His
1               5                   10                  15

<210> 491
<211> 9
<212> PRT
<213> Plasmodium falciparum

<400> 491
Lys Asn Glu Glu Leu Lys Lys Tyr His
1               5


<210> 492
<211> 44
<212> PRT
<213> Plasmodium falciparum

<400> 492
His Met Gly Asn Asn Gln Asp Ile Asn Glu Asn Val Tyr Asn Ile Lys
1               5                   10                  15

Pro Gln Glu Phe Lys Glu Glu Glu Glu Glu Asp Ile Ser Met Val Asn
            20                  25                  30

Thr Lys Lys Cys Asp Asp Ile Gln Glu Asn Ile Lys
        35                  40


<210> 493
<211> 50
<212> PRT
<213> Plasmodium falciparum

<400> 493
Lys Thr Asn Leu Tyr Asn Ile Tyr Asn Asn Lys Asn Asp Asp Lys Asp
1               5                   10                  15

Asn Ile Leu Asp Asn Glu Asn Arg Glu Gly Leu Tyr Leu Cys Asp Val
            20                  25                  30

Met Lys Asn Ser Asn Glu Leu Lys Arg Ile Asn Asp Asn Phe Phe Lys
        35                  40                  45

Leu His
    50


<210> 494
<211> 17
<212> PRT
<213> Plasmodium falciparum

<400> 494
Lys Asn Ser Asn Glu Leu Lys Arg Ile Asn Asp Asn Phe Phe Lys Leu
1               5                   10                  15

His

<210> 495
<211> 11
<212> PRT
<213> Plasmodium falciparum

<400> 495
Lys Arg Ile Asn Asp Asn Phe Phe Lys Leu His
1               5                   10


<210> 496
<211> 40
<212> PRT
<213> Plasmodium falciparum

<400> 496
His Ile Asn Asn Glu Tyr Thr Asn Lys Asn Pro Lys Asn Cys Leu Leu
1               5                   10                  15

Tyr Lys Asn Glu Glu Arg Asn Tyr Asn Asp Asn Asn Ile Lys Asp Tyr
            20                  25                  30

Ile Asn Ser Met Asn Phe Lys Lys
        35                  40


<210> 497
<211> 39
<212> PRT
<213> Plasmodium falciparum

<400> 497
His Ile Asn Asn Glu Tyr Thr Asn Lys Asn Pro Lys Asn Cys Leu Leu
1               5                   10                  15

Tyr Lys Asn Glu Glu Arg Asn Tyr Asn Asp Asn Asn Ile Lys Asp Tyr
            20                  25                  30

Ile Asn Ser Met Asn Phe Lys
        35


<210> 498
<211> 18
<212> PRT
<213> Plasmodium falciparum

<400> 498
His Ile Asn Asn Glu Tyr Thr Asn Lys Asn Pro Lys Asn Cys Leu Leu
1               5                   10                  15

Tyr Lys


<210> 499
<211> 45
<212> PRT
<213> Plasmodium falciparum

<400> 499
Lys Pro Cys Leu Tyr Lys Lys Cys Lys Ile Ser Gln Val Trp Trp Cys
1               5                   10                  15

Met Pro Val Lys Asp Thr Phe Asn Thr Tyr Glu Arg Asn Asn Val Leu
            20                  25                  30

Asn Ser Lys Ile Glu Asn Asn Ile Glu Lys Ile Pro His
        35                  40                  45


<210> 500
<211> 39
<212> PRT
<213> Plasmodium falciparum

<400> 500
Lys Cys Lys Ile Ser Gln Val Trp Trp Cys Met Pro Val Lys Asp Thr
1               5                   10                  15

Phe Asn Thr Tyr Glu Arg Asn Asn Val Leu Asn Ser Lys Ile Glu Asn
            20                  25                  30

Asn Ile Glu Lys Ile Pro His
        35


<210> 501
<211> 11
<212> PRT
<213> Plasmodium falciparum

<400> 501
Lys Ile Glu Asn Asn Ile Glu Lys Ile Pro His
1               5                   10


<210> 502
<211> 23
<212> PRT
<213> Plasmodium falciparum

<400> 502
Lys Asn Lys Thr Asn Gly Ser Lys Gly Val Lys Gly Glu Tyr Glu Lys
1               5                   10                  15

Lys Lys Glu Thr Asn Gly His
            20


<210> 503
<211> 21
<212> PRT
<213> Plasmodium falciparum

<400> 503
Lys Thr Asn Gly Ser Lys Gly Val Lys Gly Glu Tyr Glu Lys Lys Lys
1               5                   10                  15

Glu Thr Asn Gly His
            20


<210> 504
<211> 16
<212> PRT
<213> Plasmodium falciparum

<400> 504
Lys Gly Val Lys Gly Glu Tyr Glu Lys Lys Lys Glu Thr Asn Gly His
1               5                   10                  15


<210> 505
<211> 13
<212> PRT
<213> Plasmodium falciparum

<400> 505
Lys Gly Glu Tyr Glu Lys Lys Lys Glu Thr Asn Gly His
1               5                   10


<210> 506
<211> 60
<212> PRT
<213> Plasmodium falciparum

<400> 506
Lys Thr Ile Glu Lys Ile Asn Lys Ser Lys Ser Trp Phe Phe Glu Glu
1               5                   10                  15

Leu Asp Glu Ile Asp Lys Pro Leu Ala Lys Leu Arg Lys Arg Glu Lys
            20                  25                  30

Thr Gln Ile Asn Lys Thr Lys Tyr Glu Arg Gly Asp Val Ile Ile Asp
        35                  40                  45

Asn Thr Glu Ile Gln Lys Ile Ile Arg Asp Tyr His
    50                  55                  60


<210> 507
<211> 56
<212> PRT
<213> Plasmodium falciparum

<400> 507
Lys Ile Asn Lys Ser Lys Ser Trp Phe Phe Glu Glu Leu Asp Glu Ile
1               5                   10                  15

Asp Lys Pro Leu Ala Lys Leu Arg Lys Arg Glu Lys Thr Gln Ile Asn
            20                  25                  30

Lys Thr Lys Tyr Glu Arg Gly Asp Val Ile Ile Asp Asn Thr Glu Ile
        35                  40                  45

Gln Lys Ile Ile Arg Asp Tyr His
    50                  55


<210> 508
<211> 39
<212> PRT
<213> Plasmodium falciparum

<400> 508
Lys Pro Leu Ala Lys Leu Arg Lys Arg Glu Lys Thr Gln Ile Asn Lys
1               5                   10                  15

Thr Lys Tyr Glu Arg Gly Asp Val Ile Ile Asp Asn Thr Glu Ile Gln
            20                  25                  30

Lys Ile Ile Arg Asp Tyr His
        35


<210> 509
<211> 32
<212> PRT
<213> Plasmodium falciparum

<400> 509
His Ile Met Leu Lys Ser Gln Met Tyr Thr Asn Glu Gly Asn Lys Ser
1               5                   10                  15

Cys Glu Cys Ser Tyr Lys Lys Lys Ser Ser Ser Ser Asn Lys Val His
            20                  25                  30


<210> 510
<211> 35
<212> PRT
<213> Plasmodium falciparum

<400> 510
Lys Leu Arg Lys Arg Glu Lys Thr Gln Ile Asn Lys Thr Lys Tyr Glu
1               5                   10                  15

Arg Gly Asp Val Ile Ile Asp Asn Thr Glu Ile Gln Lys Ile Ile Arg
            20                  25                  30

Asp Tyr His
        35


<210> 511
<211> 32
<212> PRT
<213> Plasmodium falciparum

<400> 511
Lys Arg Glu Lys Thr Gln Ile Asn Lys Thr Lys Tyr Glu Arg Gly Asp
1               5                   10                  15

Val Ile Ile Asp Asn Thr Glu Ile Gln Lys Ile Ile Arg Asp Tyr His
            20                  25                  30


<210> 512
<211> 29
<212> PRT
<213> Plasmodium falciparum

<400> 512
Lys Thr Gln Ile Asn Lys Thr Lys Tyr Glu Arg Gly Asp Val Ile Ile
1               5                   10                  15

Asp Asn Thr Glu Ile Gln Lys Ile Ile Arg Asp Tyr His
            20                  25


<210> 513
<211> 48
<212> PRT
<213> Plasmodium falciparum

<400> 513
Lys Pro Leu Ala Lys Leu Arg Lys Arg Glu Lys Thr Gln Ile Asn Lys
1               5                   10                  15

Thr Lys Tyr Glu Arg Gly Asp Val Ile Ile Asp Asn Thr Glu Ile Gln
            20                  25                  30

Lys Ile Ile Arg Asp Tyr His Thr Leu Asn Val His Lys Leu Asp His
        35                  40                  45


<210> 514
<211> 44
<212> PRT
<213> Plasmodium falciparum

<400> 514
Lys Leu Arg Lys Arg Glu Lys Thr Gln Ile Asn Lys Thr Lys Tyr Glu
1               5                   10                  15

Arg Gly Asp Val Ile Ile Asp Asn Thr Glu Ile Gln Lys Ile Ile Arg
            20                  25                  30

Asp Tyr His Thr Leu Asn Val His Lys Leu Asp His
        35                  40


<210> 515
<211> 41
<212> PRT
<213> Plasmodium falciparum

<400> 515
Lys Arg Glu Lys Thr Gln Ile Asn Lys Thr Lys Tyr Glu Arg Gly Asp
1               5                   10                  15

Val Ile Ile Asp Asn Thr Glu Ile Gln Lys Ile Ile Arg Asp Tyr His
            20                  25                  30

Thr Leu Asn Val His Lys Leu Asp His
        35                  40


<210> 516
<211> 38
<212> PRT
<213> Plasmodium falciparum

<400> 516
Lys Thr Gln Ile Asn Lys Thr Lys Tyr Glu Arg Gly Asp Val Ile Ile
1               5                   10                  15

Asp Asn Thr Glu Ile Gln Lys Ile Ile Arg Asp Tyr His Thr Leu Asn
            20                  25                  30

Val His Lys Leu Asp His
        35


<210> 517
<211> 44
<212> PRT
<213> Plasmodium falciparum

<400> 517
Lys Pro Leu Ala Lys Leu Arg Lys Arg Glu Lys Thr Gln Ile Asn Lys
1               5                   10                  15

Thr Lys Tyr Glu Arg Gly Asp Val Ile Ile Asp Asn Thr Glu Ile Gln
            20                  25                  30

Lys Ile Ile Arg Asp Tyr His Thr Leu Asn Val His
        35                  40


<210> 518
<211> 40
<212> PRT
<213> Plasmodium falciparum

<400> 518
Lys Leu Arg Lys Arg Glu Lys Thr Gln Ile Asn Lys Thr Lys Tyr Glu
1               5                   10                  15

Arg Gly Asp Val Ile Ile Asp Asn Thr Glu Ile Gln Lys Ile Ile Arg
            20                  25                  30

Asp Tyr His Thr Leu Asn Val His
        35                  40

<210> 519
<211> 37
<212> PRT
<213> Plasmodium falciparum

<400> 519
Lys Arg Glu Lys Thr Gln Ile Asn Lys Thr Lys Tyr Glu Arg Gly Asp
1               5                   10                  15

Val Ile Ile Asp Asn Thr Glu Ile Gln Lys Ile Ile Arg Asp Tyr His
            20                  25                  30

Thr Leu Asn Val His
        35


<210> 520
<211> 34
<212> PRT
<213> Plasmodium falciparum

<400> 520
Lys Thr Gln Ile Asn Lys Thr Lys Tyr Glu Arg Gly Asp Val Ile Ile
1               5                   10                  15

Asp Asn Thr Glu Ile Gln Lys Ile Ile Arg Asp Tyr His Thr Leu Asn
            20                  25                  30

Val His


<210> 521
<211> 32
<212> PRT
<213> Plasmodium falciparum

<400> 521
His Ile Met Leu Lys Ser Gln Met Tyr Thr Asn Glu Gly Asn Lys Ser
1               5                   10                  15

Cys Glu Cys Ser Tyr Lys Lys Lys Ser Ser Ser Ser Asn Lys Val His
            20                  25                  30


<210> 522
<211> 28
<212> PRT
<213> Plasmodium falciparum

<400> 522
Lys Ser Gln Met Tyr Thr Asn Glu Gly Asn Lys Ser Cys Glu Cys Ser
1               5                   10                  15

Tyr Lys Lys Lys Ser Ser Ser Ser Asn Lys Val His
            20                  25

<210> 523
<211> 18
<212> PRT
<213> Plasmodium falciparum

<400> 523
Lys Ser Cys Glu Cys Ser Tyr Lys Lys Lys Ser Ser Ser Ser Asn Lys
1               5                   10                  15

Val His


<210> 524
<211> 11
<212> PRT
<213> Plasmodium falciparum

<400> 524
Lys Lys Lys Ser Ser Ser Ser Asn Lys Val His
1               5                   10


<210> 525
<211> 10
<212> PRT
<213> Plasmodium falciparum

<400> 525
Lys Lys Ser Ser Ser Ser Asn Lys Val His
1               5                   10


<210> 526
<211> 9
<212> PRT
<213> Plasmodium falciparum

<400> 526
Lys Ser Ser Ser Ser Asn Lys Val His
1               5


<210> 527
<211> 30
<212> PRT
<213> Plasmodium falciparum

<400> 527
His Ile Met Leu Lys Ser Gln Met Tyr Thr Asn Glu Gly Asn Lys Ser
1               5                   10                  15

Cys Glu Cys Ser Tyr Lys Lys Lys Ser Ser Ser Ser Asn Lys
            20                  25                  30


<210> 528
<211> 24
<212> PRT

<213> Plasmodium falciparum

<400> 528
His Ile Met Leu Lys Ser Gln Met Tyr Thr Asn Glu Gly Asn Lys Ser
1               5                   10                  15

Cys Glu Cys Ser Tyr Lys Lys Lys
            20


<210> 529
<211> 23
<212> PRT
<213> Plasmodium falciparum

<400> 529
His Ile Met Leu Lys Ser Gln Met Tyr Thr Asn Glu Gly Asn Lys Ser
1               5                   10                  15

Cys Glu Cys Ser Tyr Lys Lys
            20


<210> 530
<211> 22
<212> PRT
<213> Plasmodium falciparum

<400> 530
His Ile Met Leu Lys Ser Gln Met Tyr Thr Asn Glu Gly Asn Lys Ser
1               5                   10                  15

Cys Glu Cys Ser Tyr Lys
            20


<210> 531
<211> 36
<212> PRT
<213> Plasmodium falciparum

<400> 531
His Asn Asn His Asn Ile Gln Ile Tyr Lys Asp Lys Arg Ile Asn Phe
1               5                   10                  15

Met Asn Pro His Lys Val Met Tyr His Asp Asn Met Ser Lys Asn Glu
            20                  25                  30

Arg Thr Glu Lys
        35


<210> 532
<211> 30
<212> PRT
<213> Plasmodium falciparum

<400> 532
His Asn Asn His Asn Ile Gln Ile Tyr Lys Asp Lys Arg Ile Asn Phe
1               5                   10                  15

Met Asn Pro His Lys Val Met Tyr His Asp Asn Met Ser Lys
            20                  25                  30

<210> 533
<211> 21
<212> PRT
<213> Plasmodium falciparum

<400> 533
His Asn Asn His Asn Ile Gln Ile Tyr Lys Asp Lys Arg Ile Asn Phe
1               5                   10                  15

Met Asn Pro His Lys
            20

<210> 534
<211> 17
<212> PRT
<213> Plasmodium falciparum

<400> 534
His Lys Val Met Tyr His Asp Asn Met Ser Lys Asn Glu Arg Thr Glu
1               5                   10                  15

Lys

<210> 535
<211> 11
<212> PRT
<213> Plasmodium falciparum

<400> 535
His Lys Val Met Tyr His Asp Asn Met Ser Lys
1               5                   10